# EUROPEAN PATENT SPECIFICATION

(11) **EP 3 039 034 B1**
(45) Date of publication and mention of the grant of the patent: **24.04.2019**
(21) Application number: 14786859.0
(22) Date of filing: 17.10.2014
(51) Int. Cl.: C07K 14/435, C12N 9/14, C12N 5/074, A61K 38/48

(54) **CELL DIFFERENTIATION MARKER AND ITS USES**
ZELLDIFFERENZIERUNGSMARKER UND VERWENDUNGEN DAVON
MARQUEUR DE DIFFÉRENCIATION CELLULAIRE ET SES UTILISATIONS

(30) Priority: 21.10.2013 EP 13306448
(43) Date of publication of application: 06.07.2016
(73) Proprietor: Centre National de la Recherche Scientifique (C.N.R.S.), 75794 Paris Cedex 16 (FR)
(72) Inventor: MAIORANO, Domenico, F-34380 Saint Martin De Londres (FR); VAN DER LAAN, Siem, 34190 Cazilhac (FR)
(74) Representative: Monni, Richard
(86) International application number: PCT/EP2014/072298
(87) International publication number: WO 2015/059044

(56) References cited:
- KR-B1- 100 794 694
- US-A1- 2003 022 201
- YARON PEREG ET AL: "Ubiquitin hydrolase Dub3 promotes oncogenic transformation by stabilizing Cdc25A", NATURE CELL BIOLOGY, vol. 12, no. 4, 14 March 2010 (2010-03-14) , pages 400-406, XP055096955, ISSN: 1465-7392, DOI: 10.1038/ncb2041
- SEBASTIAN D. HAYES ET AL: "Cdc25A and Dub3 in a high-stakes balancing act", NATURE CELL BIOLOGY, vol. 12, no. 4, 1 April 2010 (2010-04-01), pages 311-313, XP055096984, ISSN: 1465-7392, DOI: 10.1038/ncb2043
- BURROWS J F ET AL: "DUB-3, a cytokine-inducible deubiquitinating enzyme that blocks proliferation", JOURNAL OF BIOLOGICAL CHEMISTRY, AMERICAN SOCIETY FOR BIOCHEMISTRY AND MOLECULAR BIOLOGY, US, vol. 279, no. 14, 2 April 2004 (2004-04-02), pages 13993-14000, XP002356519, ISSN: 0021-9258, DOI: 10.1074/JBC.M311291200
- YADI WU ET AL: "The Deubiquitinase USP28 Stabilizes LSD1 and Confers Stem-Cell-like Traits to Breast Cancer Cells", CELL REPORTS, vol. 5, no. 1, 1 October 2013 (2013-10-01) , pages 224-236, XP055096995, ISSN: 2211-1247, DOI: 10.1016/j.celrep.2013.08.030
- DATABASE EMBL [Online] 21 October 2005 (2005-10-21), "Homo sapiens (human) deubiquitinating enzyme 3", XP002718870, retrieved from EBI accession no. EMBL:AAO38845
- SIEM VAN DER LAAN ET AL: "High Dub3 Expression in Mouse ESCs Couples the G1/S Checkpoint to Pluripotency", MOLECULAR CELL, vol. 52, no. 3, 1 November 2013 (2013-11-01), pages 366-379, XP055096836, ISSN: 1097-2765, DOI: 10.1016/j.molcel.2013.10.003
- STEFANO FORTE ET AL: "Gene Expression Analysis of PTEN Positive Glioblastoma Stem Cells Identifies DUB3 and Wee1 Modulation in a Cell Differentiation Model", PLOS ONE, vol. 8, no. 12, 12 December 2013 (2013-12-12), page e81432, XP055096964, DOI: 10.1371/journal.pone.0081432
- SIEM VAN DER LAAN ET AL: "Cell Cycle-Dependent Expression of Dub3, Nanog and the p160 Family of Nuclear Receptor Coactivators (NCoAs) in Mouse Embryonic Stem Cells", PLOS ONE, vol. 9, no. 4, 2 April 2014 (2014-04-02), page e93663, XP055160911, DOI: 10.1371/journal.pone.0093663

## Description

The present invention relates to a cell differentiation marker, in particular a totipotent/pluripotent stem cell marker, and its uses.

Eukaryotic cells have developed checkpoints to block cell cycle progression upon DNA damage or replication stress. Two distinct pathways pertain to the G1/S checkpoint by directly reducing CDK2 activity: a) rapid destruction of the Cdc25A phosphatase resulting in increased CDK2 phosphorylation, and b) a slower, p53-mediated, transcriptional response that activates expression of, amongst others, the potent CDK2 inhibitor p21. Importantly, rapid p21 degradation observed after exposure to low UV doses may be important for optimal DNA repair, while inhibition of CDK2 activity following Cdc25A degradation is sufficient for cell cycle arrest. Cdc25A protein levels are tightly regulated by two E3 ubiquitin ligases, the Anaphase Promoting Complex/Cyclosome (APC/CCdh1) as cells exit mitosis, and the Skp1-Cullin1-Fbox (SCF(^{β-TrCP}) during both S and G2 phase and following DNA damage.

Compared to somatic cells, mouse embryonic stem (ES) cells appear to have a relaxed G1/S checkpoint. The molecular mechanism underlying this feature remains unclear. Moreover, mouse ES cell cycle has remarkably short G1 and G2 phases, with little S phase length variation. This is underpinned by high CDK2/Cyclin E activity and reduced APC/C activity leading to limited oscillation in substrate levels. Interestingly, knockdown of CDK2 protein was shown to increase G1 length although DNA damage-dependent degradation of Cdc25A was reported not to affect CDK2 activity, nor to induce a G1 arrest.

Maintenance of pluripotency depends upon expression of pluripotency genes under the combinatorial control of a regulatory network of transcription factors such as Nanog, Sox2 and Oct4. Differentiation of ES cell induces cell cycle remodelling, including appearance of longer G1 and G2 phases, but how this regulation is achieved is unknown. Moreover, how the pluripotency regulatory network impacts onto cell cycle control remains obscure. Aside from its well-known role in somatic cell cycle, very little is known about Cdc25A function in ES cells. In human ES cells, Cdc25A expression was shown to be regulated by Nanog. A recent report shows that Nanog knockdown in mouse ES cells results in G1/S transition delay by an unknown mechanism. Equally, the role of p53 in ES cells G1/S DNA damage checkpoint still remains controversial. Despite its high abundance, p53 has been proposed to be inactive in ES cells due to a predominant cytoplasmic distribution.

However, pluripotency markers that are highly specific for pluripotent cells remain to be identified, and the purification of a homogenous population of stem cells, or totipotent/pluripotent cells is still difficult to achieve.Wu et al., (2013, CEell reports, vol. 5, no. 1, pages 224-236) relates to the deubiquitinase USP28 and describes that knockdown of USP28 results in the differentiation of cancer stem cells. But this document only concern cancer stem cells.

Therefore there is a need to provide new pluripotecy/totipotency markers to allow isolation of the most undifferentiated cells among a cell population of differentiated cells.

One aim of the invention is to provide a new differentiation marker expressed in undifferentiated cells.

Another aim of the invention is to regulate cell differentiation of pluripotent/totipotent cells.

Still another aim of the invention is to provide cells expressing such differentiation marker, and process for obtaining them.

The present disclosure relates to the use of:
- the Dub3 protein, said protein comprising the amino acid sequence as set forth in SEQ ID NO : 1, or any variant thereof having at least 43 % identity with said amino acid sequence SEQ ID NO: 1, and having ubiquitin hydrolase activity or
- a nucleic acid molecule coding for said protein or said variant thereof, or
- an inhibitor of the activity and/or of the expression of said protein or said variant thereof,
for modulating cell differentiation, in particular in vitro cell differentiation.

The invention is based on the unexpected observation made by the inventors that the presence or the amount of Dub3 protein is able to modulate cell differentiation state. In other words, the inventors have demonstrated that Dub3 protein, or its variant, or nucleic acid coding them, or inhibitor of said protein and variant can modulate the differentiation status of determined cells.

Reversible modification of target proteins with ubiquitin regulates an assortment of signaling pathways either through proteasomal degradation or by altering the activity and/or localization of constituent proteins. Ubiquitin conjugation is mediated via an E1-E2-E3 cascade, whereas ubiquitin removal is catalyzed by deubiquitinating enzymes (Dubs). The deconjugation reactions are performed by specific cysteine proteases which generate monomeric ubiquitin from a variety of C-terminal adducts. Deubiquitinating enzymes (DUBs) are the largest family of enzymes in the ubiquitin system with diverse functions, making them key regulators of ubiquitin-mediated pathways and they often function by direct or indirect association with the proteasome. The activity of DUBs has been implicated in several important pathways including cell growth, oncogenesis, neuronal disease and transcriptional regulation. DUBs catalyze the removal of ubiquitin from native conjugates, ubiquitin C-terminal extension peptides and linear poly-ubiquitin fusion or precursor proteins. DUBs are classed into two distinct families: ubiquitin C-terminal hydrolases (UCHs) and the ubiquitin-specific proteases (USPs/UBPs). UCHs are relatively small enzymes (20-30 kDa) that catalyze the removal of peptides and small molecules from the C-terminus of ubiquitin. Most UCHs cannot generate monomeric ubiquitin from protein conjugates or disassemble poly-ubiquitin chains.

Human Dub3, also called ubiquitin specific peptidase 17-like family member 2, comprises or consists of the amino acid sequence as set forth SEQ ID NO: 1.

In the disclosure, expression "for modulating cell differentiation" means both "for inducing differentiation" and "maintaining cell differentiation".

According to the disclosure, "modulating cell differentiation" should also be interpreted as "modulating cell differentiation status". Modulating cell differentiation status means that a determined cell, which is at a determined state of differentiation, can be
- either maintained in said state of differentiation, by inhibiting cell differentiation,
- or engaged towards differentiation, by activating cell differentiation.

In other words, by modulating cell differentiation state, the compounds according to the invention can
- either stimulate cell differentiation, i.e. a less specialized cell becomes a more specialized cell type,
- or inhibit cell differentiation, i.e. cells are maintained at a determined differentiation state despite extra or intracellular signals inducing cell differentiation,
- or reverse cell differentiation, i.e. a more specialized cell type becomes a less specialized cell type, by dedifferentiation.

According to the diclosure, any variant of Dub3 protein having at least 43 % identity with the amino acid sequence SEQ ID NO: 1, and having ubiquitin hydrolase activity can also modulate cell differentiation state.

By at least 43% identity, it is meant that the variants encompassed by the invention can have 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 % identity with the amino acid sequence SEQ ID NO: 1.

Advantageous Dub3 variants according to the inventions comprise or consist of the amino acid sequences as set forth in SEQ ID NO: 2 to SEQ ID NO: 19, i.e. SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO : 17, SEQ ID NO : 18 and SEQ ID NO : 19.

The above variants also harbor ubiquitin hydrolase activity, in particular deubiquitinase activity. This activity can be measured as described in Burrows et al, 2004, JBC, 279(14), 13993-14000. Briefly, the deubiquitination assay is based on the cleavage of ubiquitin-β-galactosidase (substrate) fusion proteins. Dub3 open reading frame (amino acids 1 to 530 of SEQ ID NO: 1), or variant thereof, and an equivalent open reading frame containing a catalytically inactive mutant form, Dub3C/S (C89S), or variant thereof, are generated by PCR and inserted in-frame into the pGEX vector in-frame with the glutathione S-transferase epitope. Ub-Met-β-galactosidase is expressed from a pACYC184-based plasmid. Plasmids are co-transformed into MC1061 *Escherichia coli* stain. Plasmid-bearing *E. coli* MC1061 cells are lysed and proteins analyzed by immunoblotting with a rabbit anti-β-galactosidase antiserum for detecting the substrate. Proteins are separated by SDS PAGE with a high density bisacrylamide-acrylamide gel to distinguish Ub-Met-β-galactosidase (uncleaved) and -β-galactosidase (cleaved) substrates. Protocol is also available in Papa et al. 1993, vol. 366, 313-319.

Therefore, the skilled person, by measuring the ability of the variants to deubiquitinate the Ub-Met-β-galactosidase substrate, can easily determine that a variant of Dub3 harbors deubiquitinase activity, i.e. ubiquitin hydrolase activity.

According to the invention, a nucleic acid molecule coding for said protein or said variant thereof is a nucleic acid that contain the nucleic information allowing the translation into said protein or said variant thereof, taking account of the genetic code degeneracy.
Advantageously, the nucleic acid coding the protein consisting of SEQ ID NO: 1 comprises the nucleic acid sequence as set forth in SEQ ID NO: 20.
Advantageously, the nucleic acid coding the protein consisting of SEQ ID NO: 2 comprises the nucleic acid sequence as set forth in SEQ ID NO: 21.
Advantageously, the nucleic acid coding the protein consisting of SEQ ID NO: 3 comprises the nucleic acid sequence as set forth in SEQ ID NO: 22.
Advantageously, the nucleic acid coding the protein consisting of SEQ ID NO: 4 comprises the nucleic acid sequence as set forth in SEQ ID NO: 23.
Advantageously, the nucleic acid coding the protein consisting of SEQ ID NO : 5 comprises the nucleic acid sequence as set forth in SEQ ID NO: 24
Advantageously, the nucleic acid coding the protein consisting of SEQ ID NO : 6 comprises the nucleic acid sequence as set forth in SEQ ID NO: 25
Advantageously, the nucleic acid coding the protein consisting of SEQ ID NO : 7 comprises the nucleic acid sequence as set forth in SEQ ID NO: 26
Advantageously, the nucleic acid coding the protein consisting of SEQ ID NO : 8 comprises the nucleic acid sequence as set forth in SEQ ID NO: 27
Advantageously, the nucleic acid coding the protein consisting of SEQ ID NO : 9 comprises the nucleic acid sequence as set forth in SEQ ID NO: 28
Advantageously, the nucleic acid coding the protein consisting of SEQ ID NO : 10 comprises the nucleic acid sequence as set forth in SEQ ID NO: 29
Advantageously, the nucleic acid coding the protein consisting of SEQ ID NO : 11 comprises the nucleic acid sequence as set forth in SEQ ID NO: 30
Advantageously, the nucleic acid coding the protein consisting of SEQ ID NO : 12 comprises the nucleic acid sequence as set forth in SEQ ID NO: 31
Advantageously, the nucleic acid coding the protein consisting of SEQ ID NO : 13 comprises the nucleic acid sequence as set forth in SEQ ID NO: 32
Advantageously, the nucleic acid coding the protein consisting of SEQ ID NO : 14 comprises the nucleic acid sequence as set forth in SEQ ID NO: 33
Advantageously, the nucleic acid coding the protein consisting of SEQ ID NO : 15 comprises the nucleic acid sequence as set forth in SEQ ID NO: 34
Advantageously, the nucleic acid coding the protein consisting of SEQ ID NO : 16 comprises the nucleic acid sequence as set forth in SEQ ID NO: 35
Advantageously, the nucleic acid coding the protein consisting of SEQ ID NO : 17 comprises the nucleic acid sequence as set forth in SEQ ID NO: 36
Advantageously, the nucleic acid coding the protein consisting of SEQ ID NO : 18 comprises the nucleic acid sequence as set forth in SEQ ID NO: 37
Advantageously, the nucleic acid coding the protein consisting of SEQ ID NO : 19 comprises the nucleic acid sequence as set forth in SEQ ID NO: 38

According to the disclosure, an inhibitor of the activity, i.e. of the ubiquitin hydrolase activity of Dub3 or a variant thereof can be chosen among the well-known compounds inhibiting such activity. An advantageous inhibitor is the PR-619 inhibitor, having the following formula I: 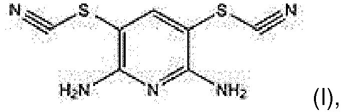 which is available from Sigma Aldrich (ref: SML0430). PR-619 is a cell permeable broad spectrum deubiquitylating enzymes (DUBs) inhibitor. PR-619 induces the accumulation of polyubiquitylated proteins in cells without directly affecting proteasome activity.

Inhibitory effect of such inhibitor can be measured as mentioned above.

Specific antibodies, which for instance recognize catalytic domain of Dub3, or variant thereof, can also be used for the purpose of the invention. Antibodies, monoclonal or polyclonal, obtained by immunization of animal with the peptide consisting of SEQ ID NO: 39 are advantageous.

According to the disclosure, an inhibitor of expression of Dub3 or a variant thereof can be chosen among miRNA, siRNA, shRNA, or antisense nucleic acid molecules specific to the Dub3 or variant thereof sequence.

Another aspect of the invention concerns a method for modulating cell differentiation, in particular in vitro, comprising a step of introduction in a cell for which a modification of the differentiation state is required of an effective amount of
- the Dub3 protein, said protein comprising the amino acid sequence as set forth in SEQ ID NO : 1, or any variant thereof having at least 43 % identity with said amino acid sequence SEQ ID NO: 1, and having ubiquitin hydrolase activity or
- a nucleic acid molecule coding for said protein or said variant thereof, or
- an inhibitor of the activity and/or of the expression of said protein or said variant thereof.

Advantageously, the disclosure relates to the use as defined above, wherein said cell is totipotent or pluripotent cell. Thus, the invention advantageously relates to the use as defined above for modulating totipotent and multipotent cell differentiation, in particular in vitro totipotent and multipotent cell differentiation.

Totipotent stem cells can differentiate into embryonic and extra-embryonic cell types. Pluripotent stem cells originate from totipotent cells and can give rise to progeny that are derivatives of the three embryonic germ layers, mesoderm, ectoderm and endoderm.

Another aspect of the disclosure concerns a method for modulating totipotent or pluripotent cell differentiation, in particular in vitro, comprising a step of introduction in a cell for which a modification of the differentiation state is required of an effective amount of
- the Dub3 protein, said protein comprising the amino acid sequence as set forth in SEQ ID NO : 1, or any variant thereof having at least 43 % identity with said amino acid sequence SEQ ID NO: 1, and having ubiquitin hydrolase activity or
- a nucleic acid molecule coding for said protein or said variant thereof, or
- an inhibitor of the activity and/or of the expression of said protein or said variant thereof.

The invention also relates to the use of
- Dub3 protein, said protein comprising the amino acid sequence as set forth in SEQ ID NO : 1, or any variant thereof comprising one of the amino acid sequences as set forth in SEQ ID NO: 2 to SEQ ID NO: 19, or
- a nucleic acid molecule coding for said protein or said variant thereof,
for inducing in vitro dedifferentiation of differentiated cells, wherein the the cells obtained from the dedifferentiation of differentiated cells are iPS cells.

The inventors have observed that Dub3 protein is expressed in stem cells, and progressively disappears during differentiation process. They postulate that enforced expression of Dub3 would, in association with other genes, induce a dedifferentiation of somatic cells.

Induced pluripotent stem cells, commonly abbreviated as iPS cells or iPSCs are a type of pluripotent stem cell artificially derived from a non-pluripotent cell - typically an adult somatic cell - by inducing a "forced" expression of specific genes. Induced pluripotent stem cells are similar to natural pluripotent stem cells, such as embryonic stem (ES) cells, in many aspects, such as the expression of certain stem cell genes and proteins, chromatin methylation patterns, doubling time, embryoid body formation, teratoma formation, viable chimera formation, and potency and differentiability. Advantageously, the invention relates to the use as defined above, for inducing in vitro dedifferentiation of differentiated cells, wherein said cells Dub3 protein, or a variant thereof, or said nucleic acid molecule coding for said protein, or said variant thereof, is associated with at least an Oct family member protein and a Sox family member protein.

According to this embodiment, iPS cells are obtained by allowing the expression, in a somatic differentiated cell, of at least Oct4 protein and a Sox2 protein, along with at Dub3 protein.

Advantageously, iPS cells can be obtained, from differentiated cells expressing Oct4/Sox2 and Dub3 genes, in particular expressing Oct4/Sox2/cMyc and Dub3 genes.

The disclosure also relates to the use as defined above, wherein said Dub3 protein or a variant thereof, or said nucleic acid molecule coding for said protein, or said variant thereof, is expressed in said iPS cells at a level corresponding to at least 2 fold lower than the expression of said Dub3 protein in totipotent or pluripotent cells.

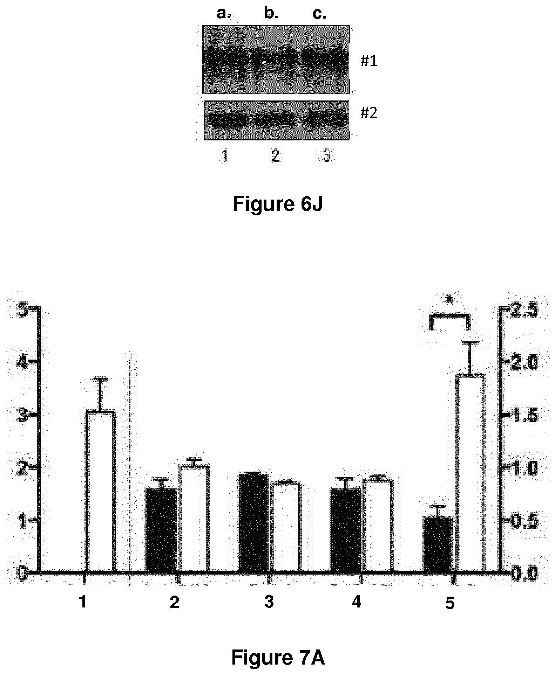

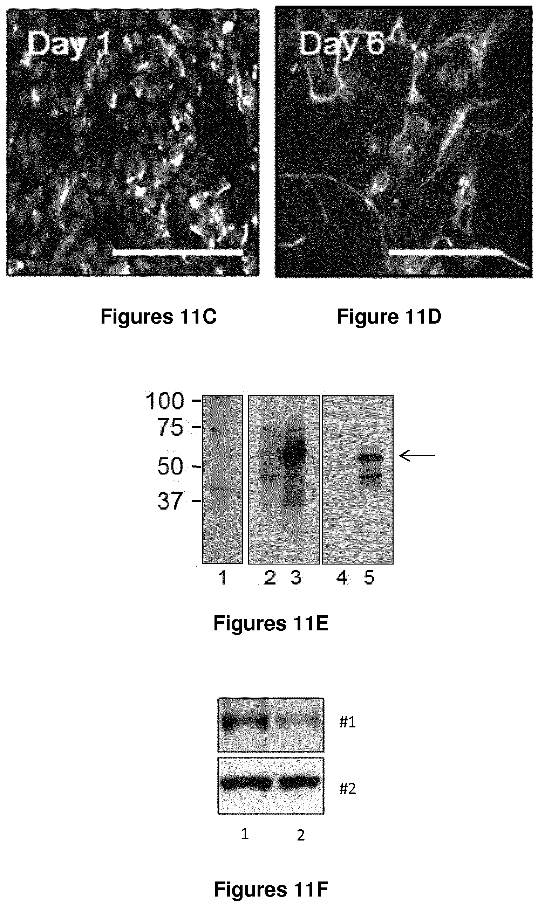

It is possible to measure the expression of Dub3 by quantitative determination of Dub3 mRNA abundance by RT-PCR, one example of which is provided in Figure 7A and/or by detection of the Dub3 protein by western blot using a specific antibody, such as one described in Figure 11E.

The advantage of this level of expression being that said iPS cells will be now able to efficiently respond to DNA damage and/or replication stress generated by ectopic expression of factors such as c-myc or Oct family proteins, required for generating said iPS cells and thereby preserving genomic stability by reduction of CDK2 activity and resulting delay in the G1 phase of the cell cycle.

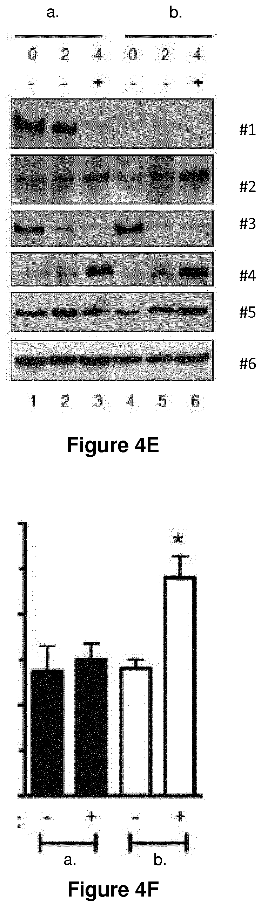

Such effect is exemplified in figure 4F. Such iPS cells, called "checkpoint-competent" pluripotent iPS, would be then advantageous in cell therapy use since unlike currently-used iPSs their teratogenic abilities are largely reduced.

The invention also relates to the use of an inhibitor of the activity and/or of the expression of the Dub3 protein or a variant thereof, said protein comprising the amino acid sequence as set forth in SEQ ID NO : 1, or any variant thereof comprising one of the amino acid sequences as set forth in SEQ ID NO: 2 to SEQ ID NO: 19, for inducing *in vitro* the spontaneous differentiation of totipotent or pluripotent cells.

The inventors have made the unexpected observation that inhibition of Dub3 activity and/or expression induce a spontaneous differentiation of totipotent or pluripotent cells. Inhibitors that can be used are those as mentioned above.

The disclosure relates to the use of Dub3 protein, said protein comprising the amino acid sequence as set forth in SEQ ID NO : 1, or any variant thereof having at least 43% identity with said amino acid sequence SEQ ID NO: 1 and having ubiquitin hydrolase activity, for determining the differentiation state of cells belonging in a population of cells.

The inventors have also made the unexpected observation that Dub3 protein is rapidly repressed during differentiation process (Dub3 expression is switch off during the differentiation process). Indeed, as shown in examples, Dub3 protein levels dropped massively very early during differentiation, much earlier than Oct4.

Thus, since Oct4 is to date the most commonly used differentiation marker used to determine the differentiation state of cells, the use according to the above definition is advantageous because it gives a more precise status of the cell differentiation state.

The disclosure also relates to a method for determining the differentiation state of cells belonging in a population of cells, comprising a step of measuring in a cell the presence or amount of Dub3 protein, said protein comprising the amino acid sequence as set forth in SEQ ID NO : 1, or any variant thereof having at least 43% identity with said amino acid sequence SEQ ID NO: 1, and having ubiquitin hydrolase activity, such that:
- if Dub3 protein or variant thereof is present, then the cell is a totipotent or a pluripotent cell, and
- if Dub3 protein or variant thereof is absent, then the cell is a differentiated cell or a differentiating cell.
By "differentiating cell" it is meant in the invention a cell that morphologically appears to be a totipotent or a pluripotent cell, but harbors molecular signs of differentiation. Molecular signs of differentiation can be, for instance, expression of specific gene such as the endoderm marker Sox7, the neuroectoderm markers Sox1 and Nestin and repression of specific genes, such as the transcription factors of the pluripotency network Nanog, Sox2, Klf4.

Moreover, the disclosure relates to a method for isolating stem cells from a population of non tumoral cells comprising the determination of the presence or the amount of the Dub3 protein, said protein comprising the amino acid sequence as set forth in SEQ ID NO : 1, or any variant thereof having at least 43% identity with said amino acid sequence SEQ ID NO: 1 and having ubiquitin hydrolase activity, and optionally a step of isolating cells expressing said Dub3 protein.
By using common technics known by the skilled person, such as flow cytometry, and immunological material (i.e. appropriate antibodies directed against Dub3 protein or variant thereof), it is possible to specifically label cells expressing said Dub3 protein, and therefore isolate them from other cells that do not express Dub3 protein or variant thereof.

The disclosure also relates to a composition comprising
- Dub3 protein, said protein comprising the amino acid sequence as set forth in SEQ ID NO : 1, or any variant thereof having at least 43% identity with said amino acid sequence SEQ ID NO: 1 and having ubiquitin hydrolase activity, or
- a nucleic acid molecule coding for said protein or said variant thereof, or
- an inhibitor of the activity, i.e. the ubiquitin hydrolase activity and/or of the expression of said protein or said variant thereof,
for its use for the treatment of therapy-resistant tumors, or cancers.
Properties of the small group of cancer cells called tumor-initiating or cancer stem cells (CSCs) involved in drug resistance and relapse of cancers can significantly affect tumor therapy. Importantly, tumor drug resistance seems to be closely related to many intrinsic or acquired properties of CSCs, such as quiescence, specific morphology, DNA repair ability and overexpression of antiapoptotic proteins, drug efflux transporters and detoxifying enzymes. The specific microenvironment (niche) and hypoxic stability provide additional protection against anticancer therapy for CSCs. Thus, CSC-focused therapy is destined to form the core of any effective anticancer strategy.

Thus the inventors, intended to solve the problem of the resistance of cancers, propose a new pharmaceutical composition for this purpose.

In one aspect, a composition comprising Dub3 protein, or variant thereof as defined above, or a nucleic acid molecule coding such protein or variant would induce differentiation process in cancer stem cells, rendering such cells susceptible to the therapy adapted to the differentiated cancer cells. In particular embodiment, cancer stem cells expressing the Dub3 protein, or variant thereof, die by apoptosis because they ectopically express Dub3 protein.

In another aspect, a composition comprising an inhibitor or the activity or of the expression of Dub3 protein or a variant thereof would induce spontaneous differentiation of cancer stem cells, rendering such cells susceptible to the therapy adapted to the differentiated cancer cells.

Therefore, the composition according to the disclosure allows to treat specific types of cancer that are resistant to conventional cancer therapies, such as chemotherapies. The disclosure also relates to a method for treating therapy-resistant tumors or cancers, comprising the administration to a patient in a need thereof of an effective amount of a composition comprising :
- Dub3 protein, said protein comprising the amino acid sequence as set forth in SEQ ID NO : 1, or any variant thereof having at least 43% identity with said amino acid sequence SEQ ID NO: 1 and having ubiquitin hydrolase activity, or
- a nucleic acid molecule coding for said protein or said variant thereof, or
- an inhibitor of the activity, i.e. the ubiquitin hydrolase activity and/or of the expression of said protein or said variant thereof.

Advantageously, the disclosure relates to a composition for its use as defined above, or a method as defined above, comprising an inhibitor of the activity, i.e. the ubiquitin hydrolase activity, and/or of the expression of said Dub3 protein, said inhibitor being chosen among siRNA, miRNA, shRNA, RNA antisense, DNA antisense, antibodies or chemical compounds.

Antibody obtained from the animal immunization by the peptide consisting of the amino acid sequence as set forth in SEQ ID NO: 39.

Compound of formula I, as defined above, is also advantageous.

More advantageously, the invention disclosure to a composition for its use as defined above, or a method as defined above, wherein said inhibitor is a siRNA comprising of the following amino acid sequence as set forth in SEQ ID NO: 41 or SEQ ID NO:42. The siRNA of SEQ ID NO: 42 is 5'- UAGCACACAUCUUACAGCC -3' .

Thus, most advantageous siRNA according to the invention is a siRNA comprising a sense strand comprising or consisting in SEQ ID NO: 41 and its complementary sequence, or antisense strand, comprising or consisting of SEQ ID NO : 42.

The above siRNA can also be modified by addition of compounds stabilizing siRNA structure. For instance, the above siRNA contain, in their 3'- end a dinucleotide: a dithymidine (TT).

In one another advantageous embodiment, the disclosure relates to a composition for its use as defined above, wherein said shRNA comprises or consists of a nucleic acid molecule comprising or being constituted by the sequence SEQ ID NO : 41 followed by the sequence SEQ ID NO: 42, the 3'-end of SEQ ID NO: 41 being linked to the 5'-end of SEQ ID NO: 42 by a linker. The linker can be chosen among the following linkers
1) UUCAAGAGA (Brummelkamp, T.R., 2002 Science.296(5567):550-3),
2) AAGUUCUCU (Promega),
3) UUUGUGUAG (Scherr, M., Curr Med Chem. 2003 Feb;10(3):245-56.),
4) CUUCCUGUCA (SEQ ID NO : 43) (Schwarz D. S. , 2003 Cell. 115(2):199-208.), and
5) CUCGAG.

Nucleic acid molecules coding said shRNA (i.e. DNA coding shRNA) are encompassed by the present invention.

The disclosure relates to the use of
- the Dub3 protein, said protein comprising the amino acid sequence as set forth in SEQ ID NO : 1, or any variant thereof having at least 43% identity with said amino acid sequence SEQ ID NO: 1, or
- a nucleic acid molecule coding for said protein or said variant thereof,
for inducing cell death of differentiating stem cells, totipotent cells and/or pluripotent stem cells, preferably in vitro.

As mentioned in the example section, the inventors have shown that enforced expression of Dub3 protein, or variant thereof as defined above, induce both differentiation process in stem cells (or totipotent or pluripotent cells), and cell death by apoptosis.

The disclosure relates to the a method for inducing cell death of totipotent and or pluripotent stem cells, comprising the administration to said cells an effective amount of :
- the Dub3 protein, said protein comprising the amino acid sequence as set forth in SEQ ID NO : 1, or any variant thereof having at least 43% identity with said amino acid sequence SEQ ID NO: 1, or
- a nucleic acid molecule coding for said protein or said variant thereof.

The invention will be better understood from the following examples and taking account of the following figures.

### Legend to the figures

**Figures 1A-I** show that DNA damage in G1 induces transient cell cycle arrest in early S-phase and not at the G1/S transition
**Figure 1A** represents a flow cytometry analysis of DNA content of ES cells treated with various doses of UV. Cell cycle profile of asynchronously growing ES cells exposed to increasing dose of UV-light (0, 2, 4, 6 or 10 J/m2 - Z-axis). Cells were collected 6 hours after UV-irradiation for FACS analysis. X axis represents the cell number, and y axis represents the DNA content measured by Propidium Iodide fluorescence.
**Figure 1B** represents a flow cytometry analysis of DNA content of ES cells treated with UV in time. Cell cycle profile of asynchronously growing ES cells exposed to increasing dose of UV-light (0, 2, 4, 6 or 10 J/m2). Asynchronously growing ES cells were exposed to 6 J/m2 UV-irradiation and collected for FACS analysis at indicated time points (0, 2, 4 or 6 hours; Z-axis). X axis represents the cell number, and y axis represents the DNA content measured by Propidium Iodide fluorescence.
**Figure 1C** is a photography showing the fluorescence detection of DNA content using DAPI in NIH-3t3 cell lines. Scale bar represents 10 µm.
**Figure 1D** is a photography showing the fluorescence detection of DNA content using DAPI in ES cells. Scale bar represents 10 µm.
**Figure 1E** is a photography showing the immunofluorescence detection of Oct4 protein using specific antibody in NIH-3t3 cell lines. Scale bar represents 10 µm.
**Figure 1F** is a photography showing the immunofluorescence detection of Oct4 protein using specific antibody in ES cells. Scale bar represents 10 µm.
**Figure 1G** represents a western blot showing the expression of Cyclin A (#1), Histone H3 (#2), γH2AX (#3), DNA polymerase α (#4) and Cdc45 (#5) proteins into soluble (a.) and insoluble (chromatine-bound; b.) fractions of ES cells released from nocodazole arrest untreated or UV-irradiated in G1 (2 hours after release) collected at indicated time points. t: time.
**Figure 1H** is a histogram showing the qPCR quantification of Cyclin mRNA normalised to multiple reference genes from ES cells released from nocodazole arrest mock or UV-irradiated in G1 and collected at indicated time points. Dotted line represents levels in G1. Data are expressed as mean ± SD (error bars) of multiple observations.
**Figure 1I** is a histogram showing the qPCR quantification of Cyclin A2 mRNA normalised to multiple reference genes from ES cells released from nocodazole arrest mock or UV-irradiated in G1 and collected at indicated time points. Dotted line represents levels in G1. Data are expressed as mean ± SD (error bars) of multiple observations.
**Figures 2A-F** show that DNA damage in G1 induces transient ES cell cycle arrest in early S-phase and not at the G1/S transition.

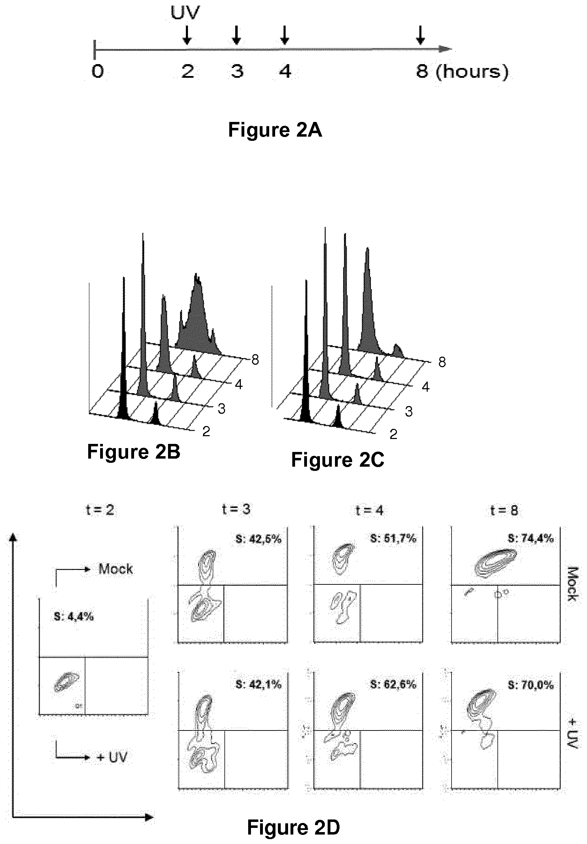

**Figure 2A** is a schematic overview of the experimental design. Arrows indicate time points at which cells were collected.
**Figure 2B** represents a FACS analysis of ES cells released from nocodazole arrest, mock. Analysis of total DNA content stained by propidium iodide at indicated time points.
**Figure 2C** represents a FACS analysis of ES cells released from nocodazole arrest, exposed to 6 J/m2 UV light in G1. Analysis of total DNA content stained by propidium iodide at indicated time points.
**Figure 2D** represents a FACS analysis of kinetics of S phase entry of synchronised ES cells, mock and UV-irradiated (6 J/m2) in G1. Cell cycle distribution was measured by BrdU incorporation followed by FACS analysis.

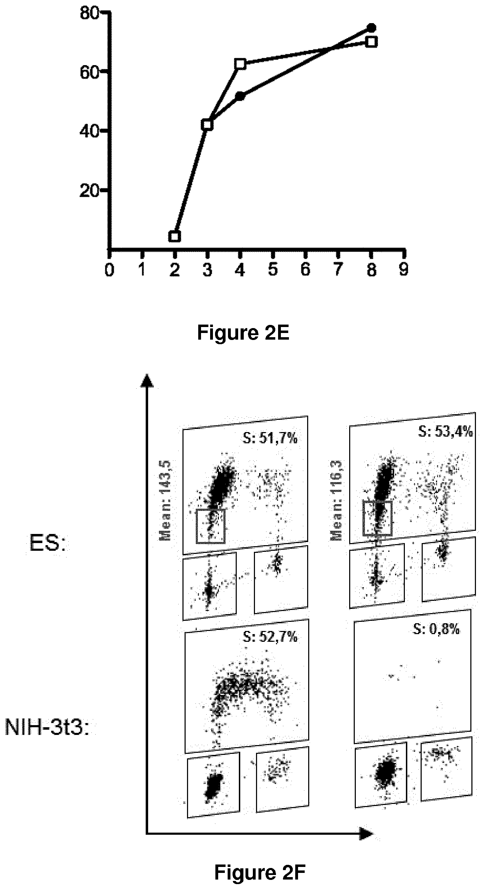

**Figure 2E** is a curve that summarize figure 2D. X-axis represents time in hours, and Y-axis represents the percentage of BrdU positive cells. Curve with black circles represents untreated cells and curve with open squares represents UV-treated cells.
**Figure 2F** shows representative FACS analysis of S-phase entry by analysis of BrdU immunoreactivity of ES and NIH-3t3 cells exposed respectively to 6 and 10 J/m2 UV light in G1. Box indicates region were differences in total events was observed. Mean fluorescence intensity of BrdU-positive cells is shown.

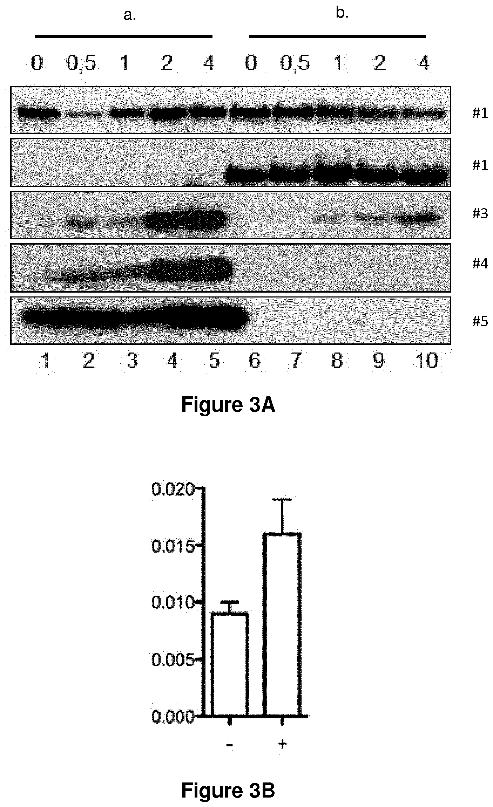

**Figures 3A-F** show that p53 is transcriptionally active in ES cells upon DNA damage.
**Figure 3A** represents a western blot showing the expression of MCM2 (#1), Chk1 (#2), p53^{S15P} (#3), γH2AX (#4) and Histone H3 (#5) in subcellular fractions of ES cells UV-irradiated and collected at indicated time points (hours post UV treatment). Cells were lysed and fractionated into soluble (b.) and insoluble (chromatin-bound; a.) fractions.
**Figure 3B** is a histogram showing the relative luciferase activity (firefly/renilla) of ES cells transfected with pG13-luc promoter (containing 13x p53 response elements) untreated (-) or UV-irradiated (+). Bars represent the mean ± SD of triplicate observations.

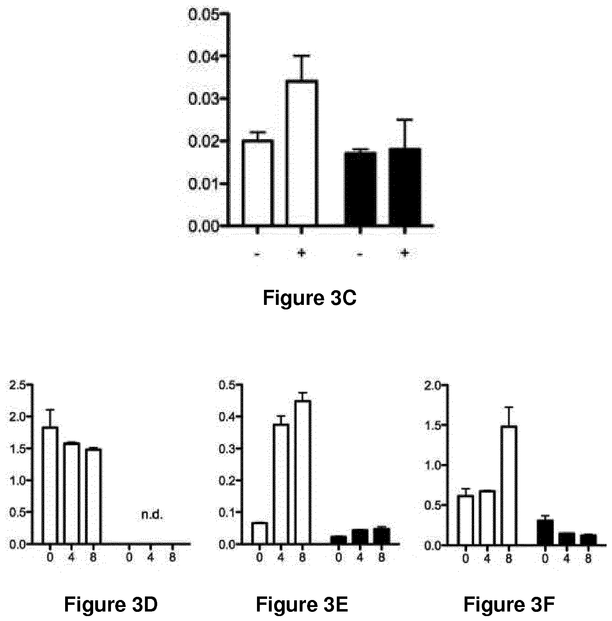

**Figure 3C** is a histogram showing the relative luciferase activity (firefly/renilla) of ES cells transfected with p21-luc (white bars) and p21-ΔREp53-luc (lacking p53 response element) (black bars) untreated (-) or UV-irradiated (+). Bars represent the mean ± SD of triplicate observations.
**Figure 3D** is a histogram showing the relative mRNA expression, measured by qPCR, of p53 gene in Wild-type (white bars) and p53 knockout (n.d.: not determined)) ES cells. ES cells were UV-irradiated and collected at indicated time points (X-axis: time after UV in hours). Bars represent the mean ± SD of triplicate observations.
**Figure 3E** is a histogram showing the relative mRNA expression, measured by qPCR, of p21 gene in Wild-type (white bars) and p53 knockout (black bars) ES cells. ES cells were UV-irradiated and collected at indicated time points (X-axis: time after UV in hours). Bars represent the mean ± SD of triplicate observations.
**Figure 3F** is a histogram showing the relative mRNA expression, measured by qPCR, of Mdm2 gene in Wild-type (white bars) and p53 knockout (black bars) ES cells. ES cells were UV-irradiated and collected at indicated time points (X-axis: time after UV in hours). Bars represent the mean ± SD of triplicate observations.

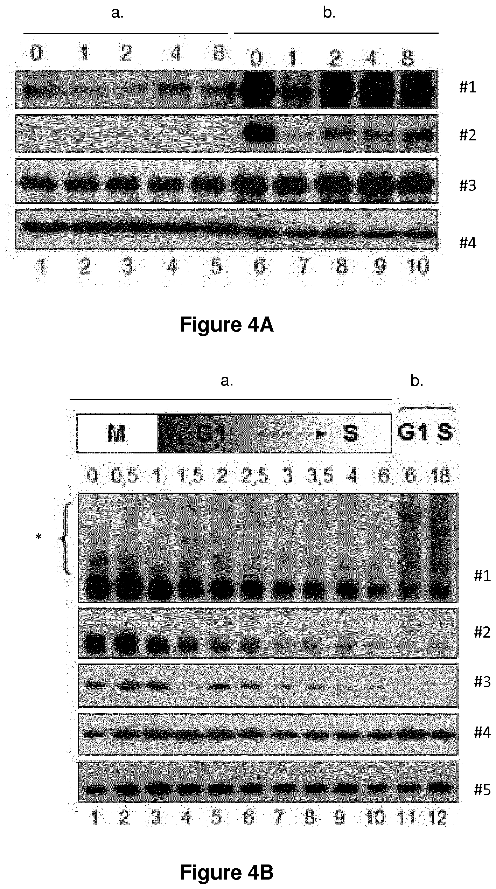

**Figures 4A-F** show the persistence of Cdc25A upon DNA damage in G1 sustains G1/S checkpoint bypass in ES cells.
**Figure 4A** is a western blot showing expression level of Cdc25A (#1, dark exposure and #2 light exposure), Cdk2 (#3) and β-actin (#4; as control) in asynchronously growing ES (b.) and NIH-3t3 (a.) cells exposed to 10 J/m2 of UV-light and collected at the indicated times (hours post UV).
**Figure 4B** is a western blot showing expression level of Cdc25A (#1, dark exposure and #2 light exposure), H3^{S10P} (#3), H3 (#4) and β-actin (#5; as control) in ES (a.) and NIH-3t3 (b.) cells synchronized in G1 and passing through S phase. ES cells were synchronized by nocodazole and collected upon release at indicated time points (release in hours). NIH-3t3 cells were synchronized by confluence, released and collected at 6 hours (G1) and 18 hours (S) after release. To observe posttranslational modifications (PTM; asterisk) of Cdc25A, dark exposure is shown.

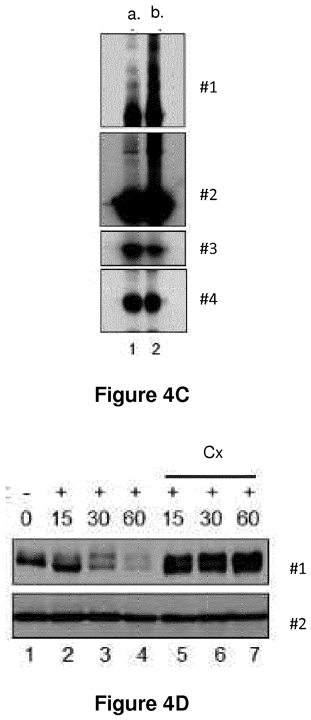

**Figure 4C** is a western blot of Flag-immunoprecipitated, ectopically expressed Flag-Cdc25A cotransfected with HA-ubiquitin in ES (a.) and NIH-3t3 cells (b.) after MG132 treatment for 1 hour. Presence of Cdc25A (#2, dark exposure and #3 light exposure) and HA (#1) is shown. Immunoglobulins (#4) are also shown.
**Figure 4D** is a western blot showing the rapid Cdc25A destruction upon DNA damage is Chk1-dependent in ES cells. Cells were UV-irradiated and incubated with cycloheximide (Cx) in absence or presence of Chk1 inhibitor SB218078, collected at the indicated times (min) and analyzed by western blotting. Cdc25A expression (#1) and β-actin (#2; as control) are shown.
**Figure 4E** is a western blot showing the downregulation of Cdc25A expression by RNAi resulting in increased inhibitory CDK2Tyr15 phosphorylation upon DNA damage in G1. Control (a.) and Cdc25A (b.) RNAi-transfected cells were released from nocodazole and exposed (+) to UV-light in G1. Samples were collected at the indicated times and analyzed by western blotting with the indicated antibodies : Cdc25A (#1), Cdk2^{Y15P} (#2), Cyclin B1 (#3), Chk1^{S345P} (#4), Chk1 (#5) and β-actin (#6; as control).
**Figure 4F** is a histogram showing Cdc25A downregulation in G1 delay upon DNA damage. Control (a.) and Cdc25A (b.) RNAi-transfected cells were released from nocodazole and exposed to UV light in G1 (t=2) and collected 2 hours (t=4) after UV-(+) or mock-irradiation (-). Prior to collection cells were pulse-labelled with BrdU. Fraction (expressed as %) of diploid BrdU negative cells is plotted (data are represented as mean ± SD). Statistical differences is indicated with a single asterisk (*) for P < 0.05. Y-axis represents the percentage of cells in G1.

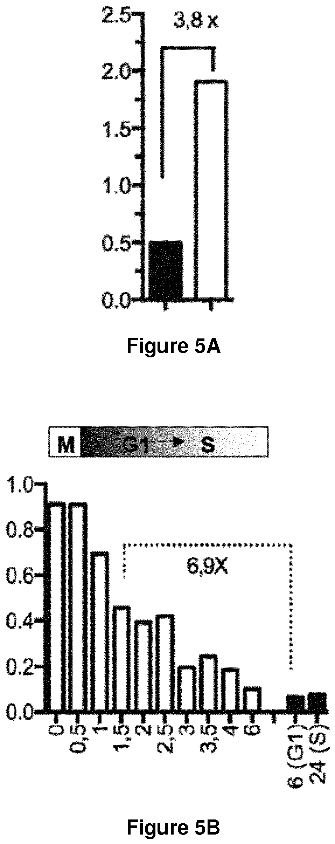

**Figures 5A-H** show that persistent Cdc25A phosphatase upon DNA damage in G1 inhibits G1/S checkpoint in ES cells.
**Figure 5A** is a histogram showing the quantification of western blotting signals shown in Fig. 4A Western blot signals (lane 1 (black bar) and lane 7(white bar)) of Cdc25A (dark exposure) were quantified by densitometry scanning and expressed as relative optical density (ROD) compared to β-actin signal as loading control (Y- axis).
**Figure 5B** is a histogram showing the quantification of western blotting signals shown in Figure 4B. Western blot signals of Cdc25A were quantified by densitometry scanning and expressed as relative optical density (ROD) compared to β-actin signal as loading control (Y- axis). Black bars represent NIT-3t3 cells and whit bars represent ES cells.

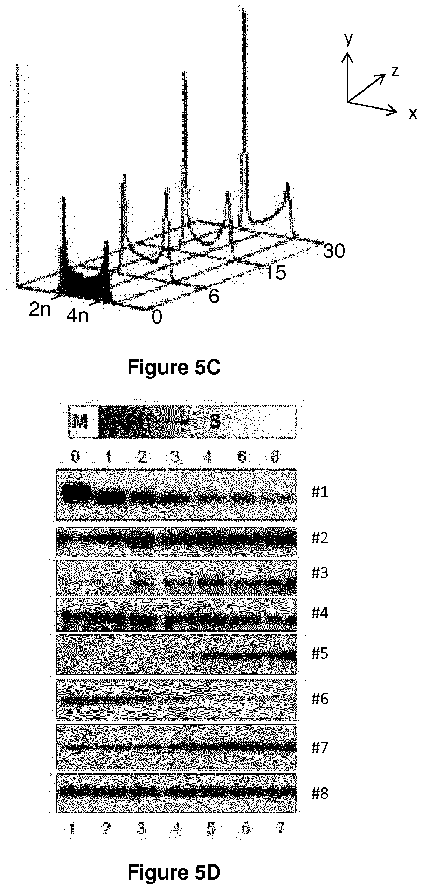

**Figure 5C** is FACS analysis of asynchronously growing ES cells treated with increasing concentration of Roscovitine (in µM; Z-axis). Roscovitine is a potent and selective inhibitor of cyclin-dependent kinases, dependent lengthening of the G1 phase of ES cells. X-axis represents DNA content (expressed in propidium iodide fluorescence) and Y-axis represents the number of cells.
**Figure 5D** is a western blot showing the Cdk2 phosphorylation status (Y15P) during an unperturbed cell cycle. ES cells were released from nocodazole arrest and collected in G1 and S-phase at indicated time points. Proteins Cdc25A (#1), Wee1 (#2), Cdk2^{Y15P} (#3), Cdk2 (#4), Cyclin A (#5), H3^{S10P} (#6), H3 (#7) and β-actin (#8; as control) were detected by western blotting.

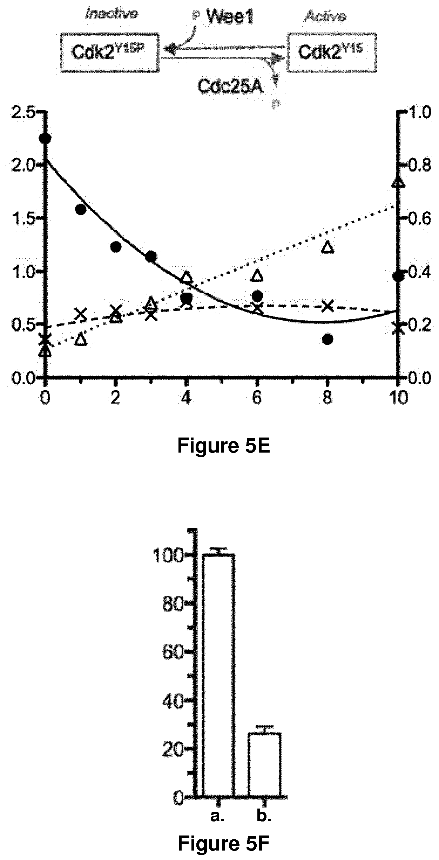

**Figure 5E** is a schematic representation of the regulation of phosphorylation on Cdk2 by Wee1 and Cdc25A. Western blot signals of figure 5D were quantified by densitometry scanning and expressed as relative optical density (ROD) compared to β-actin signal as loading control. Right X-axis represents the Cdc25A and Wee1 protein levels, relative to β-actin, and left X-axis represents the Cdk2^{Y15P} expression level. Curve with black circles represents Cdc25A expression level, curve with triangle represents Cdk2^{Y15P} expression level and curve with crosses represents the Wee1 expression level. Y-axis represents he time in hours after release.
**Figure 5F** is a histogram representing the qPCR quantification of Cdc25A mRNA normalized to multiple reference genes expressed as percentage of control. ES cells were transfected with control (a.) RNAi or Cdc25A (b.) RNAi sequences. Bars represent the mean ± SD of multiple observations.

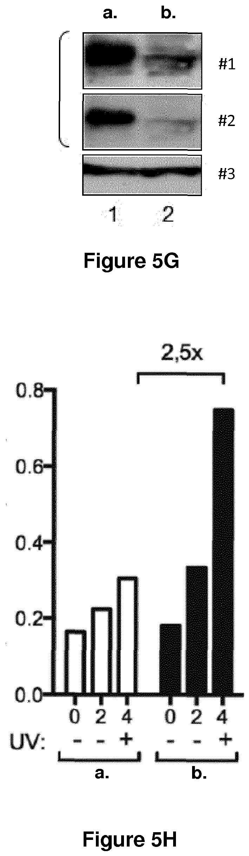

**Figure 5G** is a western blot analysis of ES cells transfected with control (a.) or Cdc25A (b.) RNAi sequences. The expression if Cdc25A of Cdc25A (#1, dark exposure and #2 light exposure), and β-actin (#2; as control) is represented.
Figure 5H is a histogram showing the quantification of western blotting signals shown in Figure 4E. Western blot signals of Fig. 4E were quantified by densitometry scanning and expressed as relative optical density (ROD) compared to Chk1 signal as loading control. Black bars represent cells treated with Cdc25A RNAi (a.) and white bars represent cells treated with control RNAi (b.).

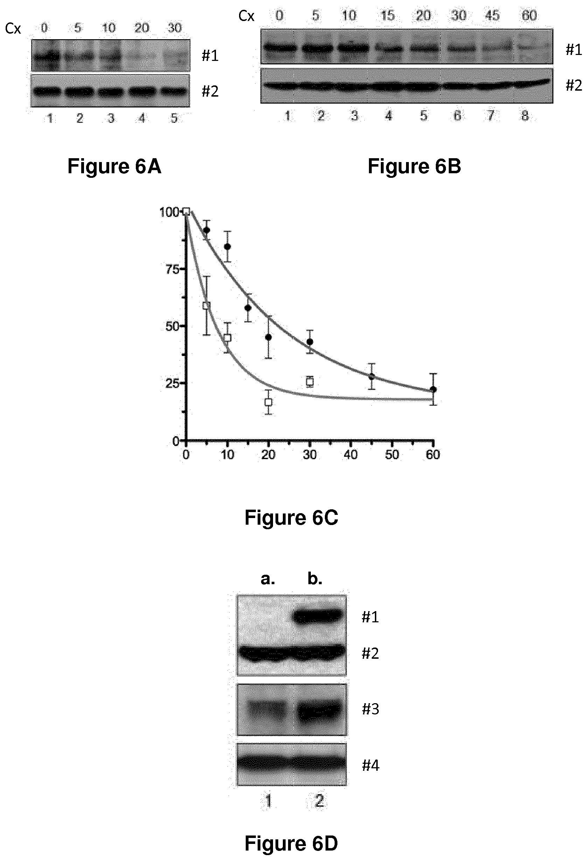

**Figures 6A-J** shows that elevated deubiquitylating enzyme Dub3 in ES cells results in Cdc25A abundance.
**Figure 6A** shows a representative western blot signal used for determination of Cdc25A turnover rate in the presence of cycloheximide (Cx) during the indicated times (min) in NIH-3t3 cells. Cells were collected at indicated time points. Expresion of Cdc25A (#1) and β-actin (#2; as control) are represented.
**Figure 6B** shows a representative western blot signal used for determination of Cdc25A turnover rate in the presence of cycloheximide (Cx) during the indicated times (min) in ES cells. Cells were collected at indicated time points. Expresion of Cdc25A (#1) and β-actin (#2; as control) are represented.
**Figure 6C** is a graph showing Cdc25A turnover rate in the presence of cycloheximide (Cx) in ES and NIH-3t3 cells. Western blot signals of Cdc25A were quantified by densitometry scanning and expressed as relative optical density (ROD) compared to β-actin signal as loading control. Signal in untreated cells were set at 100% and half-life (t1/2) of Cdc25A was determined (data are represented as mean ± SD). Curve with black circles represents ES cells, and curve with white squares represents NIH-3t3 cells. Y-axis represents Cdc25A protein levels expressed in percent and X-axis represents time in min.
**Figure 6D** shows that overexpression of Dub3 increases Cdc25A abundance. NIH-3t3 cells were transduced with empty vector (a.) or pLPC encoding Myc6-Dub3 (b.). After puromycin selection cells were collected and processed for western blot analysis. Proteins were detected with myc (#1), Chk1 (#2), Cdc25A (#3) and β-actin (#4, as control) antibodies.

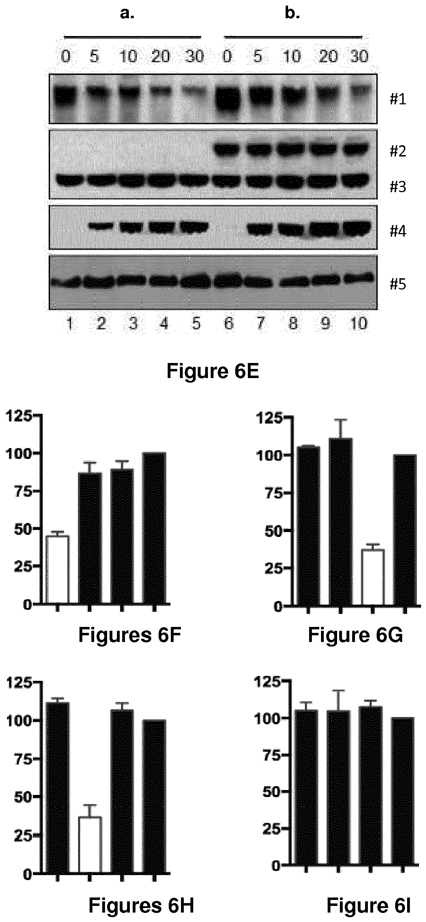

**Figure 6E** is a western blot showing Cdc25A degradation upon DNA damage in NIH-3t3 cells expressing empty vector (a.) or pLPC encoding Myc6-Dub3 (b.). Cells were collected at indicated time points (min post UV treatment) and analyzed by western blotting. Expression of Cdc25A (#1), Myc (#2), Chk1 (#3), Chk1^{S345P} (#4) and β-actin (#4, as control) is indicated.
**Figure 6F** is a histogram showing qPCR quantification of β-TrCP normalised to multiple reference genes expressed as percentage of control. ES cells were transfected with control (Luc), β-TrCP (1), Cdh1 (2) or Dub3 (3) RNAi sequences and collected 48 hours after transfection. Bars represent the mean ± SD of triplicate observations.
**Figure 6G** is a histogram showing qPCR quantification of Dub3 normalised to multiple reference genes expressed as percentage of control. ES cells were transfected with control (Luc), β-TrCP (1), Cdh1 (2) or Dub3 (3) RNAi sequences and collected 48 hours after transfection. Bars represent the mean ± SD of triplicate observations.
**Figure 6H** is a histogram showing qPCR quantification of Cdh1 normalised to multiple reference genes expressed as percentage of control. ES cells were transfected with control (Luc), β-TrCP (1), Cdh1 (2) or Dub3 (3) RNAi sequences and collected 48 hours after transfection. Bars represent the mean ± SD of triplicate observations.
**Figure 6I** is a histogram showing qPCR quantification of Cdc25A normalised to multiple reference genes expressed as percentage of control. ES cells were transfected with control (Luc), β-TrCP (1), Cdh1 (2) or Dub3 (3) RNAi sequences and collected 48 hours after transfection. Bars represent the mean ± SD of triplicate observations.
**Figure 6J** is a western blot analysis of Cdc25A protein expression in Luciferase (a.), β-TrCP (b.) and Cdh1 (c.) RNAi-transfected cells. Expression of Cdc25A (#1) and β-actin (#2) is shown.
**Figures 7A-F** show that elevated deubiquitylase Dub3 in ES cells increases Cdc25A abundance.
**Figure 7A** is a histogram showing the qPCR quantification of Oct4 (1), Cdc25A (2), Cdh1 (3), β-TrCP (4) and Dub3 (5) mRNA normalized to multiple reference genes in ES (white bars) and NIH-3t3 (black bars) cells. Data are expressed as mean ± SD (error bars) of multiple observations. Statistical differences is indicated with an asterisk *P* < 0.05. Left Y-axis represents the Oct4 mRNA expression and right Y-axis represent mRNA expression of the three other genes.

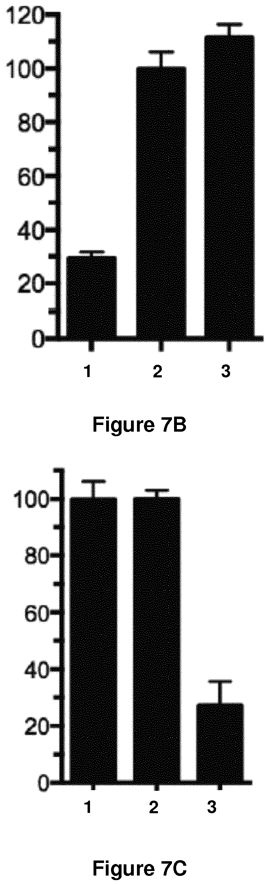

**Figure 7B** is a histogram showing the qPCR quantification of Dub3 mRNA normalised to multiple reference genes. ES cells were transfected with control (1), Dub3 (2) or Cdc25A (3) RNAi sequences.
**Figure 7C** is a histogram showing the qPCR quantification of Cdc25A mRNA normalised to multiple reference genes. ES cells were transfected with control (1), Dub3 (2) or Cdc25A (3) RNAi sequences.

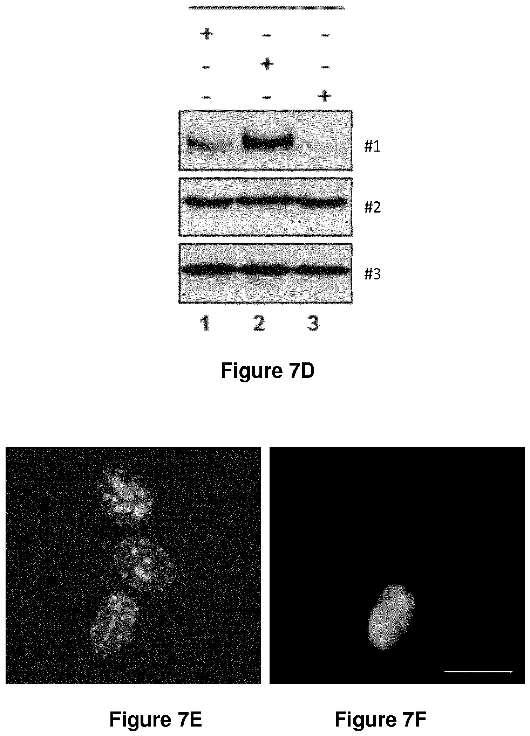

**Figure 7D** shows a Western blot analysis of ES cells transfected with Dub3 (column 1), (column 3) Cdc25A or control (column 2) RNAi sequences. Expression of CDC25A (#1), Cdc25C (#2) and β-actin (#3, as control) is represented.
**Figure 7E** represents nuclei of ES cells stained with DAPI.
**Figure 7F** represents cells indicating cellular localisation of pcDNA3-eGFP-Dub3 in ES cells. Scale bar represents 10 µM.

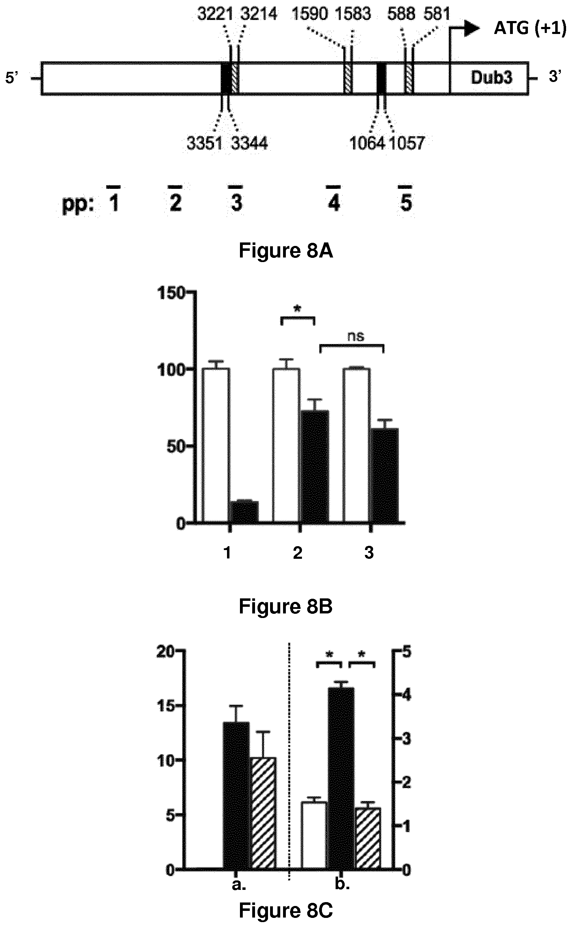

**Figures 8A-G** show that Dub3 is a target gene of the orphan receptor Esrrb.
**Figure 8A** is a schematic overview of the Dub3 proximal promoter in mouse (6kb). Esrrb (shaded boxes) and Sox2 (black boxes) consensus binding sites (RE) are indicated.
**Figure 8B** is a histogram representing qPCR quantification of Esrrb (1), Dub3 (2) and Nanog (3) mRNA normalised to multiple reference genes expressed as % of control. ES cells were transfected with control (Crtl) RNAi (white bars) or Esrrb specific RNAi sequence (black bars). Data are expressed as mean ± SD (error bars) of multiple observations. Statistical differences is indicated with a single asterisk (*) for *P* < 0.05, not significant is indicated as (ns).
**Figure 8C** is a histogram representing qPCR quantification of endogenous Esrrb (a.) or Dub3 (b.) expression in ES cells transfected with empty vector (white bars), Esrrb (black bars) or Esrrb-ΔCter (hatched bars) expressing plasmids. Data are expressed as mean ± SD (error bars) of multiple observations. Statistical differences is indicated with a single asterisk (*) for *P* < 0.05.

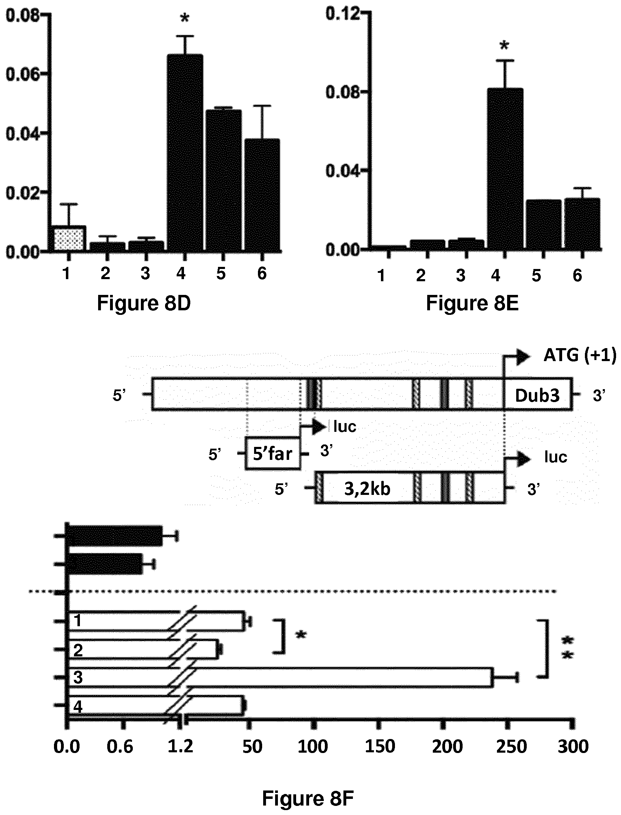

**Figure 8D** is a histogram representing ChIP of Esrrb on Dub3 promoter. Primer pair location along the 6 kb proximal promoter (Figure 8A) for scanning of Dub3 promoter for Esrrb and Sox2 occupancy. Data are expressed as mean ± SD (error bars) of multiple observations. Amylase serves here as a control. Statistical analysis using two-way ANOVA was performed. 1: amylase, 2: pp1, 3: pp2, 4: pp3, 5: pp4, 6: pp5.
**Figure 8E** is a histogram representing ChIP of Sox2 on Dub3 promoter. Primer pair location along the 6 kb proximal promoter (Figure 8A) for scanning of Dub3 promoter for Esrrb and Sox2 occupancy. Data are expressed as mean ± SD (error bars) of multiple observations. Amylase serves here as a control. Statistical analysis using two-way ANOVA was performed. 1: amylase, 2: pp1, 3: pp2, 4: pp3, 5: pp4, 6: pp5.
**Figure 8F** is a histogram showing Dub3 promoter activity using luciferase assay in CV1 cells. Cells were cotransfected with promoter construct and the indicated genes, and assessed for luciferase activity 48 hours post-transfection. Bars represent the fold induction ± SD of multiple observations. Statistical differences is indicated with a single asterisk (*) for *P* < 0.05 and (**) for *P <* 0.001. Black bars represents pGL4.10_5'far and white bars represents pGL4.10_3.2kb. 1: empty vector, 2: Sox2, 3: Essrb and 4: Δ-Cter.

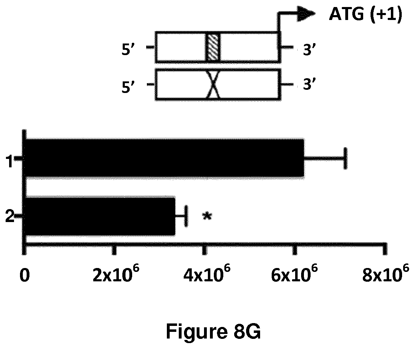

**Figure 8G** is a histogram showing basal transcriptional activity of a 1 kb proximal promoter and a mutated sequence in ES cells. Three mutations were introduced in the Esrrb consensus binding site. TCAAGGTCA was mutated to TCATTTTCA. Data are expressed as mean ± SD (error bars) of multiple observations. 1: wt, 2: mutated.

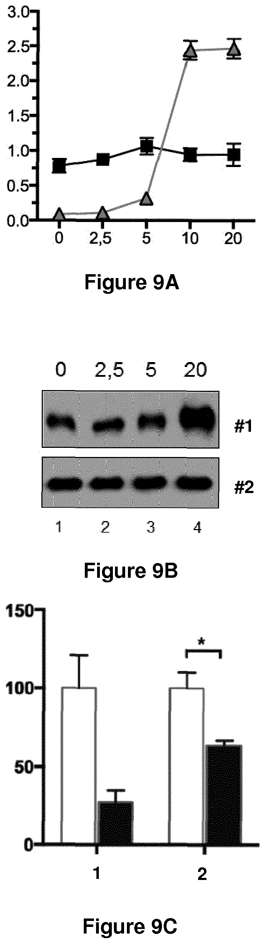

**Figures 9A-F** shows that Dub3 is a target gene of the orphan receptor Esrrb
**Figure 9A** is a graph showing qPCR quantification of Cdc25A (curves with black squares) and Dub3 (curves with triangles) mRNA in ES cells treated with increasing concentration of the selective Esrrb and Esrrg agonist DY131 (indicated doses in µM) for 16 hours. Bars represent the fold induction ± SD of triplicate observations.
**Figure 9B** is a western blot analysis of Cdc25A protein levels in ES cells treated with increasing concentrations (in µM) of the DY131 agonist for 16 hours. Cdc25A (#1) and β-actin (#2, as control) protein expression is represented.
**Figure 9C** is a histogram showing qPCR quantification of Esrrb (1) and Dub3 (2) mRNA normalised to multiple reference genes expressed as percentage of control in presence of DY131. ES cells were transfected with control (Crtl) RNAi (white bars) or Esrrb specific RNAi sequence (black bars). Data are expressed as mean ± SD (error bars) of multiple observations.

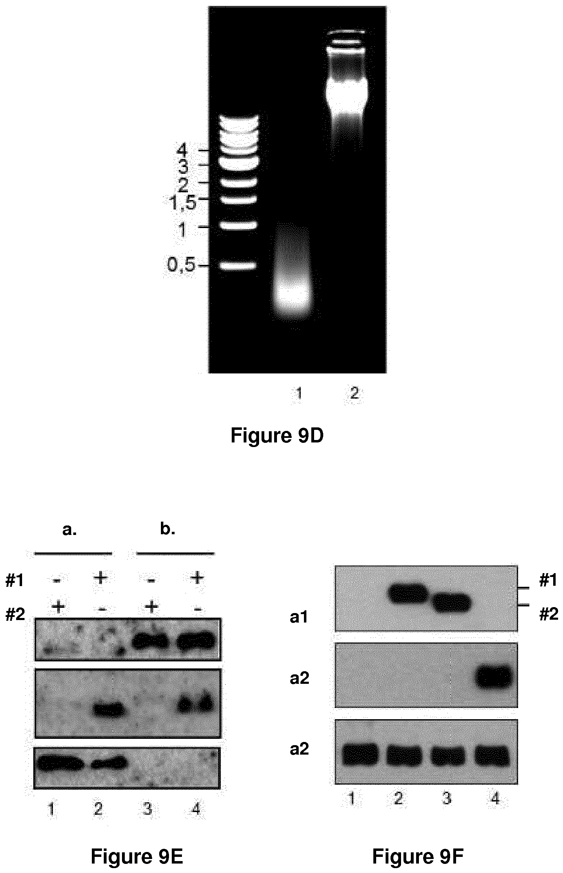

**Figure 9D** represents DNA fragments size prior to ChIP analysis. Sonication resulted to DNA fragments smaller than 500bp. 1: IP input, 2: genomic DNA.
**Figure 9E** is a western blot showing the specificity of the Esrrb antibody. Immunoprecipitation of 293T-HEK cells transfected with either empty vector (Ev; #2) or Flag-Esrrb (#1) expression plasmids. Immunoprecipitation was performed in parallel using either Flag or Esrrb (b.) antibody. Both antibodies specifically immunopreciptated Flag-Esrrb protein. a;: input. a1: IgGs, a2: Esrrb and a3: β-actin.
**Figure 9E** is a western blot analysis of expression levels of CV1 cells transfected with either empty vector (lane 1), Flag-Esrrb (lane 2), Flag-Esrrb-Δ-Cter (lane 3) or Sox2 (lane 4), 48 hours post-transfection. a1: FLAG, a2: Esrrb and a3: β-actin, #1: Esrrb and #2: Δ-Cter.

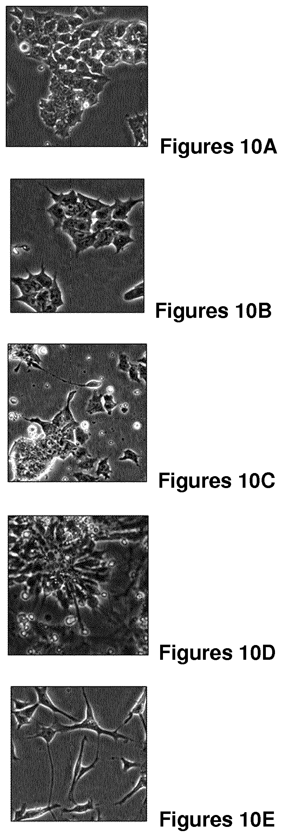

**Figures 10A-K** show Developmental regulation of Cdc25A protein abundance correlates with Dub3 expression.
**Figure 10A** is a phase-contrast photo of ES cells.
**Figure 10B** is a phase-contrast photo of N2B27-induced neural conversion of ES cells at day 1.
**Figure 10C** is a phase-contrast photo of N2B27-induced neural conversion of ES cells at day 3.
**Figure 10D** is a phase-contrast photo of N2B27-induced neural conversion of ES cells at day 7.
**Figure 10E** is a phase-contrast photo of N2B27-induced neural conversion of ES cells at neural differentiated state.

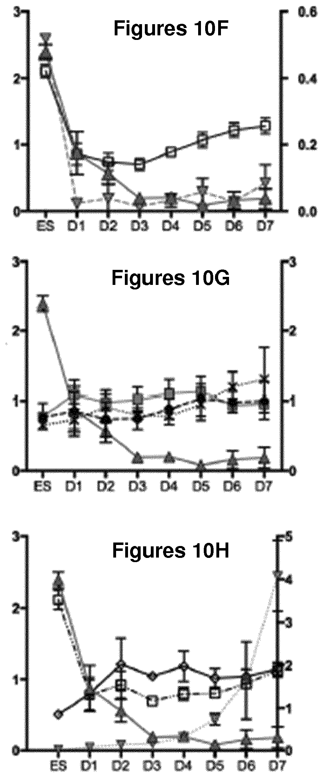

**Figure 10F** is a graph representing qPCR quantification of Dub3 (curve with triangles), Sox2 (curve with open squares) and Esrrb (curve with inversed triangles) mRNA normalised to multiple reference genes during N2B27-induced neural differentiation. Values represent mean ± SD of multiple observations.
**Figure 10G** is a graph representing qPCR quantification of Dub3 (curve with triangles), Cdc25A (curve with squares), Cdh1 (curve with circles) and β-TrCP (curve with crosses) mRNA normalised to multiple reference genes during N2B27-induced neural differentiation. Values represent mean ± SD of multiple observations.
**Figure 10H** is a graph representing qPCR quantification of Dub3 (curve with triangles), USP48 (curve with squares), USP13 (curve with diamonds) and USP29 (curve with inversed triangles) mRNA normalised to multiple reference genes during N2B27-induced neural differentiation. Values represent mean ± SD of multiple observations.

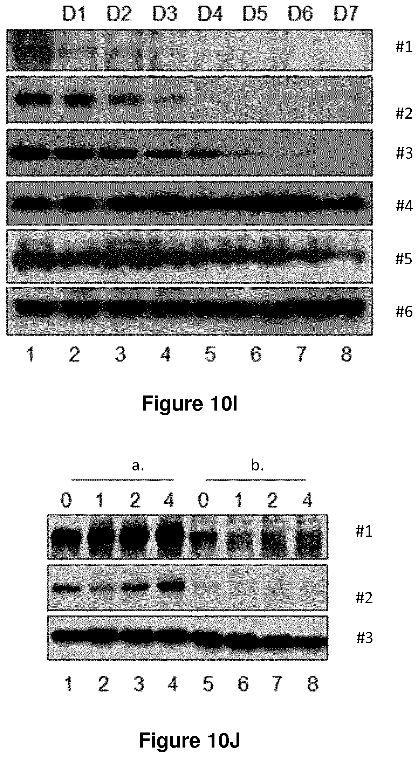

**Figure 10I** represents a western blot analysis of cell extracts collected throughout differentiation of ES cells into neural stem cells (NSC) immunoblotted Dub3 (#1), Oct4 (#2), Cdc24A (#3), RhoA (#4) Suds3 (#5) and β-actin (#6, as control) antibodies.
**Figure 10J** represents a western blot analysis of asynchronously growing ES (a.) and NSC (b.). Cells were exposed to 6 J/m2 UV-light and collected at indicated times. Expression of Cdc25A (#1, dark exposure and #2 light exposure) and β-actin (#3, as control) are represented.

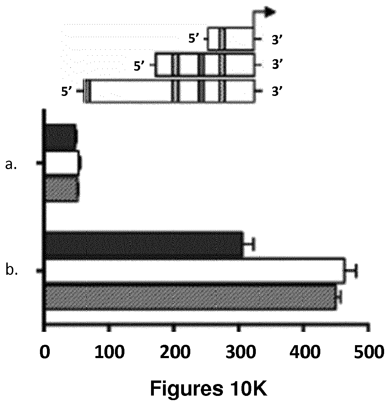

**Figure 10K** is a histogram representing the basal transcriptional activity of three different promoter lengths of the Dub3 gene in NIH-3t3 (a.) cells and ES (b.) cells. Data are expressed as mean ± SD (error bars) of multiple observations. Black bars: 1kb, white bars: 1,7kb and hatched bars : 3,2kb. X axis represents the Dub3 promoter activity expressed as luciferase fold induction.

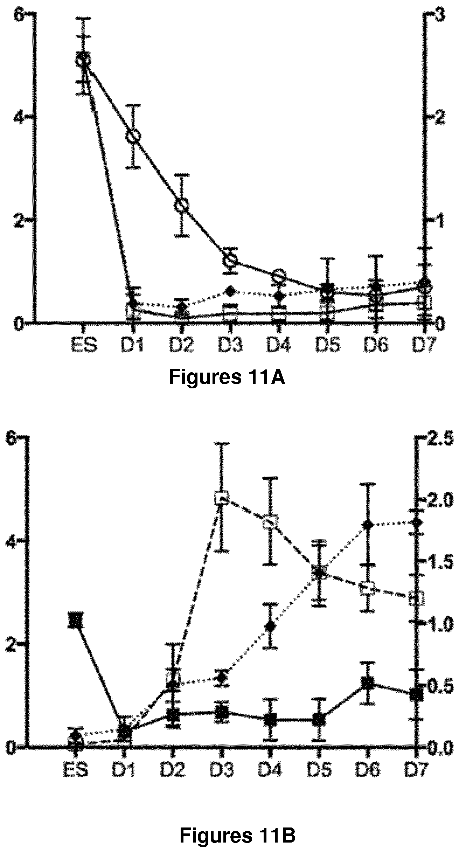

**Figures 11A-J** show that developmental regulation of Cdc25A protein abundance correlates with Dub3 expression levels.
**Figure 11A** is a graph showing qPCR quantification of pluripotency markers (Oct4 : curve with open circles, nanog: curves with black diamonds and Klf4: curve with open squares) during neural conversion of ES cells. Data were normalized to multiple reference genes. Data are expressed as mean ± SD (error bars) of multiple observations. Left Y-axis represents Nanog or Klf4 mRNA expression and right Y-axis represents Oct4 mRNA expression.
**Figure 11B** is a graph showing qPCR quantification of cell fate specification markers (Nestin: curve with diamonds, Sox7: curve with black squares and Sox1: curve with open suqares) during neural conversion of ES cells. Data were normalized to multiple reference genes. Data are expressed as mean ± SD (error bars) of multiple observations. Left Y-axis represents Sox7 expression and right Y-axis represents Sox1 or Nestin mRNA expression.
**Figure 11C** is an immunofluorescence detection of Nestin at day 1 of N2B27-induced differentiation. Nuclei were counterstained using DAPI. Scale bar 50 µM.

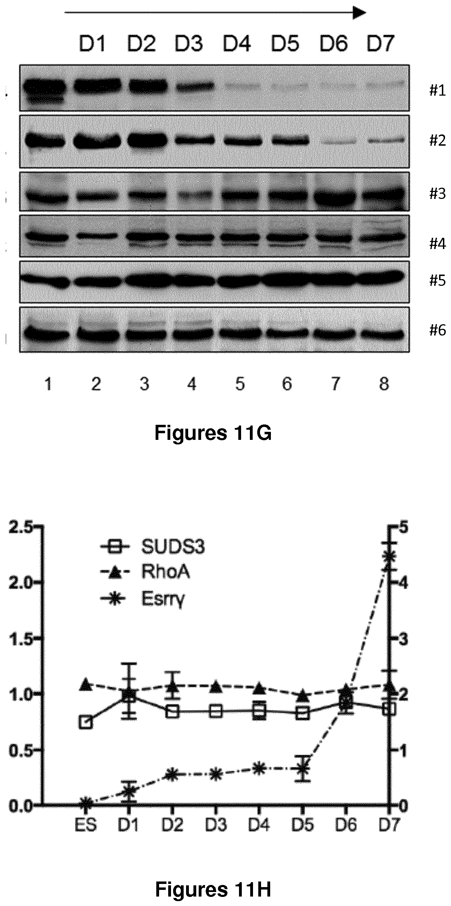

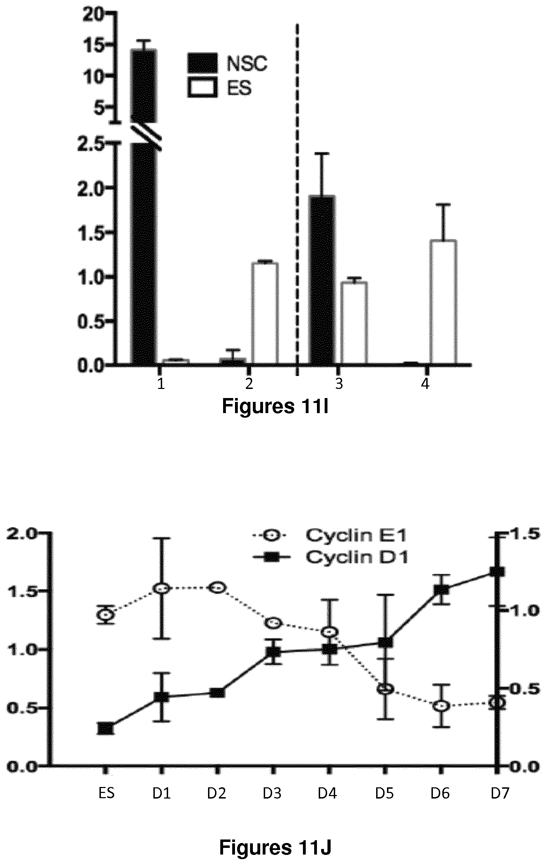

**Figure 11** **D** is an immunofluorescence detection of Nestin at day 6 of N2B27-induced differentiation. Nuclei were counterstained using DAPI. Scale bar 50 µM.
**Figure 11E** is a western blot showing specificity of the antibody raised against mouse Dub3. Human 293T cells were transfected with empty vector (EV; lanes 2 and 4) or HA-Dub3 expressing vectors (lanes 3 or 5). Cells were collected 24 hours post transfection and extracts were immunoblotted (IB) using pre-immune (PI; lane 1), Dub3 (lanes 2 and 3) or HA (lanes 4 or 4) antibodies. The Dub3 antibody recognizes a specific polypeptide of 60 kDa in SDS-PAGE (arrow) which is not recognized by the pre-immune serum.
**Figure 11F** is a western blot showing the validation of the antibody raised against mouse Dub3. Western blot analysis of ES cells transfected with control (lane 1) or Dub3 (lane 2) RNAi sequences. Cells were collected 48 hours post transfection and extracts were immunoblotted using Dub3 purified antibody (#1) and β-actin (#2).
**Figure 11G** is a western blot analysis of Dub3 substrates and other proteins (#1: Oct4, #2: Cdc25A, #3: Cdc25B, #4: Cdc25C, #5: PCNA and #6: β-actin) during neural conversion of N2B27 cells (from D1 to D7).
**Figure 11H** is a graph showing qPCR quantification of Suds3, RhoA and Esrr γ during neural conversion of ES cells. Data were normalized to multiple reference genes. Data are expressed as mean ± SD (error bars) of multiple observations.
**Figure 11I** is a histogram showing qPCR quantification of Nestin, Nanog, Cdc25A and Dub3 mRNA normalized to multiple reference genes in ES and Neural Stem Cells (NSC). Bars represent the mean ± SD of multiple observations.
**Figure 11J** is a graph showing qPCR quantification of G1 cyclin stoechiometry during neural conversion of ES cells. Data were normalised to multiple reference genes. Data are expressed as mean ± SD (error bars) of multiple observations. Left Y-axis represents Cyclin D1 expression and right Y-axis represents Cyclin E1 mRNA expression.

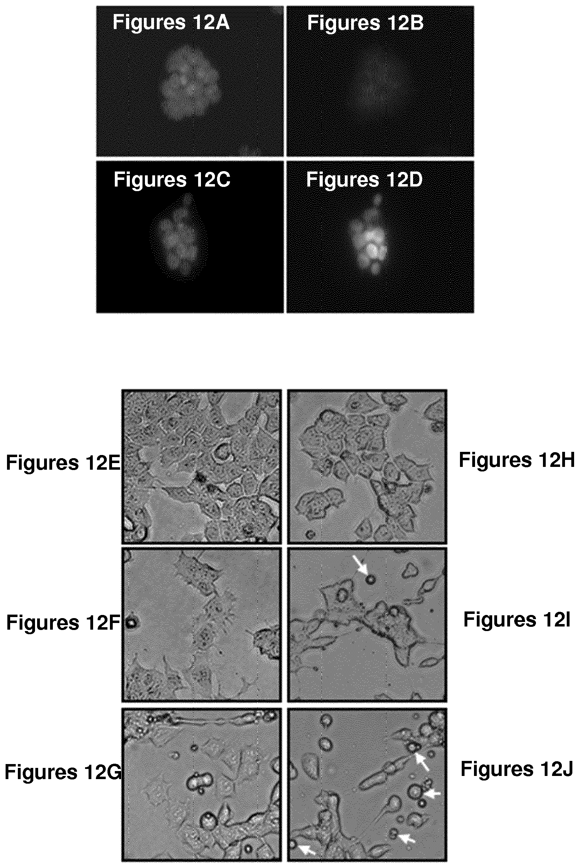

**Figures 12A-O** show that constitutive Dub3 expression leads to massive apoptosis concomitant to differentiation-induced cell cycle remodeling.
**Figure 12A** is a fluorescence detection of empty vector (EV)-expressing ES cells labeled by DAPI staining.
**Figure 12B** is an immunofluorescence detection of empty vector (EV)-expressing ES cells. eGFP expression is shown.
**Figure 12C** is a fluorescence detection of eGFP-Dub3-expressing ES cells labeled by DAPI staining.
**Figure 12D** is an immunofluorescence detection of eGFP-Dub3-expressing ES cells cells. eGFP expression is shown. All ES cells express eGFP-Dub3 at comparable levels.
**Figure 12E** is a phase-contrast photo of empty vector (EV) ES cells after LIF removal at the indicated day 0 of differentiation.
**Figure 12F** is a phase-contrast photo of empty vector (EV) ES cells after LIF removal at the indicated day 2 of differentiation.
**Figure 12G** is a phase-contrast photo of empty vector (EV) ES cells after LIF removal at the indicated day 4 of differentiation.
**Figure 12H** is a phase-contrast photo of eGFP-Dub3-expressing ES cells after LIF removal at the indicated day 0 of differentiation.
**Figure 12I** is a phase-contrast photo of eGFP-Dub3-expressing ES cells after LIF removal at the indicated day 2 of differentiation. Arrow indicates detached cells with apoptotic morphology.
**Figure 12J** is a phase-contrast photo of eGFP-Dub3-expressing ES cells after LIF removal at the indicated day 4 of differentiation. Arrows indicate detached cells with apoptotic morphology.

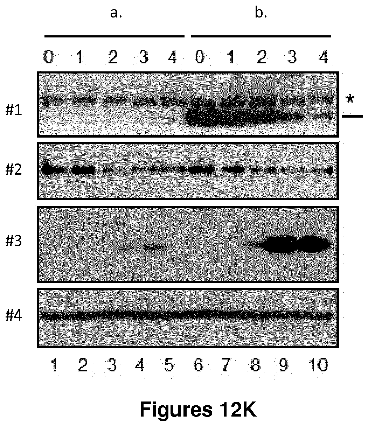

**Figure 12K** shows a western blot of cell extracts prepared every day after LIF withdrawal from empty vector (a.) or eGFP-Dub3-expressing ES cells (b.). (*) indicates a non-specific band. High caspase 3 activities in eGFP-Dub3 expressing cells indicate apoptosis. Expression of GFP-Dub3 (#1), Oct4 (#2), active caspase 3 (#3) and MCM2 (#4) is represented.

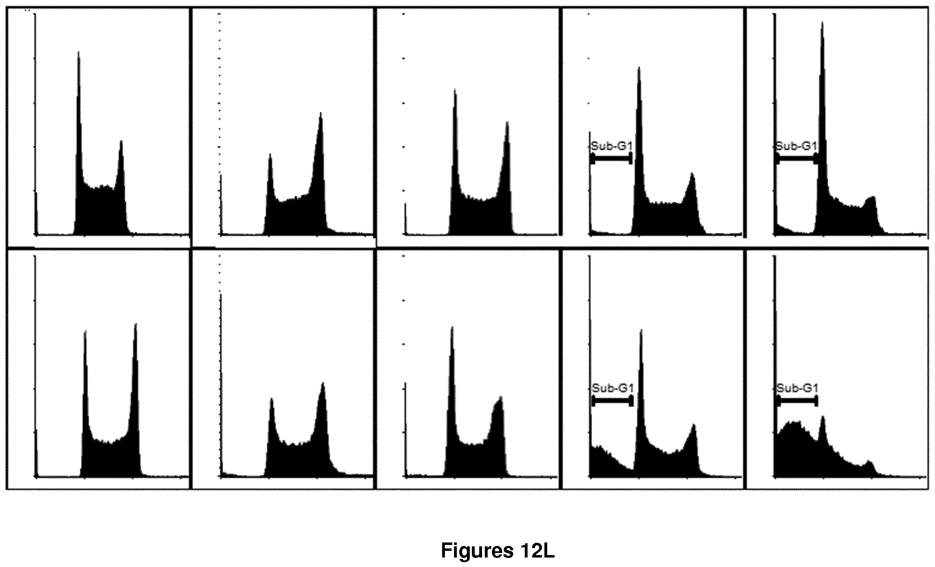

**Figure 12L** shows differentiation-induced cell cycle remodelling. Cells were collected at the indicated days and analyzed by FACS following propidium iodide staining. Cell death is illustrated by cells with subdiploid DNA content (Sub-G1). Upper lanes represents empty vector expressing cells and lower lane represents eGFP-Dub3 expressing cells. First column represents day 0 of differentiation, second column represents day 1 of differentiation, third column represents day 2 of differentiation, fourth column represents day 3 of differentiation and fifth column represents day 4 of differentiation.

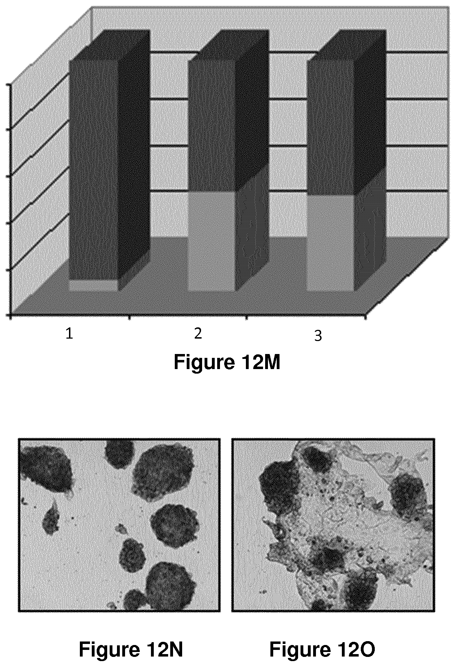

**Figure 12M** is a histogram showing a clonogenic assay of ES cells upon prolonged control (1), Dub3 (2) or Cdc25a (3) targeting RNAi sequence. Cells were plated at clonal density in LIF-containing serum and stained for AP after 7 days. Columns show the percentage of alkaline phosphatase (AP) positive (dark grey) or negative (light grey) colonies. At least 150 colonies were scored.
**Figure 12N** is a representative picture of cells transfected with control targeting RNAi sequence and assayed for AP activity.
**Figure 12O** is a representative picture of cells transfected with Dub3 targeting RNAi sequence and assayed for AP activity.

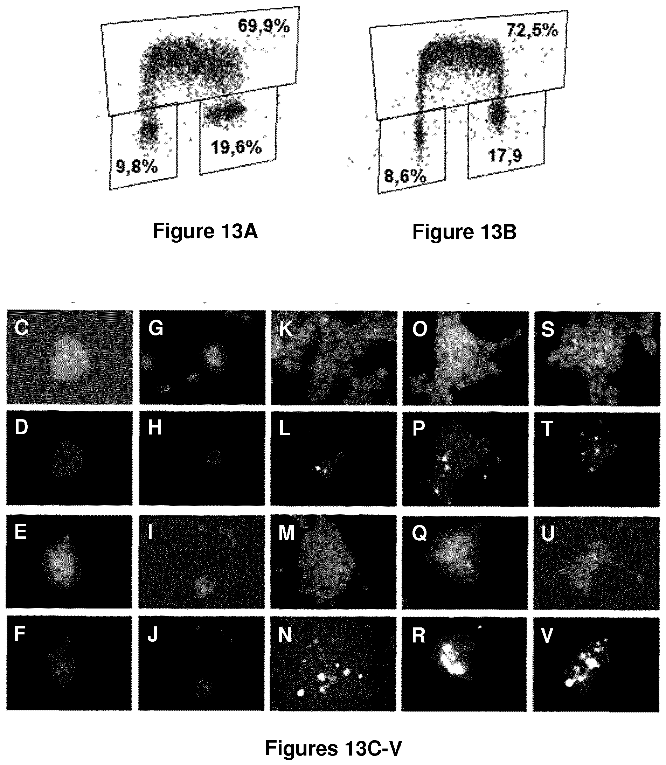

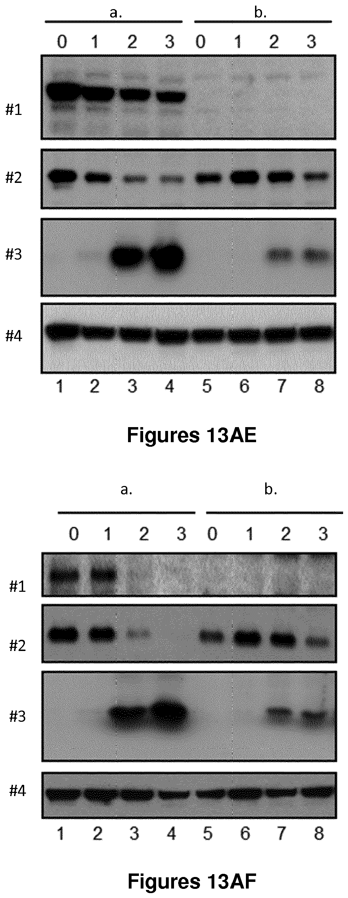

**Figures 13A****-AF** shows that constitutive Dub3 expression leads to massive apoptosis concomitant to differentiation-induced cell cycle remodeling.
**Figure 13A** shows cell cycle distribution and BrdU incorporation of empty vector expressing ES cells analyzed by FACS.
**Figure 13B** shows cell cycle distribution and BrdU incorporation of eGFP-Dub3 expressing ES cells analyzed by FACS.
**Figure 13C** is an immunofluorescence detection of DNA during LIF withdrawal in empty vector expressing ES cells at day 0 of differentiation.
**Figure 13D** is an immunofluorescence detection of active caspase 3 LIF withdrawal in empty vector expressing ES cells at day 0 of differentiation.
**Figure 13E** is an immunofluorescence detection of DNA during LIF withdrawal in eGFP-Dub3 expressing ES cells at day 0 of differentiation.
**Figure 13F** is an immunofluorescence detection of active caspase 3 LIF withdrawal eGFP-Dub3 expressing ES cells at day 0 of differentiation.
**Figure 13G** is an immunofluorescence detection of DNA during LIF withdrawal in empty vector expressing ES cells at day 1 of differentiation.
**Figure 13H** is an immunofluorescence detection of active caspase 3 LIF withdrawal in empty vector expressing ES cells at day 1 of differentiation.
**Figure 13I** is an immunofluorescence detection of DNA during LIF withdrawal in eGFP-Dub3 expressing ES cells at day 1 of differentiation.
**Figure 13J** is an immunofluorescence detection of active caspase 3 LIF withdrawal eGFP-Dub3 expressing ES cells at day 1 of differentiation.
**Figure 13K** is an immunofluorescence detection of DNA during LIF withdrawal in empty vector expressing ES cells at day 2 of differentiation.
**Figure 13L** is an immunofluorescence detection of active caspase 3 LIF withdrawal in empty vector expressing ES cells at day 2 of differentiation.
**Figure 13M** is an immunofluorescence detection of DNA during LIF withdrawal in eGFP-Dub3 expressing ES cells at day 2 of differentiation.
**Figure 13N** is an immunofluorescence detection of active caspase 3 LIF withdrawal eGFP-Dub3 expressing ES cells at day 2 of differentiation.
**Figure 13O** is an immunofluorescence detection of DNA during LIF withdrawal in empty vector expressing ES cells at day 3 of differentiation.
**Figure 13P** is an immunofluorescence detection of active caspase 3 LIF withdrawal in empty vector expressing ES cells at day 3 of differentiation.
**Figure 13Q** is an immunofluorescence detection of DNA during LIF withdrawal in eGFP-Dub3 expressing ES cells at day 3 of differentiation.
**Figure 13R** is an immunofluorescence detection of active caspase 3 LIF withdrawal eGFP-Dub3 expressing ES cells at day 3 of differentiation.
**Figure 13S** is an immunofluorescence detection of DNAduring LIF withdrawal in empty vector expressing ES cells at day 4 of differentiation.
**Figure 13T** is an immunofluorescence detection of active caspase 3 LIF withdrawal in empty vector expressing ES cells at day 4 of differentiation.
**Figure 13U** is an immunofluorescence detection of DNA during LIF withdrawal in eGFP-Dub3 expressing ES cells at day 4 of differentiation.
**Figure 13V** is an immunofluorescence detection of active caspase 3 LIF withdrawal eGFP-Dub3 expressing ES cells at day 4 of differentiation.

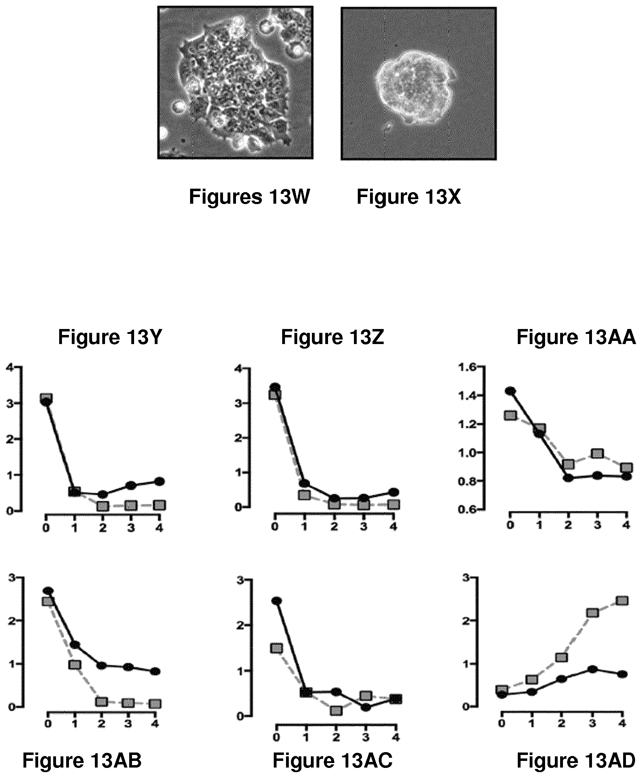

**Figure 13W** is a phase contrast photo of empty vector expressing cell-lines.
**Figure 13X** is a phase contrast photo of eGFP-Dub3 expressing cell-lines.
**Figure 13Y** is a graph showing qPCR quantification of Nanog normalised to multiple reference genes during LIF withdrawal (X-axis, in day). Curve with circles: empty vector, curve with squares: GFP-Dub3 expressing cells.
**Figure 13Z** is a graph showing qPCR quantification of Klf4 normalised to multiple reference genes during LIF withdrawal. Curve with circles: empty vector, curve with squares: GFP-Dub3 expressing cells.
**Figure 13AA** is a graph showing qPCR quantification of Oct4 normalised to multiple reference genes during LIF withdrawal. Curve with circles: empty vector, curve with squares: GFP-Dub3 expressing cells.
**Figure 13AB** is a graph showing qPCR quantification of Rex1 normalised to multiple reference genes during LIF withdrawal. Curve with circles: empty vector, curve with squares: GFP-Dub3 expressing cells.
**Figure 13AC** is a graph showing qPCR quantification of Sox7 normalised to multiple reference genes during LIF withdrawal. Curve with circles: empty vector, curve with squares: GFP-Dub3 expressing cells.
**Figure 13AD** is a graph showing qPCR quantification of Noxa normalised to multiple reference genes during LIF withdrawal. Curve with circles: empty vector, curve with squares: GFP-Dub3 expressing cells.
**Figure 13AE** is a western blot analysis of cell extracts collected every day throughout the N2B27-induced differentiation process of empty vector (b.) or eGFP-Dub3 (a.) expressing ES cells into NSCs. Four days after N2B27-mediated differentiation all eGFP-Dub3 expressing cells were all dead by apoptosis as indicated by high caspase 3 activities. Expression of GFP-Dub3 (#1), Oct4 (#2), active caspase 3 (#3) and MCM2 (#4) is represented.
**Figure 13AE** is a western blot analysis of cell extracts collected every day throughout the differentiation process of empty vector or HA-Dub3 expressing ES cells into NSCs.

The molecular and cellular phenotype of HA-Dub3 expressing cells was highly comparable to the eGFP-Dub3 expressing cells indicating that the phenotype is independent of the N-terminal tag. Expression of HA-Dub3 (#1), Oct4 (#2), active caspase 3 (#3) and MCM2 (#4) is represented.

### Examples

### Example 1 : Experimental Procedures

### 1- Cell extracts, western blotting and antibodies

Cells were rinsed once in PBS and then incubated with ice cold lysis buffer (50 mM Tris-HCI pH 7.4, 100 mM NaCl, 50 mM NaF, 5 mM EDTA, 40 mM β-glycero-phosphate, 1% Triton X-100 and protease inhibitors) for 30 min on ice before scraping. Whole cell extracts were clarified by centrifugation at 12000 rcf for 10 min at 4°C. Protein concentration of the clarified lysates was estimated using BCA method (Pierce). Equal amount of protein was used for western blot analysis. All antibodies were incubated overnight at 4 °C in phosphate-buffered saline (PBS) containing 1% BSA and 0,1% Tween (Sigma). Antibodies used from Cell Signaling: Chk1S345P (2341), p53S15P (9284), γH2AX (2577), CDK2Y15P (9111), Myc-Tag (2276); Active caspase 3 (9961); Abcam: DNA polα (ab31777), H3 (ab1791), CDK2 (ab6538), PSTAIR (ab10345), GFP (ab290), MCM2 (ab4461); Suds3 (ab3740) Santa Cruz: Cdc45 (sc-20685), Cdc25A (sc-7389), Chk1 (sc-8408), Cyclin B1 (sc-245), Cdc25C (sc-327), Cdc25B (sc-65504), p21 (sc-6246), RhoA (sc-418); anti-goat IgG-HRP (sc-2020) Sigma: (PC10), β-actin (A1978), Cyclin A (C7410), Anti-Flag M2 (F1804), Oct4 (Chemicon, AB3209), and Millipore, Nestin (Ab353), H3S10P (Millipore 09-797). Wee1 (kindly provided by T. Lorca, CRBM Montpellier).

Mouse Dub3 polyclonal antibodies were raised by immunizing rabbits with a synthetic peptide (NH2-MSPGQLCSQGGR-COOH SEQ ID NO: 39) designed from mouse Dub3 C-terminus, coupled to keyhole limpet hemocyanin (KLH). Antibodies were purified by coupling the Dub3 peptide on HiTrap NHS-activated HP columns (GE Healthcare).

### 2- Cell culture and transfection

ES cells (CGR8) were cultured on gelatin-coated dishes in the absence of feeder cells with 1,000 U LIF per ml (Millipore). Cells were grown in a humidified atmosphere of 5% CO2 at 37°C. For transient expression both NIH-3t3 and ES cells were transfected using X-tremeGENE 9 DNA (Roche), and CV1 with JetPEI (Polyplus), according to manufacturer's directions. For infection, retroviral particles were generated by transfecting Platinum-E ecotropic packaging cell line with retroviral expression vector (pLPC) encoding Myc6-Dub3 variants using home-made PEI reagent.

Briefly, ES cells were maintained in Glasgow MEM BHK-21 (GMEM) supplemented with 10% fetal bovine serum, non-essential amino acids, L-glutamine, sodium pyruvate, β-mercapthethanol. NIH-3t3 cells were maintained in Dulbecco's modified eagle's medium (DMEM) supplemented with 10% fetal bovine serum, 2 mM glutamine and antibiotics. The viruses-containing conditioned medium was incubated on exponentially growing NIH-3t3 cells for 24 hours in the presence of polybrene (10 mg/mL). 48 hours post-infection, cells were selected in puromycin (2.5 µg/mL)-containing medium for 8-10 days before use. Reverse transfection of ES cells was performed using INTERFERin (Polyplus) according to manufacturer's directions. Cells were collected 24, 36 or 48 hours after transfection for analysis. The Cdc25A RNAi sequence was :
5'-GAAAUUUCCCUGACGAGAA-3' SEQ ID NO: 40,

The Dub3 RNAi sequence was :
5'-GGCUGUAAGAUGUGUGCUA-3' SEQ ID NO: 41
and a Esrrb previously described in Feng et al., 2009, Nat Cell Biol 11, 197-203. RNAi for Cdh1 and β-TrCP knockdown were purchased from Darmacon (SMARTpool) 57371 (Cdh1) and 12234 (β-TrCP).

### 3- Cell synchronization

ES cells were arrested in prometaphase by nocodazole (Sigma) for 4-8 hours. After mitotic-shake off cells were washed 3 times in ice-cold PBS and dissolved in full ES growth medium. Cells were incubated in a humidified atmosphere of 5% CO2 at 37°C for 45 minutes and placed at 30°C for 1 hour to reduce S phase entry. Cells were mock- or UV-irradiated (6 J/m2) and incubated at 37°C prior collection. To synchronise NIH-3t3 cells in G0 cells were grown to confluence and incubated for 2-3 days. Next, cells were washed, resuspended and split at 30% confluency. Six hours after release, cells were UV-irradiated.

### 4- UV-induced DNA Damage and Drugs

UV-C irradiation at 254nm was performed with microprocessor-controlled crosslinker (BIO-LINK®) or with a UV-lamp (Hanovia). Cycloheximide and DY131 (GW4716) were from Sigma and Chk1 inhibitor SB218078 from Calbochiem.

### 5- Flow cytometry

Single-cell suspensions were prepared by trypsinisation and washed once in PBS. Cells were fixed in ice-cold 70% ethanol (-20°C) and stored at -20 °C overnight. Following RNAse A treatment, total DNA was stained with propidium iodide (25 µg/ml). For BrdU uptake analysis, ES cells and NIH-3t3 cells were grown in the presence of 10 µM BrdU for respectively 10 and 30 minutes. The BrdU content was determined by reaction with a fluorescein isothiocyanate (FITC)-conjugated anti-BrdU antibody (BD Biosciences). Cells were analyzed with a FACScalibur flow cytometer using CellQuestPro software.

### 6- RNA extraction, reverse transcription and quantitative real-time PCR

Total RNA was isolated with TRIzol reagent (Invitrogen). Reverse transcription was carried out with random hexanucleotides (Sigma) and Superscript II First-Strand cDNA synthesis kit (Invitrogen). Quantitative PCRs were performed using Lightcycler SYBR Green I Master mix (Roche) on Lightcycler apparatus (Roche). All primers used were intronspanning (primer sequences available upon request). The relative amount of target cDNA was obtained by normalisation using geometric averaging of multiple internal control genes (ACTB, HPRT, HMBS, GAPDH, SDHA).

### 7- Chromatin Immunoprecipitation

ES cells were formaldehyde cross-linked and sonicated using a Misonix sonicator S-4000. Cells were lysed in ice-cold lysis buffer (Supplemental Information). Primer pairs for promoter scanning (6 kb upstream of transcription start site, TSS) of the Dub3 murine promoter were designed approximately every 1 kb.

Cells were lysed in ice-cold lysis buffer (50 mM Tris-HCI pH 7.4, 100 mM NaCl, 50 mM NaF, 5 mM EDTA, 40 mM β-glycero-phosphate, 1% SDS, 1% Triton X-100 and protease inhibitors) for 30 min on ice. Immuoprecipitation was performed by adding 5 µg Esrrb (Sigma SAB2100715), Sox2 (Bethyl A301-739) or control antibodies (Peprotech 500-P00) to lysates and incubation with rotation overnight at 4 °C. BSA and salmon sperm-blocked Protein A-Sepharose (Amersham) beads were added to the lysate.

### 8- Monolayer differentiation of ES cells into neurectodermal precursors

ES cells were dissociated and plated in N2B27 medium onto 0.1% gelatine-coated dishes at a density of 1.10⁴ cells/cm². N2B27 medium is a 1:1 mixture of DMEM/F12 (Gibco) supplemented with modified N2 (25 µg/ml insulin, 100 µg/ml apo-transferrin, 6 ng/ml progesterone (Sigma), 16 µg/ml putrescine (Sigma), 30 nM sodium selenite (Sigma), 50 µg/ml bovine serum albumine (Gibco), Neurobasal medium supplemeted with B27 (Gibco), β-mercaptoethanol (0.1 mM) and glutamate (0.2 mM) was also added. The medium was replaced every two days until day 7.

### 9- Isolation and amplification of NSC cells from CGR8 ES cells

ES cells were induced to differentiate into NSC following the protocol described above. At day 6, cells were dissociated in 0.01% Trypsine-EDTA and plated onto Poly-L-Ornithine/Laminin coated dishes in DMEM/N2 medium with 10 ng/ml of both EGF and bFGF (Biosource). For the preparation of Poly-L-Ornithine/Laminin plates, a 0.01% solution of poly-L-ornithine (Sigma) was added to plates for at least 20 min. The solution was removed and plates were washed 3 times with PBS. A 1 µg/ml solution of laminin in PBS (Sigma) was then applied and incubated at 37°C for at least 3 hrs. Cells can then be cultivated and amplified under these conditions for several subpassages without loosing neural stem cells properties.

### 10- Establishment of a monoclonal eGFP-Dub3 expressing ES cells

Wild-type ES cells were transfected with pcDNA3-eGFPDub3, plated at clonal density and selected with G418 (Sigma). eGFP-Dub3 positive clones were expanded in continuous presence of G418 and validated by immunofluorescence and western blotting.

### 11- Plasmids

The murine Dub3 gene (Gene ID: 625530) was amplified by PCR and cloned into pLPC-Myc6, pcDNA3-GFP and pcDNA3-HA. All constructs were verified by DNA sequencing. Mouse Esrrb (pSG5FI-mEsrrb) and the C-terminal truncated pSG5FI-mEsrrb-ΔCter were previously described. Genomic sequences of the Dub3 promoter were amplified by PCR and inserted into pGL4.10 vector (Promega) for luciferase activity. pCEP4-Sox2 was a kind gift of F. Poulat (IGH-CNRS).

### 12- Luciferase assay

ES cells were transfected with following reporter constructs, pG13-luciferase, p21-luciferase and p21-ΔREp53-luc (kindly provided by J. Basbous, IGH, Montpellier). A Renilla luciferase plasmid was cotransfected as an internal control. Cells were harvested 24 hours after transfection and mock or UV-irradiated. Six hours following UV-induced DNA damage, cells were harvested and the luciferase activities of the cell lysates were measured using the Dual-luciferase Reporter Assay system (Promega). The proximal promoter of 1kb upstream ATG start codon was inserted into pGL4.10 plasmid. Three mutations of the Esrrb consensus binding site (TCAAGGTCA) were introduced by PCR to generate a mutated binding site (TCATTTTCA). All constructs were sequence verified.

### 13- Immunofluorescence microscopy

For Nestin, Oct4 and active caspase 3 staining staining, cells were fixed in 4% paraformaldehyde and permeabilized with 0,1% Triton X-100. After fixation, cells were blocked in 3% BSA PBS-Tween and incubated overnight with antibody. The slides were mounted using Prolong Gold with DAPI (Invitrogen). For determination of the cellular localisation of Dub3, mouse ES cells were transfected with pcDNA-GFP-Dub3 and directly fixed. All slides were analysed using a Leica DM6000 epifluorescence microscope. Images were acquired using a Coolsnap HQ CCD camera (Photometries) and the metamorph software (Molecular Devices).

### 14- Subcellular fractionation experiments

Chromatin-enriched and soluble fractions were prepared using CSK-extraction procedure. Briefly, pelleted cells were lysed in CSK buffer (10 mM PIPES pH 6.8, 100 mM NaCl, 300 mM sucrose, 1 mM EGTA, 1 mM MgCl₂, 0.5 mM DTT, 1 mM ATP, 0.2% Triton X-100 and protease inhibitors) for 10 min on ice. After centrifugation at 3000 rpm for 3 min at 4 °C, the supernatant (Triton-soluble fraction) was recovered and the pellet (Triton-insoluble fraction) was resuspended in CSK buffer and incubated for 10 min on ice. After centrifugation, the pellet (chromatin-enriched fraction) was resuspended in Laemmli Buffer. Equivalent amount of soluble and chromatin fractions were analyzed by immunoblotting.

### 15- Statistical analysis

Two-way ANOVA or Student t-test were used to evaluate differences between groups using Prism software (GraphPad Software). P < 0.05 was considered significant and indicated with *, P < 0.001 was indicated with **.

### Example 2: Experimental Results

### ES cells arrest in early S phase upon induction of DNA damage in G1

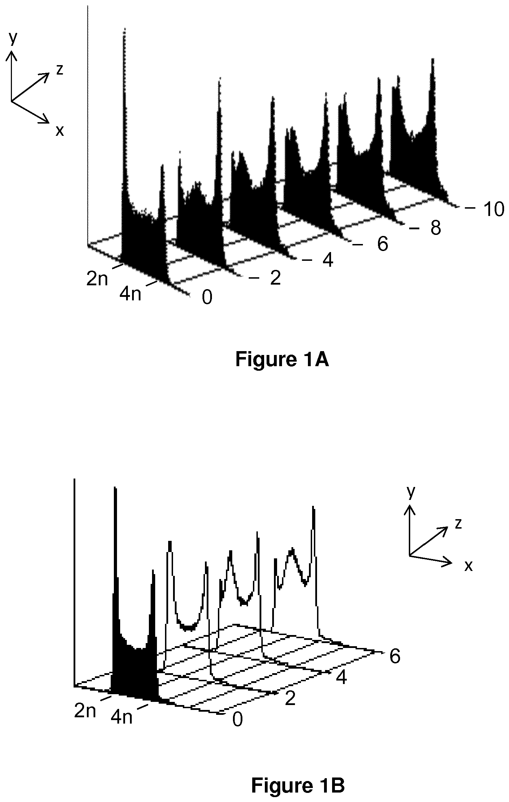

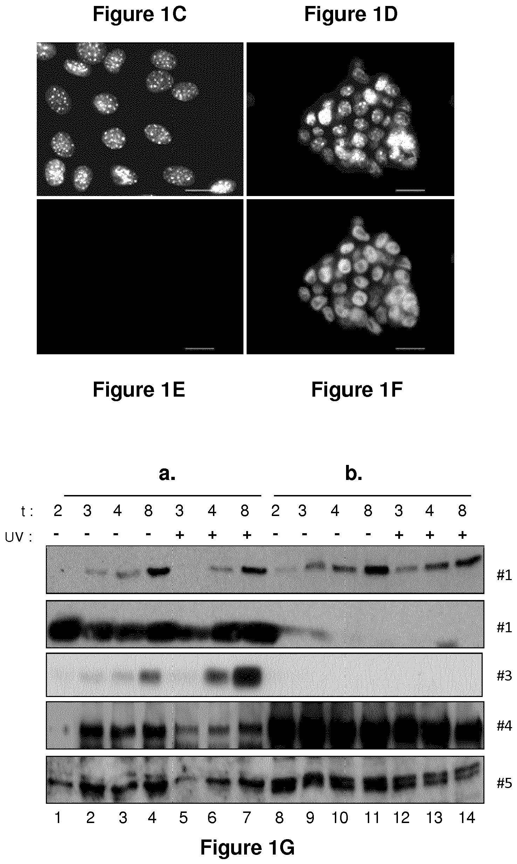

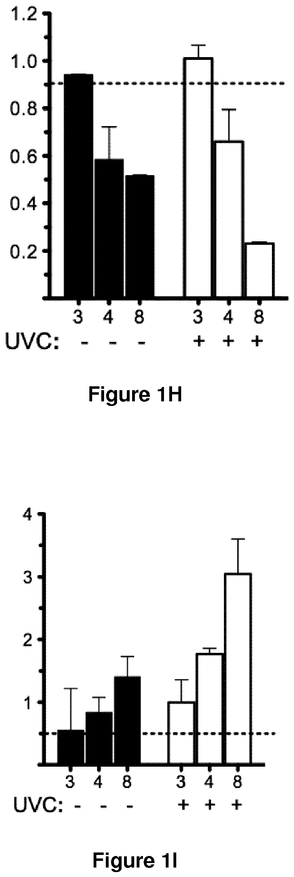

Circumstantial data suggest an impaired G1/S checkpoint in ES cells. The inventors observed that irradiation of ES cells with increasing doses of UV light induced a decrease in the number of G1 cells (**Figure 1A**). Time course analysis with a single UV dose (6 J/m2) resulted in cell cycle delay at the G1/S boundary (**Figure 1B****,** t=2). The inventors pulse-labelled nocodazole synchronized cells with BrdU (a nucleotide analogue) to allow exact distinction between late G1 (BrdU-negative) and early S-phase (BrdU-positive, **Figure 2A**). While analysis of total DNA content suggests a G1 arrest (**Figure 2B**), analysis of BrdU incorporation revealed that both untreated (Mock) and UV-irradiated cells (+ UV) entered S phase with very similar kinetics (**Figure 2C-D**). In contrast, synchronized mouse embryonic fibroblasts (NIH-3t3), which are Oct4-negative differentiated cells (**Figure 1C-F**), did not progress to S phase after UV irradiation in G1 (**Figure 2E**), in line with the presence of a stringent G1/S checkpoint. The inventors noticed that upon UV irradiation, BrdU incorporation was slightly reduced compared to mock-irradiated cells, confirmed by calculating the mean fluorescent signal of BrdU-positive cells (**Figure 1E****,** green boxes), and suggesting DNA synthesis slowdown in very early S phase. Analysis of chromatin-bound proteins shows that recruitment of both Cdc45 and DNA polymerase-a, two replication fork-associated factors, was considerably reduced upon UV irradiation, but not abolished (**Figure 1G****,** compare lanes 2-4 with 5-7), suggesting activation of the S phase checkpoint preventing late replication origins firing. Consistent with this possibility, phosphorylated H2AX histone variant (γH2AX), an ATR substrate, accumulated onto chromatin. Moreover UV-induced DNA damage did not significantly change the transcriptional program driven by E2F transcription factors required for S phase entry, as monitored by Cyclin A2 and E1 production (**Figure 1H-I**). The inventors also observed UV damage-dependent p53 phosphorylation on chromatin (**Figure 3A**), and transactivation (amongst other) of p21 gene expression (**Figures 3B-D**), demonstrating a functional p53 transcriptional response.

Persistent high levels of Cdc25A in ES cells sustain G1/S checkpoint bypass Cdc25A functions as a critical CDK2 regulator by removing an inhibitory phosphorylation on Tyrosine 15 (CDK2^{Y15P}) that in turn regulates S phase progression. The inventors compared Cdc25A and CDK2 protein abundance between ES cells and NIH-3t3 cells (**Figure 4A**). Strikingly, while CDK2 abundance is marginally higher in ES cells, the levels of Cdc25A in asynchronously growing ES cells are exceedingly high compared to NIH-3t3 cells. As expected, upon UV-induced DNA damage, Cdc25A was degraded in both cell lines (**Figure 4A**). However, one hour after irradiation, Cdc25A level remained about 4-fold higher in ES cells compared to unperturbed NIH-3t3 cells (lanes 1 and 7 and **Figure 5A**), indicating that high levels of Cdc25A persist even upon UV-induced DNA damage. Since cell cycle distribution of asynchronously growing ES and NIH-3t3 cells is different, the inventors analysed Cdc25A abundance in synchronized cells (**Figure 4B**). The inventors observed that in G1, ES cells contained about 7-fold more Cdc25A protein than NIH-3t3 cells (lanes 3 and 11 and **Figure 5B**). Proteolysis of Cdc25A mediated by the E3 ubiquitin ligase APC^{Cdh1} occurs at mitotic exit. Polyubiquitylated forms appear as a polypeptide ladder of higher molecular weight than the unmodified protein. In NIH-3t3 cells synchronized in G1 and S phase, the inventors could observe such ladders by western blot using a specific Cdc25A antibody (**Figure 4B****,** dark). Strikingly, in synchronized ES cells, these isoforms are much less abundant, whereas levels of unmodified Cdc25A are 7-fold higher than in NIH-3t3 cells (**Figure 5B**). Cdc25A immunoprecipitation from either ES or NIH-3t3 cells cotransfected with GFP-Cdc25A and HA-tagged ubiquitin, confirmed the presence of much more Cdc25A polyubiquitylated forms in NIH-3t3 than in ES cells (**Figure 4C**).

Next the inventors tested whether incomplete Cdc25A degradation may be due to impaired function of the ATR-Chk1 pathway. To this end, the inventors treated cells with a Chk1 inhibitor and analyzed Cdc25A protein levels upon UV irradiation. In contrast to a previous report in which degradation of Cdc25A was not affected by both Chk1 and Chk2 inhibitors, the inventors observed that Cdc25A degradation in ES cells is entirely dependent on Chk1 activity (**Figure 4D**).

Treatment of asynchronously growing ES cells with roscovitine (a selective CDKs inhibitor) induced dose-dependent increase of G1 cells and reduced the fraction of S phase cells (**Figure 5C**), demonstrating that, similar to somatic cells, in ES cells CDK activity is necessary for the G1/S transition. Inhibitory CDK2^{Y15} phosphorylation is mediated by Wee1 kinase and relieved through dephosphorylation by Cdc25A. The inventors therefore analysed changes in protein level of Wee1, Cdc25A, and CDK2Y15P during G1/S transition in ES cells, which, according to BrdU uptake experiments, occurs between 2-3 hours after nocodazole release (**Figure 2C**). Mitotic exit was monitored by histone H3 phosphorylation at serine 10 (H3^{S10P}), and S phase entry by H3 and Cyclin A production. Interestingly, Wee1 levels did not show significant cell cycle-dependent variations, while Cdc25A levels decreased and inversely correlated with CDK2^{Y15P} abundance (**Figures 5D-E**), suggesting that in ES cells, cell cycle-dependent fluctuation of Cdc25A levels may specifically regulate CDK2^{Y15P}.

To further pinpoint the specific role of Cdc25A in the G1/S checkpoint, the inventors examined whether interfering with Cdc25A levels by RNAi affects S-phase entry upon DNA damage (**Figures 5F-G**). To avoid undesired differentiation of ES cells due to G1 phase extension upon Cdc25A downregulation that would interfere with the interpretation of this experiment (see below and **Figures 12E-F**), knockdown was performed over a short period (24 hours). Interestingly, Cdc25A knockdown (**Figure 4E**) resulted in a significant, UV-dependent, increase of BrdU-negative cells with 2N DNA content (**Figure 4F**) mirrored by increased CDK2^{Y15P} levels (**Figure 4E**, compare lane 3 with lane 6 and **Figure 5H**). Importantly, the slight increase of CDK^{2Y15P} levels between 2 and 4 hours after release (**Figure 4E**, lane 3), also observed in synchronized undamaged cells entering S-phase (**Figure 5D**), did not result in an apparent difference in S phase entry in mock and UV-treated cells transfected with control RNAi (**Figure 4F**). Altogether, these data show that ES cells contain high levels of Cdc25A and that its knockdown leads to a UV-dependent G1 delay.

### ES cells express high Dub3 deubiquitylase

Elevated Cdc25A protein levels can be explained by increased gene expression, increased translation or reduced protein degradation. The inventors analysed protein turnover in the presence of cycloheximide to inhibit de novo protein synthesis (**Figures 6A-C**). Using this approach, the inventors found a 3-fold longer half-life of Cdc25A in ES cells (t_{1/2}= 24 min) compared to NIH-3t3 cells (t_{1/2}= 8 min). Of note, since unsynchronized cells were used, the inventors cannot exclude that the observed difference is partly due to distinct cell cycle distribution of both cell types. However, this data strongly suggests alterations in protein stability that, according to data shown in **Figures 4B-C**, might reflect differences between polyubiquitylation and ubiquitin removal by hydrolysation (deubiquitylation). To address this point, the inventors compared gene expression of Cdc25A, Cdh1, β-TrCP and that of the recently described Dub3 deubiquitylase, between ES and NIH-3t3 cells. Whereas mRNA levels of Cdc25A, Cdh1 and β-TrCP in ES cells hardly differ from NIH-3t3 cells, Dub3 mRNA level was 4-fold higher in ES cells (**Figure 7A**). Moreover, RNAi-mediated knockdown of Dub3 in ES cells (**Figure 7B**) did not affect Cdc25A mRNA level (**Figure 7C**) but resulted in 3-fold reduction of Cdc25A protein abundance (**Figure 7D**). These data are consistent with previous work in human cells and indicate that Dub3 function in regulating Cdc25A protein stability is analogous in mouse ES cells. In addition, the inventors also observed a role of Dub3 in Cdc25A stability in unperturbed and damaged NIH-3t3 cells (**Figure 6D-E**). Of note, GFP-tagged Dub3 shows an exclusive nuclear localization (**Figure 7E-F**) as previously observed for Cdc25A in ES cells. Finally, to address the role of Cdh1 and β-TrCP in regulating Cdc25A levels in ES cells, the inventors performed RNAi-mediated knockdown experiments. In contrast to Dub3 knockdown neither Cdh1, nor β-TrCP downregulation affected Cdc25A mRNA expression nor did significantly alter Cdc25A stability (**Figures 6F-J**). These observations are consistent with a previous study showing that APC/Cdh1 activity is attenuated in ES cells by high levels of the Emi1 inhibitor.

### Orphan receptor Esrrb regulates Dub3 gene expression

Based on previously described consensus sequence for binding motifs of key transcription factors involved in reprogramming, the inventors analyzed the proximal promoter (6 kb) of the Dub3 gene. Strikingly, while no Oct4, Nanog, Klf4, Smad1, Stat3, c-Myc nor n-Myc consensus sites could be detected, the inventors originally (NCBI37/mm9) found up to seven estrogen-related-receptor-b (Esrrb) putative binding motifs (consensus: 5'-TNAAGGTCA-3') and two Sox2 putative response elements (consensus: 5'-CATTGTT-3'). However the latest update of this genomic sequence (GRCm38/mm10) displays only three Esrrb sites (**Figure 8A****,** Esrrb-RE). Esrrb is a nuclear receptor belonging to the superfamily of nuclear hormone receptors. Together with Sox2, it is part of the core self-renewal machinery. Esrrb knockdown using a previously validated RNAi sequence resulted in significant decrease of endogenous Dub3 transcript level (**Figure 8B**), to a similar extent than the previously described Esrrb target gene Nanog. Inversely, ectopic expression of Esrrb in ES cells, and not of its C-terminal truncated form (A-Cter) lacking the activation function 2 (AF2) domain, led to significant increase in endogenous Dub3 mRNA level (**Figure 8C**). Moreover, treatment of ES cells with increasing dose of DY131, a previously described selective Esrrb and Esrrg agonist, boosted Dub3 gene expression and increased Cdc25A protein abundance without affecting Cdc25A transcript level (**Figures 9A-B**). Inversely, Esrrb knockdown resulted in a 40% decrease of DY131-mediated Dub3 transcription **(****Figure 9C**), while Sox2 knockdown using a previously published RNAi sequence did not strongly affected Dub3 expression, though slightly increased it (inventors unpublished observations).

Next, the inventors performed chromatin immunoprecipitation (ChIP) experiments to map Esrrb and Sox2 binding to Dub3 promoter in ES cells. To this end, the inventors designed five primer pairs (**Figure 8A****,** pp) separated by approximately 1kb to scan promoter occupancy by Esrrb and Sox2 within the 6kb upstream of the start codon (ATG+1). Sonication of chromatin resulted in fragments under 500 bp, limiting signal overlap between primers (**Figure 9D**). ChIP analysis with an anti-Esrrb antibody (**Figure 9E**) shows that Esrrb binds to the proximal Dub3 promoter in regions containing the three Esrrb consensus binding motifs (**Figure 8D**, pp 3-5), while no Esrrb binding was observed in an upstream region that does not contain Esrrb binding sites (pp 1-2). On the contrary, ChIP analysis with an anti-Sox2 antibody showed high enrichment only at one of the two consensus sites in the Dub3 promoter (Sox2-RE2), around primer pair 3, while in the region containing the second site (Sox2-RE1, pp4-5) Sox2 was bound to much lower levels.

To corroborate abovementioned ChIP data, the inventors cloned the Dub3 proximal promoter (3,2 kb) and analyzed its transcriptional activity in a reporter assay using luciferase activity as readout. For this purpose the inventors used cells that have very low expression of endogenous steroid receptors (CV1 cells). As anticipated, the inventors observed strong induction of luciferase activity upon Esrrb expression in cells cotransfected with the 3.2 kb Dub3 promoter that contains all three Esrrb binding sites (**Figure 8E**, Esrrb, white bars) while only background activity was observed on a region of the Dub3 promoter (5' far) devoid of Esrrb consensus binding sites (Esrrb, black bars). Similarly, expression of Esrrb A-Cter, resulted in basal promoter activity, comparable to that observed by expression of empty vector (EV, **Figure 8E** **and** **Figure 9F**). Interestingly, the inventors did not observe stimulation of luciferase activity upon expression of Sox2, but a small and significant repression of basal promoter activity (**Figure 8E**). Importantly, mutation of the unique Esrrb binding site in a 1kb Dub3 genomic fragment decreased transcriptional activity (**Figure 8F**). Altogether these observations suggest that Dub3 is a direct Esrrb target gene, having a positive role in regulating transcription of the Dub3 gene, while Sox2 on its own is not sufficient to stimulate Dub3 transcription.

### Developmental regulation of Dub3 expression and Cdc25A stability

Esrrb is a pluripotency factor highly expressed in ES cells that, unlike Sox2, is strongly downregulated upon ES differentiation. Since Dub3 is an Esrrb target, the inventors analyzed expression of Dub3 during neural conversion of ES cells in vitro. Plating of ES cells in N2B27 culture medium triggers conversion into neuroepithelial precursors microscopically visible as rosette conformations (**Figures 10A-E**, day 7, in particular **Figure 10E**). Loss of pluripotency was monitored by expression analysis of specific markers such as Oct4, Nanog, Klf4, and acquisition of neural identity was monitored by Nestin and Sox1 expression. Specificity was controlled by analysis of Sox7 expression, a well-established endoderm marker (**Figures 11A-D**). Importantly, Nestin was detectable in just about each individual cell of the differentiating population at day 6, indicating homogenous neural conversion. Acute (within 24 hours) decrease of Esrrb mRNA expression preceded in time a marked and dramatic decrease of Dub3 expression (**Figures 10F-H**). Expression of Sox2 also decreased after 24 hours, however of only 50% and increased afterwards. In contrast, neither Cdc25A nor Cdh1 or β-TrCP transcript levels significantly changed during differentiation (**Figure 10G**). Expression analysis of three other deubiquitylases implicated in Cdc25A stability, USP13, 29 and 48 revealed a decrease of only USP48 within 24 hours after differentiation (**Figure 10H**) that mirrored Sox2 expression. Importantly, the inventors could not find any consensus Esrrb binding sites within the USP48 proximal promoter. In contrast, USP13 gene expression did not significantly change during differentiation, while USP29 expression strongly increased during neural conversion.

To analyze Dub3 protein levels the inventors raised a specific antibody recognizing, as expected, a 60 kDa polypeptide in SDS-PAGE (**Figures 11E-F**). Dub3 protein levels dropped massively very early during differentiation, much earlier than Oct4, finely correlating with Dub3 mRNA levels **(****Figure 10I****).** Strikingly, lineage commitment between days 2-3, as monitored by Sox1 expression, led to a marked and continuous decrease of Cdc25A protein level, while the protein level of the two other Cdc25 family members, Cdc25B and Cdc25C, remained constant during differentiation (**Figure 11G**). The inventors further analyzed expression of two additional Dub3 substrates during differentiation, RhoAand Suds3, and observed no significant variations in gene expression (**Figure 11H**), nor in protein stability (**Figure 10I**), although a small decrease in Suds3 level was seen at day 7 after differentiation. Finally, the inventors found very low expression of Esrrg (another member of the subfamily) in ES cells that further increased during differentiation (**Figure 11H**), corroborating the specificity of Dub3 gene regulation by Esrrb. Altogether, these findings suggest that reduced Cdc25A protein abundance during neural differentiation is likely governed at the posttranslational level.

While retaining self-renewal properties, neural stem cells (NSC) are multipotent stem cells derived from ES cells, isolated and amplified at day 7 following differentiation. Quantification of Cdc25A abundance revealed 8-fold more Cdc25A in asynchronously growing ES cells compared to NSCs (**Figure 10J**). Similar to NIH-3t3 cells, the inventors detected very low Dub3 transcript levels in NSCs (**Figure 11I**)**.** Finally, the inventors isolated and analyzed three different genomic fragments of the Dub3 promoter and compared basal transcriptional activity in NIH-3t3 versus ES cells. The inventors observed strong transcriptional activity of all three promoter sequences in ES cells, about 10-fold higher than in NIH-3t3 cells (**Figure 10K**), further corroborating mRNA expression during differentiation (**Figures 10F-H**).

### Dub3 expression is important for maintenance of pluripotency and cell cycle remodelling during differentiation

Stable transfection of Esrrb in ES cells has been shown to be sufficient to sustain pluripotency in absence of LIF. The inventors therefore addressed whether forced Dub3 expression in ES cells could substitute Essrb function in maintaining pluripotency in absence of LIF. To this end, the inventors generated a stable ES cell line, expanded from a single ES colony, expressing eGFP-Dub3 under control of a constitutive strong promoter (**Figures 12A-D**). Remarkably, while authors reported that high Dub3 expression induces S-G2/M arrest in human somatic U2OS cells, ES cells overexpressing Dub3 could be propagated without significant differences in cell cycle distribution compared to a control cell line, indicating that in ES cells constitutive Dub3 expression is not toxic (**Figures 13A-B** **and** **W-X**). Removal of LIF led to an apparent highly similar morphological differentiation program in both cell-lines, but unexpectedly resulted in massive death of eGFP-Dub3-expressing ES cells two days after, microscopically visible as detached cells with retracted nuclei (**Figures 12E-J****,** arrows). Of note, five days following LIF withdrawal, hardly any cell survived in the eGFP-Dub3 expressing cell-line. Caspase-3 activity, essential for proper differentiation, was higher at days 3-4 in eGFP-Dub3 expressing cells compared to empty vector, strongly indicative of apoptosis (**Figure 12K** **and** **Figure 13C-V**). Finally, whereas mRNA and protein levels of pluripotency and differentiation markers were highly comparable in both cell lines, the inventors observed elevated expression of the apoptotic marker Noxa at day two and afterwards in eGFP-Dub3 expressing cells (**Figure 13Y****-AD**). Remarkably, 2-3 days upon LIF removal, a strong reduction of eGFP-Dub3 protein level was evident (**Figure 12K**), suggesting an additional control at post-transcriptional level, very likely proteolysis, occurring during differentiation. A similar phenotype was observed upon N2B27-mediated neural conversion, and a similar result was also observed with a ES cell line expressing HA N-terminal-tagged Dub3 (**Figures 13AE-****AF**), ruling out a non-specific effect of the GFP tag or of the differentiation protocol used. Onset of apoptosis, was equally observed by FACS analysis (**Figure 12L**), that showed the presence of subdiploid (less than 2N) cell debris starting from day three during differentiation and being predominant at day four. Interestingly, appearance of the sub-G1 cell population in ES cells expressing eGFP-Dub3 was concomitant to cell lineage commitment, as monitored by Sox1 and Nestin expression (**Figures 11A-B**) and cell cycle remodelling which started at day three in the control cell line (empty vector), resulting in lengthening of the G1 phase (**Figure 12L**). Altogether these results strongly suggest that high Dub3 expression is lethal during differentiation at the time when cell cycle remodelling occurs.

Finally the inventors analyzed the effect of Dub3 or Cdc25A knockdown in ES cells. Interestingly, prolonged (7 days) RNAi mediated Dub3 knockdown, resulted in an increase of alkaline phosphatase (AP)-negative colonies, as well as heterogeneous morphological differentiation of ES cells even in the presence of LIF, suggesting that Dub3 expression is important for maintenance of pluripotency (**Figures 12M-O**). A very similar result was also observed upon prolonged Cdc25A knockdown. In sum, these data couple the self-renewal machinery of ES cells through Essrb to the master cell cycle regulator Cdc25A and remodelling of the cell cycle during differentiation through modulation of Dub3 expression.

### Discussion

In this study the inventors dissected the G1/S checkpoint signalling pathway in ES cells. The inventors found that ES cells maintain high levels of the Cdc25A phosphatase in G1 that persists even after DNA damage. Knockdown of Cdc25A expression resulted in a G1 delay and increased CDK2Y15P after UV damage within 24 hours post RNAi treatment (a condition required to avoid natural G1 phase expansion due to differentiation of ES cells). Indeed, prolonged Cdc25A downregulation (or Dub3), resulted in cell differentiation in the presence of LIF, in line with the notion that lengthening of the G1 phase and deregulation of CDK2 activity is linked to differentiation. These findings provide an explanation for absent regulation of CDK2 activity upon DNA damage in ES cells. This model is also in line with existing evidence linking elevated Cdc25A expression with impaired G1/S arrest followed by radioresistant DNA synthesis in cancer cells.

Interestingly, in addition to Cdc25A, the inventors have also observed down-regulation of Cyclin E (**Figure 11J**), another CDK2 regulator that is rate limiting during the G1/S transition and opposes spontaneous differentiation of naïve ES cells. Moreover, ablation of the SCFFbw7-mediated degradation pathway controlling Cyclin E abundance in vivo results in impaired differentiation, genomic instability and hyperproliferation, illustrating the importance of Cyclin E regulation in mouse development. Taken together, both reduced abundance of Cdc25A and Cyclin E during differentiation of ES cells, likely embody key molecular adaptations that control CDK activity and consequent G1 lengthening. Importantly, as a result of expanded G1, the p53-dependent response may now become more effective in CDK2 inhibition since this requires a slow transcriptional-dependent induction of the CDK inhibitor p21 protein level. It is anticipated that p21 may have virtually no role in CDK2 regulation in ES cells since these cells spend most of their time in S phase and p21 is efficiently degraded by the PCNA-dependent CRL4Cdt2 ubiquitin ligase throughout S phase, as well as after DNA damage.

The inventors have provided evidence that post-transcriptional regulation of Cdc25A abundance in ES cells depends upon the Dub3 deubiquitylase. Expression of Dub3, and not Cdh1 or β-TrCP, is higher in ES cells compared to differentiated cells, and knockdown of Cdh1 or β-TrCP did not significantly change the stability of Cdc25A since it is already highly stabilized in ES cells. These observations are consistent with the finding that ES cells have attenuated APC activity that increases during differentiation. Of the four additional deubiquitylases implicated in Cdc25A stability in human cells (USP13, 29, 48 and Dub2A), the inventors found that only USP48 mRNA levels significantly decreased during differentiation although its expression remained high and increased towards the end of differentiation, mirroring Sox2 expression. Hence, although the inventors cannot exclude a redundant role for Dub2A and USP48 in Cdc25A stability during differentiation, the inventors data support a key role for Dub3 in this process, as previously shown in somatic cells, and suggest that in ES cells the balance of ubiquitylation and deubiquitylation activities, which fine-tunes the steady-state level of Cdc25A, is shifted towards deubiquitylation due to high Dub3 expression. The inventors showed that downregulation of Esrrb negatively affected the endogenous expression of the Dub3 gene, to a similar extent than a previously characterized Esrrb target gene, Nanog. However, expression of Oct4, another Esrrb target was not found to be much affected by Esrrb knockdown. These differences likely exist because in ES cells, expression of pluripotency genes is under the combinatorial control of transcription factors of the pluripotency gene regulatory network. This transcriptional control appears to be very complex, gene-specific and remains to be further clarified.

The inventors observed that while forced Dub3 expression could not inhibit differentiation upon LIF withdrawal, unexpectedly it induced massive apoptosis during differentiation concomitant to lineage commitment and cell cycle remodelling, such as lengthening of the G1 phase. These observations are in line with the recent finding that expression of non-degradable Cdc25A mutants leads to early embryonic lethality in mice (E3.5) showing the importance of fine-tuning the expression level of Cdc25A already at the oocyte and morula stages. Although the inventors have shown that Cdc25A is a critical Dub3 substrate in ES cells, the inventors cannot exclude the implication of other Dub3 substrates in the toxicity observed by forced Dub3 expression during differentiation. The importance of tight Cdc25A regulation during embryogenesis is also underscored by its function in regulation of pluripotency versus differentiation of ES cells since Cdc25A is expressed in progenitor cells undergoing proliferative self-renewing divisions. The inventors speculate that this developmental regulation might be governed by Dub3 to modify cell cycle dynamics under control of Esrrb.

In conclusion the inventors' results couple the Cdc25A-CDK2 cell cycle signalling pathway to the self-renewal machinery through Esrrb-dependent regulation of Dub3 in ES cells, and highlight the importance of deubiquitylases in stem cell and developmental biology. Since cell cycle regulation is a rate-limiting step in reprogramming processes, these findings put Dub3 and Cdc25A as interesting candidate genes in cell reprogramming.

## Claims

1. Use of
- Dub3 protein, said protein comprising the amino acid sequence as set forth in SEQ ID NO : 1, or any variant thereof comprising one of the amino acid sequences as set forth in SEQ ID NO: 2 to SEQ ID NO: 19, or
- a nucleic acid molecule coding for said protein or said variant thereof,
for inducing *in vitro* dedifferentiation of differentiated cells, wherein the cells obtained from the dedifferentiation of differentiated cells are iPS cells.

2. Use according to claim 1, for inducing *in vitro* dedifferentiation of differentiated cells, wherein said Dub3 protein or said nucleic acid molecule coding for said protein are associated with at least an Oct family member protein and a Sox family member protein.

3. Use of an inhibitor of the activity and/or of the expression of the Dub3 protein or a variant thereof, said protein comprising the amino acid sequence as set forth in SEQ ID NO : 1, or any variant thereof comprising one of the amino acid sequences as set forth in SEQ ID NO: 2 to SEQ ID NO: 19, and having ubiquitin hydrolase activity, for inducing *in vitro* the spontaneous differentiation of totipotent or pluripotent cells.

## Patentansprüche

1. Verwendung von
- Dub3 Protein, das die in SEQ ID NO: 1 gezeigte Aminosäuresequenz umfasst, oder einer Variante davon umfassend eine der in SEQ ID NO: 2 bis SEQ ID NO: 19 gezeigten Aminosäuresequenzen, oder
- einem Nukleinsäuremolekül, das für besagtes Protein oder besagte Variante davon kodiert,
zum Induzieren von *in vitro* Dedifferenzierung von differenzierten Zellen, wobei die aus der Dedifferenzierung von differenzierten Zellen erhaltenen Zellen iPS-Zellen sind.

2. Verwendung nach Anspruch 1 zum Induzieren von *in vitro* Dedifferenzierung von differenzierten Zellen, wobei das Dub3 Protein oder das Nukleinsäuremolekül, das für dieses Protein kodiert, mit mindestens einem Protein der Oct Familie und einem Protein der Sox Familie assoziiert ist.

3. Verwendung eines Inhibitors der Aktivität und/oder der Expression von Dub3 Protein, das die in SEQ ID NO: 1 gezeigte Aminosäuresequenz umfasst, oder einer Variante davon umfassend eine der in SEQ ID NO: 2 bis SEQ ID NO: 19 gezeigten Aminosäuresequenzen, wobei das Protein oder die Variante davon Ubiquitinhydrolase-Aktvität aufweist, zum Induzieren der spontanen Differenzierung von totipotenten oder pluripotenten Zellen *in vitro.*

## Revendications

1. Utilisation de
- La protéine Dub3, ladite protéine comprenant la séquence d'acides aminés telle qu'illustrée par la séquence SEQ ID NO : 1, ou tout variant de celle-ci comprenant l'une des séquences d'acides aminés telles qu'illustrées par les séquences SEQ ID NO : 2 à SEQ ID NO : 19, ou
- Une molécule d'acide nucléique codant ladite protéine ou un variant de celle-ci,
pour l'induction in vitro de la dédifférenciation de cellules différenciées, où lesdites cellules issues de la dédifférenciation des cellules différenciées sont des cellules IPS.

2. Utilisation selon la revendication 1, pour l'induction in vitro de la dédifférenciation de cellules différenciées, où ladite protéine Dub3 ou ladite molécule d'acide nucléique codant ladite protéine sont associés à au moins une protéine de la famille Oct et une protéine de la famille Sox.

3. Utilisation d'un inhibiteur de l'activité et/ou de l'expression de la protéine Dub3 ou un variant de celle-ci, ladite protéine comprenant une séquence d'acides aminés telle qu'illustrée par la séquence SEQ ID NO : 1, ou tout variant de celle-ci comprenant une des séquences telles qu'illustrées par SEQ ID NO : 2 à SEQ ID NO : 19, et ayant une activité ubiqutine hydrolase, pour l'induction in vitro la différenciation spontanée de cellules totipotentes ou pluripotentes.


### SEQUENCE LISTING

<110> CENTRE NATIONAL DE LA RECHERCHE SCIENTIFIQUE (CNRS)
   MAIORANO, Domenico
   VAN DER LAAN, Siem
<120> Cell differentiation marker and its uses
<130> BR 73151
<150> EP13306448.5
   <151> 2013-10-21
<160> 43
<170> PatentIn version 3.5
<210> 1
   <211> 530
   <212> PRT
   <213> Homo sapiens
<400> 1
   Met Glu Asp Asp Ser Leu Tyr Leu Gly Gly Glu Trp Gln Phe Asn His
   1               5                   10                  15
   Phe Ser Lys Leu Thr Ser Ser Arg Pro Asp Ala Ala Phe Ala Glu Ile
               20                  25                  30
   Gln Arg Thr Ser Leu Pro Glu Lys Ser Pro Leu Ser Cys Glu Thr Arg
           35                  40                  45
   Val Asp Leu Cys Asp Asp Leu Ala Pro Val Ala Arg Gln Leu Ala Pro
       50                  55                  60
   Arg Glu Lys Leu Pro Leu Ser Ser Arg Arg Pro Ala Ala Val Gly Ala
   65                  70                  75                  80
   Gly Leu Gln Asn Met Gly Asn Thr Cys Tyr Val Asn Ala Ser Leu Gln
                   85                  90                  95
   Cys Leu Thr Tyr Thr Pro Pro Leu Ala Asn Tyr Met Leu Ser Arg Glu
               100                 105                 110
   His Ser Gln Thr Cys His Arg His Lys Gly Cys Met Leu Cys Thr Met
           115                 120                 125
   Gln Ala His Ile Thr Arg Ala Leu His Asn Pro Gly His Val Ile Gln
       130                 135                 140
   Pro Ser Gln Ala Leu Ala Ala Gly Phe His Arg Gly Lys Gln Glu Asp
   145                 150                 155                 160
   Ala His Glu Phe Leu Met Phe Thr Val Asp Ala Met Lys Lys Ala Cys
                   165                 170                 175
   Leu Pro Gly His Lys Gln Val Asp His His Ser Lys Asp Thr Thr Leu
               180                 185                 190
   Ile His Gln Ile Phe Gly Gly Tyr Trp Arg Ser Gln Ile Lys Cys Leu
           195                 200                 205
   His Cys His Gly Ile Ser Asp Thr Phe Asp Pro Tyr Leu Asp Ile Ala
       210                 215                 220
   Leu Asp Ile Gln Ala Ala Gln Ser Val Gln Gln Ala Leu Glu Gln Leu
   225                 230                 235                 240
   Val Lys Pro Glu Glu Leu Asn Gly Glu Asn Ala Tyr His Cys Gly Val
                   245                 250                 255
   Cys Leu Gln Arg Ala Pro Ala Ser Lys Thr Leu Thr Leu His Thr Ser
               260                 265                 270
   Ala Lys Val Leu Ile Leu Val Leu Lys Arg Phe Ser Asp Val Thr Gly
           275                 280                 285
   Asn Lys Ile Ala Lys Asn Val Gln Tyr Pro Glu Cys Leu Asp Met Gln
       290                 295                 300
   Pro Tyr Met Ser Gln Pro Asn Thr Gly Pro Leu Val Tyr Val Leu Tyr
   305                 310                 315                 320
   Ala Val Leu Val His Ala Gly Trp Ser Cys His Asn Gly His Tyr Phe
                   325                 330                 335
   Ser Tyr Val Lys Ala Gln Glu Gly Gln Trp Tyr Lys Met Asp Asp Ala
               340                 345                 350
   Glu Val Thr Ala Ser Ser Ile Ile Ser Val Leu Ser Gln Gln Ala Tyr
           355                 360                 365
   Val Leu Phe Tyr Ile Gln Lys Ser Glu Trp Glu Arg His Ser Glu Ser
       370                 375                 380
   Val Ser Arg Gly Arg Glu Pro Ser Ala Leu Gly Ala Glu Asp Thr Asp
   385                 390                 395                 400
   Arg Arg Ala Thr Gln Gly Glu Leu Lys Arg Asp His Pro Cys Leu Gln
                   405                 410                 415
   Ala Pro Glu Leu Asp Glu His Leu Val Glu Arg Ala Thr Gln Glu Ser
   420 425 430
   Thr Leu Asp His Trp Lys Phe Leu Gln Glu Gln Asn Lys Thr Lys Pro
   435 440 445
   Glu Phe Asn Val Arg Lys Val Glu Gly Thr Leu Pro Pro Asp Val Leu
   450 455 460
   Val Ile His Gln Ser Lys Tyr Lys Cys Gly Met Lys Asn His His Pro
   465 470 475 480
   Glu Gln Gln Ser Ser Leu Leu Asn Leu Ser Ser Thr Thr Pro Thr His
   485 490 495
   Gln Glu Ser Met Asn Thr Gly Thr Leu Ala Ser Leu Arg Gly Arg Ala
   500 505 510
   Arg Arg Ser Lys Gly Lys Asn Lys His Ser Lys Arg Ala Leu Leu Val
   515 520 525
   Cys Gln
   530
<210> 2
   <211> 540
   <212> PRT
   <213> Mus musculus
<400> 2
   Met Val Val Ser Leu Ser Phe Pro Glu Glu Thr Gly Gly Glu Asn Leu
   1 5 10 15
   Pro Ser Ala Pro Leu Glu Asp Ser Ser Lys Phe Phe Glu Glu Val Phe
   20 25 30
   Gly Asp Met Val Val Ala Leu Ser Phe Pro Glu Ala Asp Pro Ala Leu
   35 40 45
   Ser Ser Pro Asp Ala Pro Glu Leu His Gln Asp Glu Ala Gln Val Val
   50 55 60
   Glu Glu Leu Thr Thr Asn Gly Lys His Ser Leu Ser Trp Glu Ser Pro
   65 70 75 80
   Gln Gly Pro Gly Cys Gly Leu Gln Asn Thr Gly Asn Ser Cys Tyr Leu
   85 90 95
   Asn Ala Ala Leu Gln Cys Leu Thr His Thr Pro Pro Leu Ala Asp Tyr
               100                 105                 110
   Met Leu Ser Gln Glu His Ser Gln Thr Cys Cys Ser Pro Glu Gly Cys
           115                 120                 125
   Lys Met Cys Ala Met Glu Ala His Val Thr Gln Ser Leu Leu His Ser
       130                 135                 140
   His Ser Gly Asp Val Met Lys Pro Ser Gln Ile Leu Thr Ser Ala Phe
   145                 150                 155                 160
   His Lys His Gln Gln Glu Asp Ala His Glu Phe Leu Met Phe Thr Leu
                   165                 170                 175
   Glu Thr Met His Glu Ser Cys Leu Gln Val His Arg Gln Ser Asp Pro
               180                 185                 190
   Thr Pro Gln Asp Thr Ser Pro Ile His Asp Ile Phe Gly Gly Trp Trp
           195                 200                 205
   Arg Ser Gln Ile Lys Cys Leu His Cys Gln Gly Thr Ser His Thr Phe
       210                 215                 220
   Asp Pro Phe Leu Asp Val Pro Leu Asp Ile Ser Ser Ala Gln Ser Val
   225                 230                 235                 240
   Asn Gln Ala Leu Trp Asp Thr Gly Lys Ser Glu Glu Leu Leu Gly Glu
                   245                 250                 255
   Asn Ala Tyr Tyr Cys Gly Arg Cys Arg Gln Lys Met Pro Ala Ser Lys
               260                 265                 270
   Thr Leu His Val His Ile Ala Pro Lys Val Leu Leu Leu Val Leu Lys
           275                 280                 285
   Arg Phe Ser Ala Phe Thr Gly Asn Lys Leu Asp Arg Lys Val Ser Tyr
       290                 295                 300
   Pro Glu Phe Leu Asp Leu Lys Pro Tyr Leu Ser Glu Pro Thr Gly Gly
   305                 310                 315                 320
   Pro Leu Pro Tyr Ala Leu Tyr Ala Val Leu Val His Asp Gly Ala Thr
                   325                 330                 335
   Ser Asn Ser Gly His Tyr Phe Cys Cys Val Lys Ala Gly His Gly Lys
                   340                 345                 350
   Trp Tyr Lys Met Asp Asp Thr Lys Val Thr Arg Cys Asp Val Thr Ser
           355                 360                 365
   Val Leu Asn Glu Asn Ala Tyr Val Leu Phe Tyr Val Gln Gln Thr Asp
       370                 375                 380
   Leu Lys Gln Val Ser Ile Asp Met Pro Glu Gly Arg Val His Glu Val
   385                 390                 395                 400
   Leu Asp Pro Lys Tyr Gln Leu Lys Lys Ser Arg Arg Lys Lys Arg Lys
                   405                 410                 415
   Lys Gln Cys His Cys Thr Asp Asp Ala Gly Glu Ala Cys Glu Asn Arg
               420                 425                 430
   Glu Lys Arg Ala Lys Lys Glu Thr Ser Leu Gly Glu Gly Lys Val Pro
           435                 440                 445
   Gln Glu Val Asn His Glu Lys Ala Gly Gln Lys His Gly Asn Thr Lys
       450                 455                 460
   Leu Val Pro Gln Glu Gln Asn His Gln Arg Ala Gly Gln Asn Leu Arg
   465                 470                 475                 480
   Asn Thr Glu Val Glu Leu Asp Leu Pro Val Asp Ala Ile Val Ile His
                   485                 490                 495
   Gln Pro Arg Ser Thr Ala Asn Trp Gly Thr Asp Ala Pro Asp Lys Glu
               500                 505                 510
   Asn Gln Pro Trp His Asn Gly Asp Arg Leu Leu Thr Ser Gln Gly Leu
           515                 520                 525
   Met Ser Pro Gly Gln Leu Cys Ser Gln Gly Gly Arg
       530                 535                 540
<210> 3
   <211> 530
   <212> PRT
   <213> Homo sapiens
<400> 3
<210> 4
   <211> 530
   <212> PRT
   <213> Homo sapiens
<400> 4
   Met Gly Asp Asp Ser Leu Tyr Leu Gly Gly Glu Trp Gln Phe Asn His
   1               5                   10                  15
   Phe Ser Lys Leu Thr Ser Ser Arg Pro Asp Ala Ala Phe Ala Glu Ile
               20                  25                  30
   Gln Arg Thr Ser Leu Pro Glu Lys Ser Pro Leu Ser Ser Glu Thr Arg
           35                  40                  45
   Val Asp Leu Cys Asp Asp Leu Ala Pro Val Ala Arg Gln Leu Ala Pro
       50                  55                  60
   Arg Glu Lys Leu Pro Leu Ser Ser Arg Arg Pro Ala Ala Val Gly Ala
   65                  70                  75                  80
   Gly Leu Gln Asn Met Gly Asn Thr Cys Tyr Glu Asn Ala Ser Leu Gln
                   85                  90                  95
   Cys Leu Thr Tyr Thr Leu Pro Leu Ala Asn Tyr Met Leu Ser Arg Glu
               100                 105                 110
   His Ser Gln Thr Cys Gln Arg Pro Lys Cys Cys Met Leu Cys Thr Met
           115                 120                 125
   Gln Ala His Ile Thr Trp Ala Leu His Ser Pro Gly His Val Ile Gln
       130                 135                 140
   Pro Ser Gln Ala Leu Ala Ser Gly Phe His Arg Gly Lys Gln Glu Asp
   145                 150                 155                 160
   Val His Glu Phe Leu Met Phe Thr Val Asp Ala Met Lys Lys Ala Cys
                   165                 170                 175
   Leu Pro Gly His Lys Gln Val Asp His His Ser Lys Asp Thr Thr Leu
               180                 185                 190
   Ile His Gln Ile Phe Gly Gly Cys Trp Arg Ser Gln Ile Lys Cys Leu
           195                 200                 205
   His Cys His Gly Ile Ser Asp Thr Phe Asp Pro Tyr Leu Asp Ile Ala
       210                 215                 220
   Leu Asp Ile Gln Ala Ala Gln Ser Val Lys Gln Ala Leu Glu Gln Leu
   225                 230                 235                 240
   Val Lys Pro Glu Glu Leu Asn Gly Glu Asn Ala Tyr His Cys Gly Leu
                   245                 250                 255
   Cys Leu Gln Arg Ala Pro Ala Ser Asn Thr Leu Thr Leu His Thr Ser
               260                 265                 270
   Ala Lys Val Leu Ile Leu Val Leu Lys Arg Phe Ser Asp Val Ala Gly
           275                 280                 285
   Asn Lys Leu Ala Lys Asn Val Gln Tyr Pro Glu Cys Leu Asp Met Gln
       290                 295                 300
   Pro Tyr Met Ser Gln Gln Asn Thr Gly Pro Leu Val Tyr Val Leu Tyr
   305                 310                 315                 320
   Ala Val Leu Val His Ala Gly Trp Ser Cys His Asp Gly His Tyr Phe
                   325                 330                 335
   Ser Tyr Val Lys Ala Gln Glu Gly Gln Trp Tyr Lys Met Asp Asp Ala
               340                 345                 350
   Glu Val Thr Val Cys Ser Ile Thr Ser Val Leu Ser Gln Gln Ala Tyr
           355                 360                 365
   Val Leu Phe Tyr Ile Gln Lys Ser Glu Trp Glu Arg His Ser Glu Ser
       370                 375                 380
   Val Ser Arg Gly Arg Glu Pro Arg Ala Leu Gly Ala Glu Asp Thr Asp
   385                 390                 395                 400
   Arg Arg Ala Lys Gln Gly Glu Leu Lys Arg Asp His Pro Cys Leu Gln
                   405                 410                 415
   Ala Pro Glu Leu Asp Glu His Leu Val Glu Arg Ala Thr Gln Glu Ser
               420                 425                 430
   Thr Leu Asp His Trp Lys Phe Leu Gln Glu Gln Asn Lys Thr Lys Pro
           435                 440                 445
   Glu Phe Asn Val Gly Lys Val Glu Gly Thr Leu Pro Pro Asn Ala Leu
       450                 455                 460
   Val Ile His Gln Ser Lys Tyr Lys Cys Gly Met Lys Asn His His Pro
   465 470 475 480
   Glu Gln Gln Ser Ser Leu Leu Asn Leu Ser Ser Thr Thr Arg Thr Asp
   485 490 495
   Gln Glu Ser Met Asn Thr Gly Thr Leu Ala Ser Leu Gln Gly Arg Thr
   500 505 510
   Arg Arg Ala Lys Gly Lys Asn Lys His Ser Lys Arg Ala Leu Leu Val
   515 520 525
   Cys Gln
   530
<210> 5
   <211> 530
   <212> PRT
   <213> Homo sapiens
<400> 5
   Met Glu Asp Asp Ser Leu Tyr Leu Gly Gly Asp Trp Gln Phe Asn His
   1 5 10 15
   Phe Ser Lys Leu Thr Ser Ser Arg Leu Asp Ala Ala Phe Ala Glu Ile
   20 25 30
   Gln Arg Thr Ser Leu Ser Glu Lys Ser Pro Leu Ser Ser Glu Thr Arg
   35 40 45
   Phe Asp Leu Cys Asp Asp Leu Ala Pro Val Ala Arg Gln Leu Ala Pro
   50 55 60
   Arg Glu Lys Leu Pro Leu Ser Ser Arg Arg Pro Ala Ala Val Gly Ala
   65 70 75 80
   Gly Leu Gln Lys Ile Gly Asn Thr Phe Tyr Val Asn Val Ser Leu Gln
   85 90 95
   Cys Leu Thr Tyr Thr Leu Pro Leu Ser Asn Tyr Met Leu Ser Arg Glu
   100 105 110
   Asp Ser Gln Thr Cys His Leu His Lys Cys Cys Met Phe Cys Thr Met
   115 120 125
   Gln Ala His Ile Thr Trp Ala Leu His Ser Pro Gly His Val Ile Gln
   130 135 140
   Pro Ser Gln Val Leu Ala Ala Gly Phe His Arg Gly Glu Gln Glu Asp
   145                 150                 155                 160
   Ala His Glu Phe Leu Met Phe Thr Val Asp Ala Met Lys Lys Ala Cys
                   165                 170                 175
   Leu Pro Gly His Lys Gln Leu Asp His His Ser Lys Asp Thr Thr Leu
               180                 185                 190
   Ile His Gln Ile Phe Gly Ala Tyr Trp Arg Ser Gln Ile Lys Tyr Leu
           195                 200                 205
   His Cys His Gly Val Ser Asp Thr Phe Asp Pro Tyr Leu Asp Ile Ala
       210                 215                 220
   Leu Asp Ile Gln Ala Ala Gln Ser Val Lys Gln Ala Leu Glu Gln Leu
   225                 230                 235                 240
   Val Lys Pro Lys Glu Leu Asn Gly Glu Asn Ala Tyr His Cys Gly Leu
                   245                 250                 255
   Cys Leu Gln Lys Ala Pro Ala Ser Lys Thr Leu Thr Leu Pro Thr Ser
               260                 265                 270
   Ala Lys Val Leu Ile Leu Val Leu Lys Arg Phe Ser Asp Val Thr Gly
           275                 280                 285
   Asn Lys Leu Ala Lys Asn Val Gln Tyr Pro Lys Cys Arg Asp Met Gln
       290                 295                 300
   Pro Tyr Met Ser Gln Gln Asn Thr Gly Pro Leu Val Tyr Val Leu Tyr
   305                 310                 315                 320
   Ala Val Leu Val His Ala Gly Trp Ser Cys His Asn Gly His Tyr Phe
                   325                 330                 335
   Ser Tyr Val Lys Ala Gln Glu Gly Gln Trp Tyr Lys Met Asp Asp Ala
               340                 345                 350
   Glu Val Thr Ala Ser Gly Ile Thr Ser Val Leu Ser Gln Gln Ala Tyr
           355                 360                 365
   Val Leu Phe Tyr Ile Gln Lys Ser Glu Trp Glu Arg His Ser Glu Ser
       370                 375                 380
   Val Ser Arg Gly Arg Glu Pro Arg Ala Leu Gly Ala Glu Asp Thr Asp
   385                 390                 395                 400
   Arg Pro Ala Thr Gln Gly Glu Leu Lys Arg Asp His Pro Cys Leu Gln
                   405                 410                 415
   Val Pro Glu Leu Asp Glu His Leu Val Glu Arg Ala Thr Gln Glu Ser
               420                 425                 430
   Thr Leu Asp His Trp Lys Phe Pro Gln Glu Gln Asn Lys Thr Lys Pro
           435                 440                 445
   Glu Phe Asn Val Arg Lys Val Glu Gly Thr Leu Pro Pro Asn Val Leu
       450                 455                 460
   Val Ile His Gln Ser Lys Tyr Lys Cys Gly Met Lys Asn His His Pro
   465                 470                 475                 480
   Glu Gln Gln Ser Ser Leu Leu Asn Leu Ser Ser Thr Lys Pro Thr Asp
                   485                 490                 495
   Gln Glu Ser Met Asn Thr Gly Thr Leu Ala Ser Leu Gln Gly Ser Thr
               500                 505                 510
   Arg Arg Ser Lys Gly Asn Asn Lys His Ser Lys Arg Ser Leu Leu Val
           515                 520                 525
   Cys Gln
       530
<210> 6
   <211> 530
   <212> PRT
   <213> Homo sapiens
<400> 6
   Met Gly Asp Asp Ser Leu Tyr Leu Gly Gly Glu Trp Gln Phe Asn His
   1               5                   10                  15
   Phe Ser Lys Leu Thr Ser Ser Arg Pro Asp Ala Ala Phe Ala Glu Ile
               20                  25                  30
   Gln Arg Thr Ser Leu Pro Glu Lys Ser Pro Leu Ser Ser Glu Thr Arg
           35                  40                  45
   Val Asp Leu Cys Asp Asp Leu Ala Pro Val Ala Arg Gln Leu Ala Pro
       50                  55                  60
   Arg Glu Lys Leu Pro Leu Ser Ser Arg Arg Pro Ala Ala Val Gly Ala
   65 70 75 80
   Gly Leu Gln Asn Met Gly Asn Thr Cys Tyr Glu Asn Ala Ser Leu Gln
   85 90 95
   Cys Leu Thr Tyr Thr Leu Pro Leu Ala Asn Tyr Met Leu Ser Arg Glu
   100 105 110
   His Ser Gln Thr Cys Gln Arg Pro Lys Cys Cys Met Leu Cys Thr Met
   115 120 125
   Gln Ala His Ile Thr Trp Ala Leu His Ser Pro Gly His Val Ile Gln
   130 135 140
   Pro Ser Gln Ala Leu Ala Ala Gly Phe His Arg Gly Lys Gln Glu Asp
   145 150 155 160
   Val His Glu Phe Leu Met Phe Thr Val Asp Ala Met Lys Lys Ala Cys
   165 170 175
   Leu Pro Gly His Lys Gln Val Asp His His Ser Lys Asp Thr Thr Leu
   180 185 190
   Ile His Gln Ile Phe Gly Gly Cys Trp Arg Ser Gln Ile Lys Cys Leu
   195 200 205
   His Cys His Gly Ile Ser Asp Thr Phe Asp Pro Tyr Leu Asp Ile Ala
   210 215 220
   Leu Asp Ile Gln Ala Ala Gln Ser Val Lys Gln Ala Leu Glu Gln Leu
   225 230 235 240
   Val Lys Pro Glu Glu Leu Asn Gly Glu Asn Ala Tyr His Cys Gly Leu
   245 250 255
   Cys Leu Gln Arg Ala Pro Ala Ser Asn Thr Leu Thr Leu His Thr Ser
   260 265 270
   Ala Lys Val Leu Ile Leu Val Leu Lys Arg Phe Ser Asp Val Ala Gly
   275 280 285
   Asn Lys Leu Ala Lys Asn Val Gln Tyr Pro Glu Cys Leu Asp Met Gln
   290 295 300
   Pro Tyr Met Ser Gln Gln Asn Thr Gly Pro Leu Val Tyr Val Leu Tyr
   305 310 315 320
   Ala Val Leu Val His Ala Gly Trp Ser Cys His Asp Gly Tyr Tyr Phe
   325 330 335
   Ser Tyr Val Lys Ala Gln Glu Gly Gln Trp Tyr Lys Met Asp Asp Ala
   340 345 350
   Glu Val Thr Val Cys Ser Ile Thr Ser Val Leu Ser Gln Gln Ala Tyr
   355 360 365
   Val Leu Phe Tyr Ile Gln Lys Ser Glu Trp Glu Arg His Ser Glu Ser
   370 375 380
   Val Ser Arg Gly Arg Glu Pro Arg Ala Leu Gly Ala Glu Asp Thr Asp
   385 390 395 400
   Arg Pro Ala Thr Gln Gly Glu Leu Lys Arg Asp His Pro Cys Leu Gln
   405 410 415
   Val Pro Glu Leu Asp Glu His Leu Val Glu Arg Ala Thr Glu Glu Ser
   420 425 430
   Thr Leu Asp His Trp Lys Phe Pro Gln Glu Gln Asn Lys Met Lys Pro
   435 440 445
   Glu Phe Asn Val Arg Lys Val Glu Gly Thr Leu Pro Pro Asn Val Leu
   450 455 460
   Val Ile His Gln Ser Lys Tyr Lys Cys Gly Met Lys Asn His His Pro
   465 470 475 480
   Glu Gln Gln Ser Ser Leu Leu Asn Leu Ser Ser Met Asn Ser Thr Asp
   485 490 495
   Gln Glu Ser Met Asn Thr Gly Thr Leu Ala Ser Leu Gln Gly Arg Thr
   500 505 510
   Arg Arg Ser Lys Gly Lys Asn Lys His Ser Lys Arg Ser Leu Leu Val
   515 520 525
   Cys Gln
   530
<210> 7
   <211> 526
   <212> PRT
   <213> Mus musculus
<400> 7
   Met Val Val Ala Leu Ser Phe Pro Glu Ala Asp Pro Ala Leu Ser Ser
   1               5                   10                  15
   Pro Asp Ala Pro Glu Leu His Gln Asp Glu Ala Gln Val Val Glu Glu
               20                  25                  30
   Leu Thr Val Asn Gly Lys His Ser Leu Ser Trp Glu Ser Pro Gln Gly
           35                  40                  45
   Pro Gly Cys Gly Leu Gln Asn Thr Gly Asn Ser Cys Tyr Leu Asn Ala
       50                  55                  60
   Ala Leu Gln Cys Leu Thr His Thr Pro Pro Leu Ala Asp Tyr Met Leu
   65                  70                  75                  80
   Ser Gln Glu His Ser Gln Thr Cys Cys Ser Pro Glu Gly Cys Lys Leu
                   85                  90                  95
   Cys Ala Met Glu Ala Leu Val Thr Gln Ser Leu Leu His Ser His Ser
               100                 105                 110
   Gly Asp Val Met Lys Pro Ser His Ile Leu Thr Ser Ala Phe His Lys
           115                 120                 125
   His Gln Gln Glu Asp Ala His Glu Phe Leu Met Phe Thr Leu Glu Thr
       130                 135                 140
   Met His Glu Ser Cys Leu Gln Val His Arg Gln Ser Lys Pro Thr Ser
   145                 150                 155                 160
   Glu Asp Ser Ser Pro Ile His Asp Ile Phe Gly Gly Trp Trp Arg Ser
                   165                 170                 175
   Gln Ile Lys Cys Leu Leu Cys Gln Gly Thr Ser Asp Thr Tyr Asp Arg
               180                 185                 190
   Phe Leu Asp Ile Pro Leu Asp Ile Ser Ser Ala Gln Ser Val Lys Gln
           195                 200                 205
   Ala Leu Trp Asp Thr Glu Lys Ser Glu Glu Leu Cys Gly Asp Asn Ala
       210                 215                 220
   Tyr Tyr Cys Gly Lys Cys Arg Gln Lys Met Pro Ala Ser Lys Thr Leu
   225                 230                 235                 240
   His Val His Ile Ala Pro Lys Val Leu Met Val Val Leu Asn Arg Phe
                   245                 250                 255
   Ser Ala Phe Thr Gly Asn Lys Leu Asp Arg Lys Val Ser Tyr Pro Glu
               260                 265                 270
   Phe Leu Asp Leu Lys Pro Tyr Leu Ser Glu Pro Thr Gly Gly Pro Leu
           275                 280                 285
   Pro Tyr Ala Leu Tyr Ala Val Leu Val His Asp Gly Ala Thr Ser His
       290                 295                 300
   Ser Gly His Tyr Phe Cys Cys Val Lys Ala Gly His Gly Lys Trp Tyr
   305                 310                 315                 320
   Lys Met Asp Asp Thr Lys Val Thr Arg Cys Asp Val Thr Ser Val Leu
                   325                 330                 335
   Asn Glu Asn Ala Tyr Val Leu Phe Tyr Val Gln Gln Ala Asn Leu Lys
               340                 345                 350
   Gln Val Ser Ile Asp Met Pro Glu Gly Arg Ile Asn Glu Val Leu Asp
           355                 360                 365
   Pro Glu Tyr Gln Leu Lys Lys Ser Arg Arg Lys Lys His Lys Lys Lys
       370                 375                 380
   Ser Pro Phe Thr Glu Asp Leu Gly Glu Pro Cys Glu Asn Arg Asp Lys
   385                 390                 395                 400
   Arg Ala Ile Lys Glu Thr Ser Leu Gly Lys Gly Lys Val Leu Gln Glu
                   405                 410                 415
   Val Asn His Lys Lys Ala Gly Gln Lys His Gly Asn Thr Lys Leu Met
               420                 425                 430
   Pro Gln Lys Gln Asn His Gln Lys Ala Gly Gln Asn Leu Arg Asn Thr
           435                 440                 445
   Glu Val Glu Leu Asp Leu Pro Ala Asp Ala Ile Val Ile His Gln Pro
       450                 455                 460
   Arg Ser Thr Ala Asn Trp Gly Arg Asp Ser Pro Asp Lys Glu Asn Gln
   465                 470                 475                 480
   Pro Leu His Asn Ala Asp Arg Leu Leu Thr Ser Gln Gly Pro Val Asn
                   485                 490                 495
   Thr Trp Gln Leu Cys Arg Gln Glu Gly Arg Arg Arg Ser Lys Lys Gly
               500                 505                 510
   Gln Asn Lys Asn Lys Gln Gly Gln Arg Leu Leu Leu Val Cys
   515 520 525
<210> 8
   <211> 467
   <212> PRT
   <213> Mus musculus
<400> 8
   Met Val Val Ala Leu Ser Phe Pro Glu Asp Pro Ala Met Ser Pro Pro
   1 5 10 15
   Ser Ala Pro Glu Leu His Gln Asp Glu Ala Gln Val Val Glu Glu Leu
   20 25 30
   Ala Ala Asn Gly Lys His Ser Leu Ser Trp Glu Ser Pro Gln Gly Pro
   35 40 45
   Gly Cys Gly Leu Gln Asn Thr Gly Asn Ser Cys Tyr Leu Asn Ala Ala
   50 55 60
   Leu Gln Cys Leu Thr His Thr Pro Pro Leu Ala Asp Tyr Met Leu Ser
   65 70 75 80
   Gln Glu His Ser Gln Thr Cys Cys Ser Pro Glu Gly Cys Lys Met Cys
   85 90 95
   Ala Met Glu Ala His Val Thr Gln Ser Leu Leu His Thr His Ser Gly
   100 105 110
   Asp Val Met Lys Pro Ser Gln Ile Leu Thr Ser Ala Phe His Lys Arg
   115 120 125
   Lys Gln Glu Asp Ala His Glu Phe Leu Met Phe Thr Leu Glu Thr Met
   130 135 140
   His Glu Ser Cys Leu Gln Val His Arg Gln Ser Glu Pro Thr Ser Glu
   145 150 155 160
   Asp Ser Ser Pro Ile His Asp Ile Phe Gly Gly Trp Trp Arg Ser Gln
   165 170 175
   Ile Lys Cys His His Cys Gln Gly Thr Ser Tyr Ser Tyr Asp Pro Phe
   180 185 190
   Leu Asp Ile Pro Leu Asp Ile Ser Ser Val Gln Ser Val Lys Gln Ala
   195 200 205
   Leu Gln Asp Thr Glu Lys Ala Glu Glu Leu Cys Gly Glu Asn Ser Tyr
       210                 215                 220
   Tyr Cys Gly Arg Cys Arg Gln Lys Lys Pro Ala Ser Lys Thr Leu Lys
   225                 230                 235                 240
   Leu Tyr Ser Ala Pro Lys Val Leu Met Leu Val Leu Lys Arg Phe Ser
                   245                 250                 255
   Gly Ser Met Gly Lys Lys Leu Asp Arg Lys Val Ser Tyr Pro Glu Phe
               260                 265                 270
   Leu Asp Leu Lys Pro Tyr Leu Ser Gln Pro Thr Gly Gly Pro Leu Pro
           275                 280                 285
   Tyr Ala Leu Tyr Ala Val Leu Val His Glu Gly Ala Thr Cys His Ser
       290                 295                 300
   Gly His Tyr Phe Cys Cys Val Lys Ala Gly His Gly Lys Trp Tyr Lys
   305                 310                 315                 320
   Met Asp Asp Thr Lys Val Thr Ser Cys Asp Val Thr Ser Val Leu Asn
                   325                 330                 335
   Glu Asn Ala Tyr Val Leu Phe Tyr Val Gln Gln Asn Asp Leu Lys Lys
               340                 345                 350
   Gly Ser Ile Asn Met Pro Glu Gly Arg Ile His Glu Val Leu Asp Ala
           355                 360                 365
   Glu Tyr Gln Leu Lys Lys Ser Gly Glu Lys Lys His Asn Lys Ser Pro
       370                 375                 380
   Cys Thr Glu Asp Ala Gly Glu Pro Cys Glu Asn Arg Glu Lys Arg Ser
   385                 390                 395                 400
   Ser Lys Glu Thr Ser Leu Gly Glu Gly Lys Val Leu Gln Glu Gln Asp
                   405                 410                 415
   His Gln Lys Ala Gly Gln Lys Gln Glu Asn Thr Lys Leu Thr Pro Gln
               420                 425                 430
   Glu Gln Asn His Gln Lys Gly Gly Gln Asn Leu Arg Asn Thr Glu Gly
           435                 440                 445
   Glu Leu Asp Arg Leu Ser Gly Ala Ile Val Val Tyr Gln Pro Ile Cys
   450 455 460
   Thr Ala Asn
   465
<210> 9
   <211> 545
   <212> PRT
   <213> Mus musculus
<400> 9
   Met Val Val Ser Leu Ser Phe Pro Glu Ala Asp Pro Ala Leu Ser Ser
   1               5                   10                  15
   Pro Gly Ala Gln Gln Leu His Gln Asp Glu Ala Gln Val Val Val Glu
               20                  25                  30
   Leu Thr Ala Asn Asp Lys Pro Ser Leu Ser Trp Glu Cys Pro Gln Gly
           35                  40                  45
   Pro Gly Cys Gly Leu Gln Asn Thr Gly Asn Ser Cys Tyr Leu Asn Ala
       50                  55                  60
   Ala Leu Gln Cys Leu Thr His Thr Pro Pro Leu Ala Asp Tyr Met Leu
   65                  70                  75                  80
   Ser Gln Glu Tyr Ser Gln Thr Cys Cys Ser Pro Glu Gly Cys Lys Met
                   85                  90                  95
   Cys Ala Met Glu Ala His Val Thr Gln Ser Leu Leu His Ser His Ser
               100                 105                 110
   Gly Asp Val Met Lys Pro Ser Gln Ile Leu Thr Ser Ala Phe His Lys
           115                 120                 125
   His Gln Gln Glu Asp Ala His Glu Phe Leu Met Phe Thr Leu Glu Thr
       130                 135                 140
   Met His Glu Ser Cys Leu Gln Val His Arg Gln Ser Glu Pro Thr Ser
   145                 150                 155                 160
   Glu Asp Ser Ser Pro Ile His Asp Ile Phe Gly Gly Leu Trp Arg Ser
                   165                 170                 175
   Gln Ile Lys Cys Leu His Cys Gln Gly Thr Ser Asp Thr Tyr Asp Arg
               180                 185                 190
   Phe Leu Asp Val Pro Leu Asp Ile Ser Ser Ala Gln Ser Val Asn Gln
           195                 200                 205
   Ala Leu Trp Asp Thr Glu Lys Ser Glu Glu Leu Arg Gly Glu Asn Ala
       210                 215                 220
   Tyr Tyr Cys Gly Arg Cys Arg Gln Lys Met Pro Ala Ser Lys Thr Leu
   225                 230                 235                 240
   His Ile His Ser Ala Pro Lys Val Leu Leu Leu Val Leu Lys Arg Phe
                   245                 250                 255
   Ser Ala Phe Met Gly Asn Lys Leu Asp Arg Lys Val Ser Tyr Pro Glu
               260                 265                 270
   Phe Leu Asp Leu Lys Pro Tyr Leu Ser Gln Pro Thr Gly Gly Pro Leu
           275                 280                 285
   Pro Tyr Ala Leu Tyr Ala Val Leu Val His Glu Gly Ala Thr Cys His
       290                 295                 300
   Ser Gly His Tyr Phe Ser Tyr Val Lys Ala Arg His Gly Ala Trp Tyr
   305                 310                 315                 320
   Lys Met Asp Asp Thr Lys Val Thr Ser Cys Asp Val Thr Ser Val Leu
                   325                 330                 335
   Asn Glu Asn Ala Tyr Val Leu Phe Tyr Val Gln Gln Thr Asp Leu Lys
               340                 345                 350
   Gln Val Ser Ile Asp Met Pro Glu Gly Arg Val His Glu Val Leu Asp
           355                 360                 365
   Pro Glu Tyr Gln Leu Lys Lys Ser Arg Arg Lys Lys His Lys Lys Lys
       370                 375                 380
   Ser Pro Cys Thr Glu Asp Ala Gly Glu Pro Cys Lys Asn Arg Glu Lys
   385                 390                 395                 400
   Arg Ala Thr Lys Glu Thr Ser Leu Gly Glu Gly Lys Val Leu Gln Glu
                   405                 410                 415
   Lys Asn His Lys Lys Ala Gly Gln Lys His Glu Asn Thr Lys Leu Val
               420                 425                 430
   Pro Gln Glu Gln Asn His Gln Lys Leu Gly Gln Lys His Arg Ile Asn
           435                 440                 445
   Glu Ile Leu Pro Gln Glu Gln Asn His Gln Lys Ala Gly Gln Ser Leu
       450                 455                 460
   Arg Asn Thr Glu Gly Glu Leu Asp Leu Pro Ala Asp Ala Ile Val Ile
   465                 470                 475                 480
   His Leu Leu Arg Ser Thr Glu Asn Trp Gly Arg Asp Ala Pro Asp Lys
                   485                 490                 495
   Glu Asn Gln Pro Trp His Asn Ala Asp Arg Leu Leu Thr Ser Gln Asp
               500                 505                 510
   Pro Val Asn Thr Gly Gln Leu Cys Arg Gln Glu Gly Arg Arg Arg Ser
           515                 520                 525
   Lys Lys Gly Lys Asn Lys Asn Lys Gln Gly Gln Arg Leu Leu Leu Val
       530                 535                 540
   Cys
   545
<210> 10
   <211> 545
   <212> PRT
   <213> Mus musculus
<400> 10
   Met Val Val Ser Leu Ser Phe Pro Glu Ala Asp Pro Ala Leu Ser Ser
   1               5                   10                  15
   Pro Gly Ala Gln Gln Leu His Gln Asp Glu Ala Gln Val Val Val Glu
               20                  25                  30
   Leu Thr Ala Asn Asp Lys Pro Ser Leu Ser Trp Glu Cys Pro Gln Gly
           35                  40                  45
   Pro Gly Cys Gly Leu Gln Asn Thr Gly Asn Ser Cys Tyr Leu Asn Ala
       50                  55                  60
   Ala Leu Gln Cys Leu Thr His Thr Pro Pro Leu Ala Asp Tyr Met Leu
   65                  70                  75                  80
   Ser Gln Glu Tyr Ser Gln Thr Cys Cys Ser Pro Glu Gly Cys Lys Met
                   85                  90                  95
   Cys Ala Met Glu Ala His Val Thr Gln Ser Leu Leu His Ser His Ser
               100                 105                 110
   Gly Asp Val Met Lys Pro Ser Gln Ile Leu Thr Ser Ala Phe His Lys
           115                 120                 125
   His Gln Gln Glu Asp Ala His Glu Phe Leu Met Phe Thr Leu Glu Thr
       130                 135                 140
   Met His Glu Ser Cys Leu Gln Val His Arg Gln Ser Glu Pro Thr Ser
   145                 150                 155                 160
   Glu Asp Ser Ser Pro Ile His Asp Ile Phe Gly Gly Leu Trp Arg Ser
                   165                 170                 175
   Gln Ile Lys Cys Leu His Cys Gln Gly Thr Ser Asp Thr Tyr Asp Arg
               180                 185                 190
   Phe Leu Asp Val Pro Leu Asp Ile Ser Ser Ala Gln Ser Val Asn Gln
           195                 200                 205
   Ala Leu Trp Asp Thr Glu Lys Ser Glu Glu Leu Arg Gly Glu Asn Ala
       210                 215                 220
   Tyr Tyr Cys Gly Arg Cys Arg Gln Lys Met Pro Ala Ser Lys Thr Leu
   225                 230                 235                 240
   His Ile His Ser Ala Pro Lys Val Leu Leu Leu Val Leu Lys Arg Phe
                   245                 250                 255
   Ser Ala Ser Met Gly Asn Lys Leu Asp Arg Lys Val Ser Tyr Pro Glu
               260                 265                 270
   Phe Leu Asp Leu Lys Pro Tyr Leu Ser Gln Pro Thr Gly Gly Pro Leu
           275                 280                 285
   Pro Tyr Ala Leu Tyr Ala Val Leu Val His Glu Gly Ala Thr Cys His
       290                 295                 300
   Ser Gly His Tyr Phe Ser Tyr Val Lys Ala Gly His Gly Lys Trp Tyr
   305                 310                 315                 320
   Lys Met Asp Asp Thr Lys Val Thr Ser Cys Asp Val Thr Ser Val Leu
                   325                 330                 335
   Asn Glu Asn Ala Tyr Val Leu Phe Tyr Val Gln Gln Thr Asp Leu Lys
               340                 345                 350
   Glu Cys Ser Ile Asp Met Pro Glu Gly Arg Ile His Glu Val Leu Asp
           355                 360                 365
   Pro Glu Tyr Gln Leu Lys Lys Ser Arg Arg Lys Lys His Lys Lys Lys
   370 375 380
   Ser Pro Cys Thr Glu Asp Val Gly Glu Pro Ser Lys Asn Arg Glu Lys
   385 390 395 400
   Lys Ala Thr Lys Glu Thr Ser Leu Gly Glu Gly Lys Val Leu Gln Glu
   405 410 415
   Lys Asn His Lys Lys Ala Gly Gln Lys His Glu Asn Thr Lys Leu Val
   420 425 430
   Pro Gln Glu Gln Asn His Gln Lys Leu Gly Gln Lys His Arg Asn Asn
   435 440 445
   Glu Ile Leu Pro Gln Glu Gln Asn His Gln Lys Thr Gly Gln Ser Leu
   450 455 460
   Arg Asn Thr Glu Gly Glu Leu Asp Ser Pro Ala Asp Ala Ile Val Ile
   465 470 475 480
   His Leu Pro Arg Ser Ile Ala Asn Trp Gly Arg Asp Thr Pro Asp Lys
   485 490 495
   Val Asn Gln Pro Trp His Asn Ala Asp Arg Leu Leu Thr Ser Gln Asp
   500 505 510
   Leu Val Asn Thr Gly Leu Leu Cys Arg Gln Glu Gly Arg Arg Arg Ser
   515 520 525
   Lys Lys Gly Lys Asn Lys Asn Asn Gln Gly Gln Lys Leu Leu Leu Val
   530 535 540
   Arg
   545
<210> 11
   <211> 549
   <212> PRT
   <213> Rattus Norvegicus
<220>
   <221> misc_feature
   <222> (40)..(40)
   <223> Xaa can be any naturally occurring amino acid
<400> 11
   Met Gln Ser Asp Phe Thr Ala Ser Glu Gly Asp Arg Ala Gly Ile Lys
   1               5                   10                  15
   Lys Val Phe Ala Ile Phe Trp Arg Glu Ser Leu Pro Ser Ala His Leu
               20                  25                  30
   Glu Asn Ser Ser Arg Leu Phe Xaa Asp Asp His Arg Asn Met Val Thr
           35                  40                  45
   Ala His Ser Phe Thr Glu Glu Asp Pro Ala Met Ser Pro Pro Ala Thr
       50                  55                  60
   Pro Glu Leu His Gln Asp Glu Ala Arg Val Leu Glu Glu Leu Ser Ala
   65                  70                  75                  80
   Lys Gly Lys Pro Ser Leu Ser Leu Gln Arg Ile Gln Ser Pro Gly Ser
                   85                  90                  95
   Gly Leu Gln Asn Ile Gly Asn Ser Cys Tyr Leu Asn Ala Val Leu Gln
               100                 105                 110
   Cys Leu Thr His Thr Pro Pro Leu Ala Asp Tyr Met Leu Ser Gln Glu
           115                 120                 125
   His Ser Gln Arg Cys Cys Tyr Pro Glu Gly Cys Lys Met Cys Ala Met
       130                 135                 140
   Glu Ala His Val Thr Gln Ser Leu Leu His Ser His Ser Gly Gly Val
   145                 150                 155                 160
   Met Lys Pro Ser Glu Ile Leu Thr Ser Thr Phe His Lys His Arg Gln
                   165                 170                 175
   Glu Asp Ala His Glu Phe Leu Met Phe Thr Leu Asn Ala Met His Glu
               180                 185                 190
   Ser Cys Leu Arg Gly Cys Lys Gln Ser Glu Thr Ser Ser Lys Asp Ser
           195                 200                 205
   Ser Leu Ile Tyr Asp Ile Phe Gly Gly Gln Met Arg Ser Gln Ile Lys
       210                 215                 220
   Cys His His Cys Gln Gly Thr Leu Asp Ser Tyr Asp Pro Phe Leu Asn
   225                 230                 235                 240
   Leu Phe Leu Asp Ile Cys Ser Ala Gln Ser Val Lys Gln Ala Leu Glu
                   245                 250                 255
   Asp Leu Val Lys Leu Glu Glu Leu Gln Gly Asp Asn Ala Tyr Tyr Cys
               260                 265                 270
   Gly Arg Cys Arg Glu Lys Met Pro Ala Ser Lys Thr Thr Lys Val Gln
           275                 280                 285
   Thr Ala Ser Lys Val Leu Leu Leu Val Leu Asn Arg Ser Tyr Asp Phe
       290                 295                 300
   Gly Gly Asp Lys Leu Asn Arg Val Val Ser Tyr Pro Glu Tyr Leu Asp
   305                 310                 315                 320
   Leu Gln Pro Tyr Leu Ser Gln Pro Thr Ala Gly Pro Leu Pro Tyr Ala
                   325                 330                 335
   Leu Tyr Ala Val Leu Val His Asp Gly Val Thr Cys Ser Ser Gly His
               340                 345                 350
   Tyr Phe Cys Tyr Val Lys Ala Ser His Gly Lys Trp Tyr Lys Met Asp
           355                 360                 365
   Asp Ser Lys Val Thr Arg Cys Asp Val Ser Ser Val Leu Ser Glu Pro
       370                 375                 380
   Ala Tyr Leu Leu Phe Tyr Val Gln Gln Thr Asp Leu Glu Lys Val Asn
   385                 390                 395                 400
   Val Asp Val Ser Val Gly Arg Val His Gly Val Leu His Pro Glu Ser
                   405                 410                 415
   Gln Gln Lys Lys Thr Arg Lys Lys Lys His Lys Arg Ser Ser Cys Thr
               420                 425                 430
   Glu Ala Val His Met Pro Arg Glu Asn Arg Glu Asn Thr Ala Thr Lys
           435                 440                 445
   Glu Thr Ser Leu Gly Glu Gly Lys Val Leu Gln Glu Gln Asn His Gln
       450                 455                 460
   Lys Ala Gly Gln Asn Leu Lys Thr Thr Lys Val Asn Leu Ser Ala Asn
   465                 470                 475                 480
   Gly Thr Val Ile His Gln Pro Arg Tyr Thr Ala Asn Trp Gly Arg Asn
                   485                 490                 495
   Ala Pro Asp Lys Asp Asp Gln Pro Gly His Ser Gly Asp Arg Leu Leu
               500                 505                 510
   Thr Thr Gln Gly Ser Met Asn Thr Gly Gln Leu Cys Gly His Gly Gly
   515 520 525
   Ser Gln Arg Ser Lys Lys Arg Lys Asn Lys Asn Lys Gln Gly Gln Arg
   530 535 540
   Pro Leu Leu Val Cys
   545
<210> 12
   <211> 468
   <212> PRT
   <213> Microtus ochrogaster
<400> 12
   Met Ala Ala Pro Ala Ala Pro Asp Leu Arg Pro Asp Glu Gly Leu Val
   1 5 10 15
   Val Ala Glu Leu Ala Ala Arg Ala Lys Pro Arg Met Ser Trp Glu Arg
   20 25 30
   Ile His Ser Val Gly Ala Gly Leu Gln Asn Thr Gly Asn Ser Cys Tyr
   35 40 45
   Leu Asn Ala Ala Leu Gln Cys Leu Thr His Thr Pro Pro Leu Ala Asn
   50 55 60
   Tyr Met Leu Ser Arg Glu His Ser Gln Ser Cys Gly His Gln Gly Gly
   65 70 75 80
   Cys Pro Met Cys Ala Met Glu Ala His Val Thr Gln Ser Phe Arg His
   85 90 95
   Ser Gly Glu Val Met Gln Pro Ser Lys Lys Leu Thr Gly Ala Phe His
   100 105 110
   Lys His Lys Gln Glu Asp Ala His Glu Phe Leu Met Phe Thr Leu Asn
   115 120 125
   Ala Met His Glu Ser Cys Leu Arg Gly Ser Lys Tyr Ser Gly Ala Pro
   130 135 140
   Ser Glu Asn Ser Thr Pro Ile His Ala Ile Phe Gly Gly Ser Trp Arg
   145 150 155 160
   Ser Gln Ile Lys Cys Leu His Cys Gln Gly Thr Ser Asp Ser Phe Asn
   165 170 175
   Pro Phe Leu Asp Ile Ser Leu Asp Ile His Ala Ala Gln Ser Val Lys
               180                 185                 190
   Gln Ala Leu Glu Asp Leu Val Gln Ala Glu Val Leu Cys Gly Glu Asn
           195                 200                 205
   Ala Tyr His Cys Asp His Cys Gln Gly Lys Thr Thr Ala Ser Lys Thr
       210                 215                 220
   Leu Met Val Gln Thr Ala Pro Lys Val Leu Met Leu Val Leu Asn Arg
   225                 230                 235                 240
   Phe Ser Gly Phe Thr Gly Asp Lys Val Asp Arg Lys Val Ser Tyr Pro
                   245                 250                 255
   Glu Ser Leu Asp Met Arg Pro Tyr Met Thr Gln Pro Asn Arg Gly Pro
               260                 265                 270
   Ser Val Tyr Val Leu Tyr Ala Val Leu Val His Ala Gly Leu Thr Cys
           275                 280                 285
   His Ser Gly His Tyr Phe Cys Tyr Val Arg Ala Gly Asn Gly Lys Trp
       290                 295                 300
   Tyr Lys Met Asp Asp Ser Lys Val Ala Arg Cys Asp Val Thr Ser Val
   305                 310                 315                 320
   Leu Ser Glu Pro Ala Tyr Val Leu Leu Tyr Val Arg Glu Thr Glu Leu
                   325                 330                 335
   Gln Lys Asp Ser Val Thr Gly Pro Val Asp Thr Val Gly Gln Asp Arg
               340                 345                 350
   Gln Arg Lys Leu Asn Arg Gly Ser Cys Val Gly Ala Ala Glu Pro Arg
           355                 360                 365
   Arg Pro Val Glu Ser Ala Ala Ala Lys Glu Ile Ser Leu Asp Gln Trp
       370                 375                 380
   Lys Ala Leu Leu Glu His Thr Arg Pro Asn Pro Ala Leu Asn Leu Arg
   385                 390                 395                 400
   Lys Thr Glu Ser Thr Leu Pro Val Asp Ala Val Val Ile His Gln Pro
                   405                 410                 415
   Arg His Arg Gly His Trp Asp Thr Asn Gly Pro Asp Lys Glu Asn Tyr
               420                 425                 430
   Pro Cys His Thr Ser Thr Arg Leu Leu Pro Ala Gln Arg Ala Met Gly
   435 440 445
   Thr Gln Gly Gly Arg Ser Arg Thr Lys Lys Asn Lys Gln Arg Trp Arg
   450 455 460
   Ser Leu Val Val
   465
<210> 13
   <211> 444
   <212> PRT
   <213> Mesocricetus auratus
<400> 13
   Met Asp Val Ser Val Asp Pro Ala Leu Ser Ser Pro Asp Gln Pro Asp
   1 5 10 15
   Leu Pro Gln Glu Glu Ala Gln Val Val Pro Glu Leu Ala Val Arg Glu
   20 25 30
   Glu His Arg Leu Ser Trp Lys Arg Pro His Gly Val Gly Ala Gly Leu
   35 40 45
   Glu Asn Thr Gly Asn Ser Cys Tyr Leu Asn Ala Ala Leu Gln Cys Leu
   50 55 60
   Thr His Thr Pro Pro Leu Ala Ser Tyr Met Leu Ser Arg Glu His Ser
   65 70 75 80
   Gln Asn Cys Cys His Arg Gly Ala Cys Met Met Cys Ala Met Glu Ala
   85 90 95
   His Val Thr Gln Ser Phe Leu Tyr Ser Gly Asp Val Ile Gln Pro Ser
   100 105 110
   Glu Met Leu Thr Ala Ala Phe His Lys His Arg Glu Glu Asp Ala His
   115 120 125
   Glu Phe Leu Met Phe Thr Leu Asn Ala Met His Thr Ser Cys Leu Pro
   130 135 140
   Gly Ser Lys Leu Met Gly Cys Thr Ser Lys Gln Ser Ser Ile Ile His
   145 150 155 160
   Glu Ile Phe Gly Gly Ser Trp Glu Ser Lys Ile Lys Cys Leu Cys Cys
   165 170 175
   Gln Ala Thr Thr Asp Thr Leu Glu Pro Phe Leu Asp Ile Thr Leu Asp
               180                 185                 190
   Ile Gln Thr Ala Gln Ser Val Asn Gln Ala Leu Glu Asn Leu Val Lys
           195                 200                 205
   Glu Glu Lys Leu Cys Gly Glu Asn Ala Tyr His Cys Asp Ile Cys Trp
       210                 215                 220
   Lys Asn Thr Pro Ala Ser Lys Thr Leu Ile Val Lys Asp Ala Pro Gln
   225                 230                 235                 240
   Val Leu Leu Leu Val Leu Asn Arg Phe Glu Glu Phe Thr Gly Asp Lys
                   245                 250                 255
   Lys Asp Arg Glu Val Ser Tyr Ser Glu Phe Leu Asp Phe Gln Pro Tyr
               260                 265                 270
   Val Ser Gln Ser Pro Arg Asp Pro Leu Leu Tyr Val Leu Tyr Ala Val
           275                 280                 285
   Leu Val His Asp Gly Met Thr Cys His Ser Gly His Tyr Phe Cys Tyr
       290                 295                 300
   Val Arg Ala Gly Asn Gly His Trp Tyr Lys Met Asn Asp Ser Ser Val
   305                 310                 315                 320
   Thr Arg Cys Asp Met Lys Ser Val Leu Ser Glu Pro Ala Tyr Val Leu
                   325                 330                 335
   Phe Tyr Val Gln Gln Thr Glu Leu Lys Lys Asn Leu Trp Met Leu Pro
               340                 345                 350
   Gln Ala Glu His Gln Ala Gly Glu Ser Arg His Thr Thr Ile Asn Arg
           355                 360                 365
   Gly Ser Pro Thr Glu Ala Glu Glu Ala Pro Asp His Ile Glu Asn Thr
       370                 375                 380
   Thr Val Gln Asp Phe Leu Gly His Trp Lys Ala Pro Lys Pro Leu Thr
   385                 390                 395                 400
   Asp Trp Arg Lys Asn His Leu Asp Arg Glu Asn Ser Pro Ile Arg Leu
                   405                 410                 415
   Leu Pro Gly Phe Cys Leu Ser His Gln Glu Thr Met Asp Thr Gly Gln
   420 425 430
   Leu Cys Ser Lys Gly Glu Arg Pro Arg Ser Lys Lys
   435 440
<210> 14
   <211> 482
   <212> PRT
   <213> Cricetulus griseus
<400> 14
   Met Lys Lys Arg Arg Arg His Leu Gln Glu Gly Lys Asp Pro Ser Asp
   1               5                   10                  15
   His Ser Gln His Ser Arg Thr Ile Ser Gly Ser Asn Pro Glu Asp Met
               20                  25                  30
   Glu Ala Ala Arg Glu Leu Ser Val Gly Glu Ser His Ser Lys Ser Leu
           35                  40                  45
   Ser Val Tyr Met Ala Ser Thr Lys Ala Val Gly Thr Glu Val Tyr Leu
       50                  55                  60
   Ser Ser Cys Pro Ala Thr Asp Pro Thr Leu Ser Ser Pro Asp Glu Pro
   65                  70                  75                  80
   Asn Arg Pro Gln Asn Glu Ala Gln Val Val Pro Glu Leu Ala Ala Lys
                   85                  90                  95
   Glu Glu Phe His Leu Ser Trp Gln Arg Pro His Asp Val Gly Ala Gly
               100                 105                 110
   Leu Glu Asn Thr Gly Asn Ser Cys Tyr Met Asn Ala Val Leu Gln Cys
           115                 120                 125
   Leu Thr His Thr Pro Pro Leu Val Asn Tyr Met Leu Ser Arg Glu His
       130                 135                 140
   Ser Gln Asn Cys Cys His Gln Gly Asp Cys Met Ile Cys Ala Met Glu
   145                 150                 155                 160
   Ala His Val Thr Arg Ser Leu Leu Tyr Ser Gly Asp Val Ile Gln Pro
                   165                 170                 175
   Ser Glu Lys Leu Thr Ala Ala Phe His Lys His Arg Gln Glu Asp Ala
               180                 185                 190
   His Glu Phe Leu Leu Phe Thr Leu Asn Ala Met His Thr Ser Cys Leu
               195                 200                 205
   Pro Gly Ser Lys Leu Leu Gly Cys Thr Ser Glu Gln Ser Ser Leu Ile
           210                 215                 220
   His Glu Ile Phe Gly Gly Ser Trp Lys Ser Gln Ile Lys Cys Leu His
   225                 230                 235                 240
   Cys Asn Glu Thr Thr Asp Leu Leu Glu Pro Phe Leu Asp Ile Thr Leu
                   245                 250                 255
   Asp Ile Gln Thr Ala Gln Ser Val Asn Gln Ala Leu Glu Asn Leu Val
               260                 265                 270
   Met Glu Glu Gln Leu Cys Gly Glu Asn Ala Tyr His Cys Asp Asn Cys
               275                 280                 285
   Arg Gln Lys Thr Met Ala Ser Lys Thr Leu Thr Val Lys Asp Ala Pro
           290                 295                 300
   Lys Val Leu Leu Leu Val Leu Asn Arg Phe Ser Glu Phe Thr Gly Asp
   305                 310                 315                 320
   Lys Lys Asp Arg Lys Val Ser Tyr Pro Glu Ser Phe Asp Phe Gln Pro
                   325                 330                 335
   Tyr Ile Ser Gln Ser His Arg Gln Pro Leu Phe Tyr Ser Leu Tyr Ala
               340                 345                 350
   Val Leu Val His Asp Gly Val Thr Cys His Ser Gly His Tyr Phe Cys
               355                 360                 365
   Tyr Val Lys Ala Gly Asn Gly His Trp Tyr Lys Met Asp Asp Ser Ser
           370                 375                 380
   Val Thr Arg Cys Asp Val Asn Ser Val Leu Ser Glu Pro Ala Tyr Val
   385                 390                 395                 400
   Leu Phe Tyr Val Gln Gln Thr Asp Leu Arg Thr Asn Leu Trp Val Leu
                   405                 410                 415
   Ser Gln Ala Glu His Gln Val Gly Glu Ser Trp Tyr Thr Thr Ile Asn
               420                 425                 430
   Arg Gly Ser Pro Thr Glu Ala Ala Glu Pro Pro Asp His Thr Glu Asn
               435                 440                 445
   Thr Ala Ala Lys Asn Phe Leu Asp His Trp Lys Thr Leu Leu Asn Met
   450 455 460
   Asn Thr Lys Ala Phe Gly Glu Thr Trp Lys Thr Gln Thr Tyr Ser Glu
   465 470 475 480
   Ser Lys
<210> 15
   <211> 529
   <212> PRT
   <213> Nomascus leucogenys
<400> 15
   Met Glu His Asp Ser Leu Tyr Ser Gly Gly Glu Trp His Phe Ser Arg
   1 5 10 15
   Phe Ser Lys Leu Thr Ser Ser Arg Pro His Ala Ala Phe Ala Glu Ile
   20 25 30
   Gln Arg Thr Ser Leu Pro Glu Lys Ser Pro Leu Ser Ser Glu Thr Arg
   35 40 45
   Val Asp Pro Cys Asp Asp Leu Ala Pro Val Ala Arg Gln Leu Ala Pro
   50 55 60
   Arg Glu Lys Leu Pro Leu Ser Ser Arg Gly Pro Ala Ala Val Gly Ala
   65 70 75 80
   Gly Leu Gln Asn Met Gly Asn Thr Cys Tyr Val Asn Ala Ser Leu Gln
   85 90 95
   Cys Leu Thr Tyr Thr Pro Pro Leu Ala Asn Tyr Met Leu Ser Arg Glu
   100 105 110
   His Ser Gln Thr Cys His Arg His Lys Cys Cys Met Leu Cys Thr Met
   115 120 125
   Gln Ala His Ile Thr Arg Ala Leu His Arg Pro Gly Asp Val Ile Gln
   130 135 140
   Pro Ser Gln Ala Leu Ala Ala Gly Phe His Arg Gly Lys Gln Glu Asp
   145 150 155 160
   Ala His Glu Phe Leu Met Phe Thr Val Asp Ala Met Arg Lys Ala Cys
   165 170 175
   Leu Pro Gly His Lys Gln Val Asp Pro His Ser Lys Asp Thr Thr Leu
               180                 185                 190
   Ile His Gln Ile Phe Gly Gly Tyr Trp Arg Ser Gln Ile Lys Cys Leu
           195                 200                 205
   His Cys Gln Gly Ile Ser Asp Thr Phe Asp Pro Tyr Leu Asp Ile Ala
       210                 215                 220
   Leu Asp Ile Gln Ala Ala Gln Ser Val Lys Gln Ala Leu Glu Gln Leu
   225                 230                 235                 240
   Val Lys Pro Glu Glu Leu Asn Gly Glu Asn Ala Tyr His Cys Gly Leu
                   245                 250                 255
   Cys Leu Gln Lys Ala Pro Ala Ser Lys Thr Leu Thr Leu His Thr Ser
               260                 265                 270
   Ala Lys Val Leu Ile Leu Val Leu Lys Arg Phe Ser Asp Val Thr Gly
           275                 280                 285
   Asn Lys Leu Ala Lys Asn Val Gln Tyr Pro Glu Cys Leu Asp Met Gln
       290                 295                 300
   Pro Tyr Met Ser Gln Gln Asn Thr Gly Pro Leu Val Tyr Val Leu Tyr
   305                 310                 315                 320
   Ala Val Leu Val His Ala Gly Trp Ser Cys His Asn Gly His Tyr Phe
                   325                 330                 335
   Ser Tyr Val Lys Ala Gln Glu Gly Gln Trp Tyr Lys Met Asp Asp Ala
               340                 345                 350
   Glu Val Thr Ala Ser Gly Ile Thr Ser Val Leu Ser Gln Gln Ala Tyr
           355                 360                 365
   Val Leu Phe Tyr Ile Gln Lys Ser Glu Leu Glu Arg His Ser Glu Gly
       370                 375                 380
   Val Ser Arg Gly Arg Glu Pro Arg Ala Leu Gly Pro Ala Asp Thr Asp
   385                 390                 395                 400
   Arg Arg Ala Thr Gln Gly Glu Leu Lys Arg Glu Pro Cys Leu Gln Val
                   405                 410                 415
   Pro Glu Leu Asp Glu His Ser Val Glu Arg Ala Thr Gln Glu Ser Thr
               420                 425                 430
   Leu Asp His Trp Lys Phe Leu Gln Glu Gln Asn Lys Thr Lys Pro Glu
   435 440 445
   Phe Asn Val Arg Lys Val Glu Gly Ser Leu Pro Pro Asn Val Val Val
   450 455 460
   Ile His Gln Ser Lys Tyr Lys Cys Gly Thr Lys Asn His His Pro Glu
   465 470 475 480
   Gln Gln Ser Ser Leu Leu Asn Leu Ser Ser Thr Asn Pro Thr Asp Gln
   485 490 495
   Glu Ser Ile Asn Thr Gly Thr Pro Ala Ser Arg Gln Gly Arg Thr Arg
   500 505 510
   Arg Ser Lys Gly Lys Asn Lys His Ser Asn Arg Ala Leu Leu Leu Cys
   515 520 525
   Gln
<210> 16
   <211> 530
   <212> PRT
   <213> Pan troglodytes
<400> 16
   Met Glu Asp Asp Ser Leu Tyr Leu Gly Gly Glu Trp Gln Phe Asn His
   1 5 10 15
   Phe Ser Lys Leu Thr Ser Ser Arg Pro Asp Ala Ala Phe Ala Glu Ile
   20 25 30
   Gln Arg Thr Ser Leu Pro Glu Lys Ser Pro Leu Ser Ser Glu Thr Arg
   35 40 45
   Val Asp Leu Cys Asp Asp Leu Ala Pro Val Ala Arg Gln Leu Ala Pro
   50 55 60
   Gly Glu Lys Leu Leu Leu Ser Ser Arg Arg Pro Ala Ala Val Gly Ala
   65 70 75 80
   Gly Leu Gln Asn Met Gly Asn Thr Cys Tyr Val Asn Ala Ser Leu Gln
   85 90 95
   Cys Leu Thr Tyr Thr Pro Pro Leu Ala Asn Tyr Met Leu Ser Arg Glu
   100 105 110
   His Ser Gln Thr Cys His Arg His Lys Gly Cys Met Leu Cys Thr Met
           115                 120                 125
   Gln Ala His Ile Thr Arg Ala Leu His Ile Pro Gly His Val Ile Gln
       130                 135                 140
   Pro Ser Gln Ala Leu Ala Ala Gly Phe His Arg Gly Lys Gln Glu Asp
   145                 150                 155                 160
   Ala His Glu Phe Leu Met Phe Thr Val Asp Ala Met Glu Lys Ala Cys
                   165                 170                 175
   Leu Pro Gly His Lys Gln Val Glu His His Ser Lys Asp Thr Thr Leu
               180                 185                 190
   Ile His Gln Ile Phe Gly Gly Tyr Trp Arg Ser Gln Ile Lys Cys Leu
           195                 200                 205
   His Cys His Gly Ile Ser Asp Thr Phe Asp Pro Tyr Leu Asp Ile Ala
       210                 215                 220
   Leu Asp Ile Gln Ala Ala Gln Ser Val Gln Gln Ala Leu Glu Gln Leu
   225                 230                 235                 240
   Val Lys Pro Glu Glu Leu Asn Gly Glu Asn Ala Tyr His Cys Gly Leu
                   245                 250                 255
   Cys Leu Gln Arg Ala Pro Ala Ser Lys Thr Leu Thr Leu His Thr Ser
               260                 265                 270
   Ala Lys Val Leu Ile Leu Val Leu Lys Arg Phe Ser Asp Val Thr Gly
           275                 280                 285
   Asn Lys Leu Ala Lys Asn Val Gln Tyr Pro Glu Cys Leu Asp Met Gln
       290                 295                 300
   Pro Tyr Met Ser Gln Gln Asn Thr Gly Pro Leu Val Tyr Val Leu Tyr
   305                 310                 315                 320
   Ala Val Leu Val His Ala Gly Trp Ser Cys His Asn Gly His Tyr Phe
                   325                 330                 335
   Ser Tyr Val Lys Ala Gln Glu Gly Gln Trp Tyr Lys Met Asp Asp Ala
               340                 345                 350
   Glu Val Thr Ala Ser Ser Ile Thr Ser Val Leu Ser Gln Gln Ala Tyr
           355                 360                 365
   Val Leu Phe Tyr Ile Gln Lys Ser Glu Trp Glu Arg His Ser Glu Ser
   370 375 380
   Ala Ser Arg Gly Arg Glu Pro Arg Ala Leu Gly Ala Glu Asp Thr Asp
   385 390 395 400
   Arg Arg Ala Thr Gln Gly Glu Leu Lys Arg Asp His Pro Cys Leu Gln
   405 410 415
   Ala Pro Glu Leu Gly Glu His Leu Val Glu Arg Ala Thr Gln Glu Ser
   420 425 430
   Thr Leu Asp His Trp Lys Phe Leu Gln Glu Gln Asn Lys Thr Lys Pro
   435 440 445
   Glu Phe Asn Val Arg Lys Val Glu Gly Thr Leu Pro Pro Asn Val Leu
   450 455 460
   Val Ile His Gln Ser Lys Tyr Lys Cys Gly Met Lys Asn His His Pro
   465 470 475 480
   Glu Gln Gln Ser Ser Leu Leu Asn Leu Ser Ser Thr Thr Pro Thr Asp
   485 490 495
   Gln Glu Ser Met Asn Thr Gly Thr Leu Ala Ser Leu Gln Gly Arg Thr
   500 505 510
   Arg Arg Ser Lys Gly Lys Asn Lys His Ser Lys Arg Ala Leu Leu Val
   515 520 525
   Cys Gln
   530
<210> 17
   <211> 530
   <212> PRT
   <213> Macaca mulatta
<400> 17
   Met Glu Asp Asp Ser Leu Tyr Leu Gly Gly Glu Trp Gln Phe Asn His
   1 5 10 15
   Phe Ser Lys Leu Thr Ser Ser Arg Pro Asp Ala Ala Phe Ala Glu Ile
   20 25 30
   Gln Arg Thr Ser Leu Pro Glu Lys Ser Pro Leu Ser Ser Glu Thr Arg
   35 40 45
   Val Asp Leu Cys Asp Asp Leu Ala Pro Val Ala Arg Gln Leu Ala Pro
       50                  55                  60
   Arg Glu Lys Leu Pro Leu Ser Ser Arg Arg Pro Ala Ala Val Gly Ala
   65                  70                  75                  80
   Gly Leu Gln Asn Met Gly Asn Thr Cys Tyr Val Asn Ala Ser Leu Gln
                   85                  90                  95
   Cys Leu Thr Tyr Thr Pro Pro Leu Ala Asn Tyr Met Leu Ser Arg Glu
               100                 105                 110
   His Ser Pro Thr Cys His Arg His Lys Gly Cys Met Leu Cys Thr Met
           115                 120                 125
   Gln Ala His Ile Thr Arg Ala Leu His Ile Pro Gly Arg Val Ile Gln
       130                 135                 140
   Pro Ser Gln Ala Leu Ala Ala Asp Phe His Arg Gly Lys Gln Glu Asp
   145                 150                 155                 160
   Ala His Glu Phe Leu Met Phe Thr Val Asp Ala Met Lys Lys Ala Cys
                   165                 170                 175
   Leu Pro Gly His Lys Gln Val Asp His His Ser Lys Asp Thr Thr Leu
               180                 185                 190
   Ile His Gln Ile Phe Gly Gly Tyr Trp Arg Ser Gln Ile Lys Cys Leu
           195                 200                 205
   His Cys His Gly Ile Ser Asp Thr Phe Asp Pro Tyr Leu Asp Ile Ala
       210                 215                 220
   Leu Asp Ile Gln Ala Ala Gln Ser Val Lys Gln Ala Leu Glu Gln Leu
   225                 230                 235                 240
   Val Lys Pro Glu Glu Leu Asn Gly Glu Asn Ala Tyr His Cys Gly Leu
                   245                 250                 255
   Cys Leu Gln Arg Ala Pro Ala Ser Lys Thr Leu Thr Leu His Thr Ser
               260                 265                 270
   Ala Lys Val Leu Ile Leu Val Leu Lys Arg Phe Ser Asp Val Thr Gly
           275                 280                 285
   Ser Lys Leu Ala Lys Asn Val His Tyr Pro Glu Cys Leu Asp Met Gln
       290                 295                 300
   Pro Tyr Met Ser Gln Gln Asn Thr Gly Pro Leu Val Tyr Val Leu Tyr
   305                 310                 315                 320
   Ala Val Leu Val His Ala Gly Trp Ser Cys His Asn Gly His Tyr Phe
                   325                 330                 335
   Ser Tyr Val Lys Ala Gln Glu Gly Gln Trp Tyr Lys Met Asp Asp Ala
               340                 345                 350
   Glu Val Thr Ala Ser Ser Ile Thr Ser Val Leu Ser Gln Gln Ala Tyr
           355                 360                 365
   Val Leu Phe Tyr Ile Gln Lys Ser Glu Trp Glu Arg His Ser Glu Ser
       370                 375                 380
   Ala Ser Arg Gly Arg Glu Pro Arg Ala Leu Gly Ala Glu Asp Thr Asp
   385                 390                 395                 400
   Arg Arg Ala Thr Gln Gly Glu Leu Lys Arg Asp His Pro Cys Leu Gln
                   405                 410                 415
   Ala Pro Glu Leu Asp Glu His Leu Val Glu Arg Ala Thr Gln Glu Ser
               420                 425                 430
   Thr Leu Asp His Trp Lys Phe Leu Gln Glu Gln Asn Lys Thr Lys Pro
           435                 440                 445
   Glu Phe Asn Val Arg Lys Val Glu Gly Thr Leu Pro Pro Asn Val Leu
       450                 455                 460
   Val Ile His Gln Ser Lys Tyr Lys Cys Gly Met Lys Asn His His Pro
   465                 470                 475                 480
   Glu Gln Gln Ser Ser Pro Leu Asn Leu Ser Ser Thr Thr Pro Thr Asp
                   485                 490                 495
   Gln Glu Ser Val Asn Thr Gly Thr Leu Ala Ser Leu Gln Gly Arg Thr
               500                 505                 510
   Arg Arg Ser Lys Gly Lys Asn Lys His Ser Lys Arg Ala Leu Leu Val
           515                 520                 525
   Cys Gln
       530
<210> 18
   <211> 533
   <212> PRT
   <213> Bos Taurus
<400> 18
   Met Glu Thr Leu Val Gly Leu Val Cys Arg Ala Pro Gly Ala Ala Ser
   1               5                   10                  15
   Glu His Gly Gly Gly Val Cys Pro Ser Pro Phe Asp Val Phe Pro Gly
               20                  25                  30
   Gly Gln Gly Cys Gly Pro Ser Ala Ala Gly Ala Asp Ala Leu Arg Gly
           35                  40                  45
   Pro Ser Val Pro Glu Gly Pro Ser Pro Ala Leu Gly Arg Pro Gln Arg
       50                  55                  60
   Gly Asp Leu Ala Pro Gly Ser Ala Gly Leu Thr Pro Gly Gln Lys Gly
   65                  70                  75                  80
   Ala Leu Ser Trp Lys Gly Pro Trp Gly Val Gly Ala Gly Leu Gln Asn
                   85                  90                  95
   Leu Gly Asn Thr Cys Tyr Val Asn Ala Ala Leu Gln Cys Leu Ser His
               100                 105                 110
   Thr Pro Pro Leu Ala Ser Trp Met Val Ser Gln Gln His Ala Thr Leu
           115                 120                 125
   Cys Pro Ala Arg Ser Ala Cys Thr Leu Cys Ala Met Arg Ala His Val
       130                 135                 140
   Thr Arg Ala Leu Leu His Ala Gly Glu Val Ile Arg Pro Arg Lys Asp
   145                 150                 155                 160
   Leu Leu Ala Gly Phe His Arg His Gln Gln Glu Asp Ala His Glu Phe
                   165                 170                 175
   Leu Met Phe Thr Leu Asn Ala Met Gln Gln Gly Cys Leu Ser Ala Ser
               180                 185                 190
   Gln Pro Ser Gly His Ala Ser Glu Asp Thr Thr Val Ile Arg Gln Ile
           195                 200                 205
   Phe Gly Gly Thr Trp Arg Ser Gln Ile Gln Cys Leu Arg Cys Leu Gly
       210                 215                 220
   Val Ser Asp Thr Phe Asp Pro Tyr Leu Asp Ile Ser Leu Asp Ile Thr
   225 230 235 240
   Ala Ala Gln Ser Val Glu Gln Ala Leu Arg Glu Leu Val Lys Pro Glu
   245 250 255
   Lys Leu Asp Ala Asp Asn Ala Tyr Asp Cys Gly Val Cys Leu Arg Lys
   260 265 270
   Val Pro Ala Thr Lys Arg Leu Thr Leu His Ser Thr Ser Gln Val Leu
   275 280 285
   Val Leu Val Leu Lys Arg Phe Thr Pro Val Ser Gly Ala Lys Arg Ala
   290 295 300
   Gln Glu Val Arg Tyr Pro Gln Cys Leu Asp Leu Gln Pro Tyr Thr Ser
   305 310 315 320
   Glu Arg Lys Ala Gly Pro Leu Gly Tyr Val Leu Tyr Ala Val Leu Val
   325 330 335
   His Ser Gly Trp Ser Cys Glu Arg Gly His Tyr Phe Cys Tyr Val Arg
   340 345 350
   Ala Gly Asn Gly Gln Trp Tyr Lys Met Asp Asp Ala Lys Val Thr Ala
   355 360 365
   Cys Asp Glu Thr Ala Ala Leu Ser Gln Ser Ala Tyr Val Leu Phe Tyr
   370 375 380
   Ala Arg Glu Gly Ala Trp Glu Gly Gly Ala Gly Gly Gly Ala Ala Ala
   385 390 395 400
   Pro Val Gly Ala Asp Pro Thr Glu Pro Gly Gln Pro Ala Gly Asp Ala
   405 410 415
   Ser Gly Arg Ala Pro Gly Ser Glu Glu Ser Pro Gly Asp Thr Glu Val
   420 425 430
   Glu Gly Met Ser Leu Glu Gln Trp Arg Arg Leu Gln Glu His Ser Arg
   435 440 445
   Pro Lys Pro Ala Leu Glu Leu Arg Lys Val Gln Ser Ala Leu Pro Ala
   450 455 460
   Gly Ala Val Val Ile His Gln Ser Lys His Gly Gly Gly Arg Asn Arg
   465 470 475 480
   Thr Pro Pro Gln Gln Glu His Glu Arg Leu Asp Arg Pro Ser Thr Asp
   485 490 495
   Thr Pro Pro Pro Gly Pro Lys Asn Val Gly Asn Gly Pro Cys Ala Gly
   500 505 510
   Gly Arg Ala Arg Ala Thr Lys Gly Lys Asn Lys Lys Pro Arg Pro Ser
   515 520 525
   Leu Gly Leu Trp Arg
   530
<210> 19
   <211> 535
   <212> PRT
   <213> Canis lupus familiaris
<400> 19
   Met Glu Ala Ala His Leu His Pro Ser Glu Glu Pro Gln Phe Ser Ala
   1 5 10 15
   Ser Pro Lys Pro Gln Ser Tyr Trp Ser Arg Gly Gly Gly Ala Glu Val
   20 25 30
   His Gly Gly Pro Ser Val Pro Glu Thr Thr Ser Pro Ala Ser Lys Thr
   35 40 45
   Leu Ser Ser Pro Thr Asp Pro Leu Ala Pro Thr Ser Ala Gly Leu Pro
   50 55 60
   Pro Thr Lys Thr Pro Leu Ser Trp Arg Ser Leu Ser Gln Val Gly Ala
   65 70 75 80
   Gly Leu Gln Asn Met Gly Asn Thr Cys Tyr Val Asn Ala Thr Leu Gln
   85 90 95
   Cys Leu Thr Tyr Thr Glu Pro Leu Ala Ser Tyr Met Leu Ser Gln Gln
   100 105 110
   His Gly Thr Thr Cys Arg Arg Gln Thr Ser Cys Met Leu Cys Thr Leu
   115 120 125
   Gln Ala His Leu Thr Arg Val Leu Cys His Pro Gly Arg Val Leu Arg
   130 135 140
   Pro Leu Pro Leu Leu Leu Ala Ala Phe His Arg His Lys Gln Glu Asp
   145 150 155 160
   Ala His Glu Tyr Leu Met Phe Ile Leu Asp Ala Met Gln Gln Ala Cys
                   165                 170                 175
   Leu Pro Glu Asp Lys Leu Ser Asp Pro Glu Cys Pro Gln Asp Ser Thr
               180                 185                 190
   Leu Ile Gln Gln Leu Phe Gly Gly Tyr Trp Arg Ser Gln Ile Gln Cys
           195                 200                 205
   Leu His Cys Gln Gly Ile Ser Ser Thr Leu Glu Pro Tyr Leu Asp Ile
       210                 215                 220
   Ser Leu Asp Ile Gly Ala Ala His Ser Ile Ser Gln Ala Leu Glu Gln
   225                 230                 235                 240
   Leu Met Lys Pro Glu Leu Leu Glu Gly Glu Asn Ala Tyr His Cys Ser
                   245                 250                 255
   Lys Cys Leu Glu Lys Val Pro Ala Ser Lys Val Leu Thr Leu His Thr
               260                 265                 270
   Ser Pro Lys Val Leu Ile Leu Val Leu Arg Arg Phe Ser Asp Leu Thr
           275                 280                 285
   Gly Asn Lys Met Thr Lys Glu Val Gln Tyr Pro Glu Arg Leu Asp Met
       290                 295                 300
   Gln His Tyr Leu Ser Glu Gln Arg Ala Gly Pro Leu Val Tyr Val Leu
   305                 310                 315                 320
   Tyr Ala Val Leu Val His Ala Gly Arg Ser Cys His Ser Gly His Tyr
                   325                 330                 335
   Phe Cys Phe Val Lys Ala Gly Asn Gly Gln Trp Tyr Lys Met Asp Asp
               340                 345                 350
   Ala Lys Val Ser Ala Cys Asp Val Thr Cys Ala Leu Arg Gln Pro Ala
           355                 360                 365
   Tyr Val Leu Phe Tyr Met Gln Lys Thr Asp Leu Glu Arg Asp Leu Gly
       370                 375                 380
   Arg Glu Ser Val Glu Glu Gly Gly Leu Ala Ser Pro Glu Ala Asp Pro
   385                 390                 395                 400
   Thr Val Val Gly Glu Ala Ser Gly Glu Pro Ala Thr Asp Pro Ser Gly
                   405                 410                 415
   Asn His Pro Glu Leu Glu Glu Arg Gly Glu Glu Thr Ser Arg Gln Gln
               420                 425                 430
   Met Thr Leu Asp Gln Trp Arg Cys Leu Gln Glu Cys Asn Arg Pro Lys
           435                 440                 445
   Pro Glu Leu His Val Arg Arg Arg Glu Ile Ala Leu Pro Ala Asn Ala
       450                 455                 460
   Val Ile Leu His His Ser Lys Tyr Arg Pro Glu Met Pro Lys Asn His
   465                 470                 475                 480
   Pro Gln Pro Thr Val Asp Leu Leu Thr Thr Ala Ala Gly Met Leu Pro
                   485                 490                 495
   Pro Gln Val Ala Gly Asp Met Ala Lys Val Pro Arg Val Pro Gly Arg
               500                 505                 510
   Ala Arg Pro Thr Lys Arg Thr Ser Lys Lys Gly Gln Arg Ser Gly Glu
           515                 520                 525
   Ala Val Gln Gly Cys Val Ser
       530                 535
<210> 20
   <211> 1593
   <212> DNA
   <213> Homo sapiens
<400> 20
   atggaggacg actcactcta cttgggaggt gagtggcagt tcaaccactt ttcaaaactc      60
   acatcttctc ggccagatgc agcttttgct gaaatccagc ggacttctct ccctgagaag     120
   tcaccactct catgtgagac ccgtgtcgac ctctgtgatg atttggctcc tgtggcaaga     180
   cagcttgctc ccagggagaa gcttcctctg agtagcagga gacctgctgc ggtgggggct     240
   gggctccaga atatgggaaa tacctgctac gtgaacgctt ccttgcagtg cctgacatac     300
   acaccgcccc ttgccaacta catgctgtcc cgggagcact ctcaaacgtg tcatcgtcac     360
   aagggctgca tgctctgtac gatgcaagct cacatcacac gggccctcca caatcctggc     420
   cacgtcatcc agccctcaca ggcattggct gctggcttcc atagaggcaa gcaggaagat     480
   gcccatgaat ttctcatgtt cactgtggat gccatgaaaa aggcatgcct tcccgggcac     540
   aagcaggtgg atcatcactc taaggacacc accctcatcc accaaatatt tggaggctac     600
   tggagatctc aaatcaagtg tctccactgc cacggcattt cagacacttt tgacccttac     660
   ctggacatcg ccctggatat ccaggcagct cagagtgtcc agcaagcttt ggaacagttg     720
   gtgaagcccg aagaactcaa tggagagaat gcctatcatt gtggtgtttg tctccagagg     780
   gcgccggcct ccaagacgtt aactttacac acctctgcca aggtcctcat ccttgtattg     840
   aagagattct ccgatgtcac aggcaacaag attgccaaga atgtgcaata tcctgagtgc     900
   cttgacatgc agccatacat gtctcagccg aacacaggac ctctcgtcta tgtcctctat     960
   gctgtgctgg tccacgctgg gtggagttgt cacaacggac attacttctc ttatgtcaaa    1020
   gctcaagaag gccagtggta taaaatggat gatgccgagg tcaccgcctc tagcatcatt    1080
   tctgtcctga gtcaacaggc ctacgtcctc ttttacatcc agaagagtga atgggaaaga    1140
   cacagtgaga gtgtgtcaag aggcagggaa ccaagtgccc ttggcgcaga agacacagac    1200
   aggcgagcaa cgcaaggaga gctcaagaga gaccacccct gcctccaggc ccccgagttg    1260
   gacgagcact tggtggaaag agccactcag gaaagcacct tagaccactg gaaattcctt    1320
   caagagcaaa acaaaacgaa gcctgagttc aacgtcagaa aagtcgaagg taccctgcct    1380
   cccgacgtac ttgtgattca tcaatcaaaa tacaagtgtg ggatgaagaa ccatcatcct    1440
   gaacagcaaa gctccctgct aaacctctct tcgacgaccc cgacacatca ggagtccatg    1500
   aacactggca cactcgcttc cctgcgaggg agggccagga gatccaaagg gaagaacaaa    1560
   cacagcaaga gggctctgct tgtgtgccag tga                                 1593
<210> 21
   <211> 1885
   <212> DNA
   <213> Mus musculus
<400> 21
   atggtggttt ctctttcctt cccagaagag actggagggg aaaatcttcc ttctgctccc      60
   ttagaagact ccagcaagtt ctttgaagag gtctttggag acatggtggt tgctctttcc     120
   ttcccagaag cagatccagc actatcatct cctgatgccc cagagctgca tcaggatgaa     180
   gctcaggtgg tggaggagct aactaccaat ggaaagcaca gtctgagttg ggagagtccc     240
   caaggaccag gatgcgggct ccagaacaca ggcaacagct gctacctgaa cgcagccctg     300
   cagtgcttga cacacacacc acctctagct gactacatgc tgtcccagga gcacagtcaa     360
   acctgttgtt ccccagaagg ctgtaagatg tgtgctatgg aagcccatgt gacccagagt     420
   ctcctgcact ctcactcggg ggatgtcatg aagccctccc agattttgac ctctgccttc     480
   cacaagcacc agcaggaaga tgcccatgag tttctcatgt tcaccttgga aacaatgcat     540
   gaatcctgcc ttcaagtgca cagacaatca gatcccaccc ctcaggatac gtcacccatt     600
   catgacatat ttggaggctg gtggaggtct cagatcaagt gtctccattg ccagggcacc     660
   tcacatacct tcgatccctt cctggatgtc cccctggata tcagctcagc tcagagtgta     720
   aatcaagcct tgtgggatac agggaagtca gaagagctac ttggagagaa tgcctactac     780
   tgtggtaggt gtagacagaa gatgccagct tctaagaccc tgcatgttca tattgctcca 840
   aaggtactcc tgctagtgtt aaagcgcttc tcagccttca cgggtaacaa gttagacaga 900
   aaagtaagct acccggagtt ccttgacctg aagccatacc tgtctgagcc tactggagga 960
   cctttgcctt atgccctcta tgccgtcctg gtccatgatg gtgcgacttc taacagtgga 1020
   cattacttct gttgtgtcaa agctggtcat gggaagtggt acaagatgga tgatactaag 1080
   gtcaccaggt gtgatgtgac ttctgtcctg aatgagaatg cctatgtgct cttctatgtg 1140
   cagcagaccg acctcaaaca ggtcagtatt gacatgccag agggcagagt acatgaggtt 1200
   cttgacccta aataccagct gaagaaatcc cggagaaaaa agcgtaagaa gcaatgccat 1260
   tgcacagatg atgccggaga ggcatgcgaa aacagggaga agagagcaaa gaaagaaacc 1320
   tccttagggg aggggaaagt gcctcaggaa gtgaaccacg agaaagctgg gcagaaacat 1380
   gggaatacca aactcgtgcc tcaggaacag aaccaccaga gagctgggca gaacctcagg 1440
   aatactgaag ttgaacttga tctgcctgtt gatgcaattg tgattcacca gcccagatcc 1500
   acagcaaact ggggcacgga tgctccagac aaagagaatc aaccctggca caatggtgac 1560
   aggctcctca cctctcaggg cctcatgagc cctgggcagc tctgtagtca gggtgggaga 1620
   tgaagatcga agaaggggaa gaacaagaac aagcaagggc agaggcttct gcttgtttgc 1680
   tagtgctcac ccacccactc acacaggctc ctgtggacac actgttgacc caaggtgcct 1740
   ggaacaagag gtttggatct ctgtttcagg cagggacaat gccttaccct tcacgtgggg 1800
   tccacatttc ctctgggtcc ttgcctgttt ttgctgactg actctctgat cgtttgaatg 1860
   tggaaaaaat gcccaggatg ttggt 1885
<210> 22
   <211> 1593
   <212> DNA
   <213> Homo sapiens
<400> 22
   atggaggacg actcactcta cttgggaggt gagtggcagt tcaaccactt ttcaaaactc      60
   acatctcctc ggcccgatgc agcttttgct gaaatccagc ggacttctct ccctgagaag     120
   tcaccactct catgtgagac ccgtgtcgac ctctgtgatg atttggctcc tgtggcaagg     180
   cagcttgctc ccagggagaa gcttcctctg agtagcagga gacctgctgc ggtgggggct     240
   gggcttcaga atatgggaaa tacctgctac gtgaacgctt ccttgcagtg cctgacatac     300
   acaccgcccc ttgccaacta catgctgtcc cgggagcact ctcaaacgtg tcatcgtcac     360
   aagggctgca tgctctgtac tatgcaagct cacatcacac gggccctcca caatcctggc     420
   cacgtcatcc agccctcaca ggcattggct gctggcttcc atagaggcaa gcaggaagat     480
   gcccatgaat ttctcatgtt cactgtggat gccatgaaaa aggcatgcct tcccgggcac     540
   aagcaggtgg atcatcactc taaggacacc accctcatcc accaaatatt tggaggctac     600
   tggagatctc aaatcaagtg tctccactgc cacggcattt cagacacttt tgacccttac     660
   ctggacatcg ccctggatat ccaggcagct cagagtgtcc agcaagcttt ggaacagttg     720
   gtgaagcccg aagaactcaa tggagagaat gcctatcatt gtggtgtttg tctccagagg     780
   gcgccggcct ccaagacgtt aactttacac acctctgcca aggtcctcat ccttgtattg     840
   aagagattct ccgatgtcac aggcaacaag attgccaaga atgtgcaata tcctgagtgc     900
   cttgacatgc agccatacat gtctcagcag aacacaggac ctcttgtcta tgtcctctat     960
   gctgtgctgg tccacgctgg gtggagttgt cacaacggac attacttctc ttatgtcaaa    1020
   gctcaagaag gccagtggta taaaatggat gatgccgagg tcaccgcctc tagcatcact    1080
   cctgtcctga ctcaacaggc ctacgtcctc ttttacatcc agaagagtga atgggaaaga    1140
   cacagtgaga gtgtgtcaag aggcagggaa ccaagagccc ttggcgcaga agccacagac    1200
   aggcgagcaa cgcaaggaga gctcaagaga gaccacccct gcctccaggc ccccgagttg    1260
   gacgagcact tggtggaaag agccactcac gaaagcacct tagaccactg gaaattcctt    1320
   caagagcaaa acaaaacgaa gcctgagttc aacgtcagaa aagtcgaagg taccctgcct    1380
   cccgacgtac ttgtgattca tcaatcaaaa tacaagtgtg ggatgaagaa ccatcatcct    1440
   gaacagcaaa gctccctgct aaacctctct tcgacgaccc cgacacatca ggagtccatg    1500
   aacactggca cactcgcttc cctgcgaggg ggggccagga gatccaaagg gaagaacaaa    1560
   cacagcaaga gggctctgct tgtgtgccag tga                                 1593
<210> 23
   <211> 1593
   <212> DNA
   <213> Homo sapiens
<400> 23
   atgggggatg actcactcta cttgggaggt gagtggcagt tcaaccactt ttcaaaactc      60
   acatcttctc ggccagatgc agcttttgct gaaatccagc ggacttctct ccctgagaag     120
   tcaccactct catctgagac ccgtgtcgac ctctgtgatg atttggctcc tgtggcaaga     180
   cagctcgctc ccagggagaa gcttcctctg agtagcagga gacctgctgc ggtgggggct     240
   gggctccaga atatgggaaa tacctgctac gagaacgctt ccctgcagtg cctgacatac     300
   acactgcccc ttgccaacta catgctgtcc cgggagcact ctcaaacatg tcagcgtccc     360
   aagtgctgca tgctctgtac tatgcaagct cacatcacat gggccctcca cagtcctggc     420
   catgtcatcc agccctcaca ggcattggct tctggcttcc atagaggcaa gcaggaagat     480
   gtccatgaat ttctcatgtt cactgtggat gccatgaaaa aggcatgcct tcccggccac     540
   aagcaggtag atcatcactc taaggacacc accctcatcc accaaatatt tggaggctgc     600
   tggagatctc aaatcaagtg tctccactgc cacgggattt cagacacttt tgacccttac     660
   ctggacatcg ccctggatat ccaggcagct cagagtgtca agcaagcttt ggaacagttg     720
   gtgaagcccg aagaactcaa tggagagaat gcctatcatt gcggtctttg tctccagagg     780
   gcgccggcct ccaacacgtt aactttacac acttctgcca aggtcctcat ccttgtcttg     840
   aagagattct ccgatgtcgc aggcaacaaa cttgccaaga atgtgcaata tcctgagtgc     900
   cttgacatgc agccatacat gtctcagcag aacacaggac ctcttgtcta tgtcctctat     960
   gctgtgctgg tccacgctgg gtggagttgt cacgacggac attacttctc ttatgtcaaa    1020
   gctcaagaag gccagtggta taaaatggat gatgccgagg tcactgtctg tagcatcact    1080
   tctgtcctga gtcaacaggc ctatgtcctc ttttacatcc agaagagtga atgggaaaga    1140
   cacagtgaga gtgtgtcaag aggcagggaa ccaagagccc tcggcgctga agacacagac    1200
   aggcgagcaa agcaaggaga gctcaagaga gaccacccct gcctccaggc acccgagttg    1260
   gacgagcact tggtggaaag agccactcag gaaagcacct tagaccactg gaaattcctc    1320
   caagagcaaa acaaaacgaa gcctgagttc aacgtcggaa aagtcgaagg taccctgcct    1380
   cccaacgcac ttgtgattca tcaatcaaaa tacaagtgtg ggatgaaaaa ccatcatcct    1440
   gaacagcaaa gctccctgct aaacctctct tcgacgaccc ggacagatca ggagtccatg    1500
   aacactggca cactcgcttc tctgcaaggg aggaccagga gagccaaagg gaagaacaaa    1560
   cacagcaaga gggctctgct tgtgtgccag tga                                 1593
<210> 24
   <211> 1593
   <212> DNA
   <213> Homo sapiens
<400> 24
   atggaagacg actcactcta tttgggaggt gactggcagt tcaatcactt ttcaaaactc 60
   acatcttctc ggctagatgc agcttttgct gaaatccagc ggacttctct ctctgaaaag 120
   tcaccactct catctgagac ccgtttcgac ctctgtgatg atttggctcc tgtggcaaga 180
   cagcttgctc ccagggagaa gcttcctctg agtagcagga gacctgctgc ggtgggggct 240
   gggctccaga agataggaaa taccttctat gtgaacgttt ccctgcagtg cctgacatac 300
   acactgccgc tttccaacta catgctgtcc cgggaggact ctcaaacgtg tcatcttcac 360
   aagtgctgca tgttctgtac tatgcaagct cacatcacat gggccctcca cagtcctggc 420
   catgtcatcc agccctcaca ggtattggct gctggcttcc atagaggtga gcaggaggat 480
   gcccatgaat ttctcatgtt tactgtggat gccatgaaaa aggcatgcct tcccgggcac 540
   aagcagctag atcatcactc caaggacacc accctcatcc accaaatatt tggagcgtat 600
   tggagatctc aaatcaagta tctccactgc cacggcgttt cagacacctt tgacccttac 660
   ctggacatcg ccctggatat ccaggcagct cagagtgtca agcaagcttt ggaacagttg 720
   gtgaagccca aagaactcaa tggagagaat gcctatcatt gtggtctttg tctccagaag 780
   gcgcctgcct ccaagacgtt aactttaccc acttctgcca aggtcctcat tcttgtattg 840
   aagagattct ccgatgtcac aggcaacaaa cttgccaaga atgtgcaata tcctaagtgc 900
   cgtgacatgc agccatacat gtctcagcag aacacaggac ctcttgtcta tgtcctctat 960
   gctgtgctgg tccacgctgg gtggagttgt cacaacggac attacttctc ttatgtcaaa 1020
   gctcaagaag gccagtggta taaaatggat gatgccgagg tcactgcctc tggcatcacc 1080
   tctgtcctga gtcaacaggc ctatgtcctc ttttacatcc agaagagtga atgggaaaga 1140
   cacagtgaga gtgtgtcaag aggcagggaa ccaagagccc ttggtgctga agacacagac 1200
   aggccagcaa cgcaaggaga gctcaagaga gaccacccct gcctccaggt acccgagttg 1260
   gacgagcact tggtggaaag agccactcag gaaagcacct tagaccactg gaaattcccc 1320
   caagagcaaa acaaaacgaa gcctgagttc aacgtcagaa aagttgaagg taccctgcct 1380
   cccaacgtac ttgtgattca tcaatcaaaa tacaagtgtg gtatgaaaaa ccatcatcct 1440
   gaacagcaaa gctccctgct aaacctctct tcgacgaaac cgacagatca ggagtccatg 1500
   aacactggca cactcgcttc tctgcaaggg agcaccagga gatccaaagg gaataacaaa 1560
   cacagcaaga gatctctgct tgtgtgccag tga 1593
<210> 25
   <211> 1593
   <212> DNA
   <213> Homo sapiens
<400> 25
   atgggggacg actcactcta cttgggaggt gagtggcagt tcaaccactt ttcaaaactc      60
   acatcttctc ggccagatgc agcttttgct gaaatccagc ggacttctct ccctgagaag     120
   tcaccactct catctgagac ccgtgtcgac ctctgtgatg atttggctcc tgtggcaaga     180
   cagctcgctc ccagggagaa gcttcctctg agtagcagga gacctgctgc ggtgggggct     240
   gggctccaga atatgggaaa tacctgctac gagaacgctt ccctgcagtg cctgacatac     300
   acactgcccc ttgccaacta catgctgtcc cgggagcact ctcaaacatg tcagcgtccc     360
   aagtgctgca tgctctgtac tatgcaagct cacatcacat gggccctcca cagtcctggc     420
   catgtcatcc agccctcaca ggcattggct gctggcttcc atagaggcaa gcaggaagat     480
   gtccatgaat ttctcatgtt cactgtggat gccatgaaaa aggcatgcct tcccggccac     540
   aagcaggtag atcatcactc taaggacacc accctcatcc accaaatatt tggaggctgc     600
   tggagatctc aaatcaagtg tctccactgc cacgggattt cagacacttt tgacccttac     660
   ctggacatcg ccctggatat ccaggcagct cagagtgtca agcaagcttt ggaacagttg     720
   gtgaagcccg aagaactcaa tggagagaat gcctatcatt gcggtctttg tctccagagg     780
   gcgccggcct ccaacacgtt aactttacac acttctgcca aggtcctcat ccttgtcttg     840
   aagagattct ccgatgtcgc aggcaacaaa cttgccaaga atgtgcaata tcctgagtgc     900
   cttgacatgc agccatacat gtctcagcag aacacaggac ctcttgtcta tgtcctctat     960
   gctgtgctgg tccacgctgg gtggagttgt cacgacggat attacttctc ttatgtcaaa    1020
   gctcaagaag gccagtggta taaaatggat gatgccgagg tcactgtctg tagcatcact    1080
   tctgtcctga gtcaacaggc ctatgtcctc ttttacatcc agaagagtga atgggaaaga    1140
   cacagtgaga gtgtgtcaag aggcagggaa ccaagagccc ttggcgctga agacacagac    1200
   aggccagcaa cgcaaggaga gctcaagaga gaccacccct gcctccaggt acccgagttg    1260
   gacgagcact tggtggaaag agccactgag gaaagcacct tagaccactg gaaattcccc    1320
   caagagcaaa acaaaatgaa gcctgagttc aacgtcagaa aagttgaagg taccctgcct    1380
   cccaacgtac ttgtgattca tcaatcaaaa tacaagtgtg ggatgaaaaa ccaccatcct    1440
   gaacagcaaa gctccctgct aaacctctct tcgatgaact cgacagatca ggagtccatg    1500
   aacactggca cactcgcttc tctgcaaggg aggaccagga gatccaaagg gaagaacaaa    1560
   cacagcaaga gatctctgct tgtgtgccag tga                                 1593
<210> 26
   <211> 2661
   <212> DNA
   <213> Mus musculus
<400> 26
   aggaaaaact tccttctgct cccttagaag actccagcta gttatttgaa gaggtctttg      60
   tagacacggt ggttgctctt tcctcccaag aagagattct ctagaaggga aaaacttcct     120
   tctgctccct tagaagacta cagcaagttc tttgaagagg tctttggaga catggtggtt     180
   gctctttcct tcccagaagc agatccagcc ctatcatctc ctgatgcccc agagctgcat     240
   caggatgaag ctcaggtggt ggaggagcta actgtcaatg gaaagcacag tctgagttgg     300
   gagagtcccc aaggaccagg atgcgggctc cagaacacag gcaacagctg ctacctgaat     360
   gcagccctgc agtgcttgac acacacacca cctctagctg actacatgct gtcccaggag     420
   cacagtcaaa cctgttgttc cccagaaggc tgtaagttgt gtgctatgga agcccttgtg     480
   acccagagtc tcctgcactc tcactcgggg gatgtcatga agccctccca tattttgacc     540
   tctgccttcc acaagcacca gcaggaagat gcccacgagt ttctcatgtt caccttggaa     600
   acaatgcatg aatcctgcct tcaagtgcac agacaatcaa aacccacctc tgaggacagc     660
   tcacccattc atgacatatt tggaggctgg tggaggtctc agatcaagtg tctcctttgc     720
   cagggtacct cagataccta tgatcgcttc ctggacatcc ccctggatat cagctcagct     780
   cagagtgtaa agcaagcctt gtgggataca gagaagtcag aagagctatg tggagataat 840
   gcctactact gtggtaagtg tagacagaag atgccagctt ctaagaccct gcatgttcat 900
   attgctccaa aggtactcat ggtagtgtta aatcgcttct cagccttcac gggtaacaag 960
   ttagacagaa aagtaagtta cccggagttc cttgacctga agccatacct gtctgagcct 1020
   actggaggac ctttgcctta tgccctctat gccgtcctgg tccatgatgg tgcgacttct 1080
   cacagtggac attacttctg ttgtgtcaaa gctggtcatg ggaagtggta caagatggat 1140
   gatactaaag tcaccaggtg tgatgtgact tctgtcctga atgagaatgc ctatgtgctc 1200
   ttctatgtgc agcaggccaa cctcaaacag gtcagtattg acatgccaga gggaagaata 1260
   aatgaggttc ttgaccctga ataccagctg aagaaatcac ggagaaaaaa gcataagaag 1320
   aaaagccctt tcacagaaga tttaggagag ccctgcgaaa acagggataa gagagcaatt 1380
   aaagaaacct ccttaggaaa ggggaaagtg cttcaggaag tgaaccacaa gaaagctggg 1440
   cagaaacacg ggaataccaa actcatgcct cagaaacaga accaccagaa agctgggcag 1500
   aacctcagga atactgaagt tgaacttgat ctgcctgctg atgcaattgt gattcaccag 1560
   cccagatcca ctgcaaactg gggcagggat tctccagaca aggagaatca acccttgcac 1620
   aatgctgaca ggctcctcac ctctcagggc cctgtgaaca cttggcagct ctgtagacag 1680
   gaagggagac gaagatcgaa gaaggggcag aacaagaaca agcaagggca gaggcttctg 1740
   cttgtttgct agtgatcacc cacccactca cacaggctcc tgtggacaca ctgttgaccc 1800
   aaggtgcctg gaacaagagg tttggatctc tgtttcaggc agggacaatg cctcaccctt 1860
   cacgtggggt ccacctatcc tctgggccct tgcctgtttt tgctgactga ctctctgatt 1920
   gtttgaatgt ggaaaaaaag tgcccaggat gttggtacag gttaaagaca agaagctgga 1980
   cacccggagg aggtctgaat agcctctcct gcaactcatg gaatctgagc agcatagaga 2040
   ctaaatcacc acactggagc tttcttttct tttcttttct tttcttttct tttcttttct 2100
   tttcttttct cttctcttct cttctcttct cttctcttct cttctcttct cttctcttct 2160
   cttctcttct ctcctctcct ctcctctcct ctcctctcct ctcctctcct ctcctttcct 2220
   ttcctttcct ttcctttttt tttaaattta ttttttgtta ttagatattt tctttattta 2280
   catttcaaat gctatcccaa aagttcccta taccctcccc caactctgcc accctaccca 2340
   cccactccca cttcttggct ctggcatttc cctgtactgg ggcatataaa gtttgcaata 2400
   ccaaagggcc tctcttccca atgatggcca actaggtcac cttctgctac atatgcagct 2460
   agagacccta agaaaacaca ctggaactct tgaggtttgg agttttcgct caggcaacaa 2520
   gttgcttttc aactgccctt tctaacctct tacccagaaa atgtgtagtt caccctgtag 2580
   agatagatgc tcttattctt agtgtgtgat caacagttct ttggtcaaat aaattctgtt 2640
   acttcacaaa aaaaaaaaaa a 2661
<210> 27
   <211> 1407
   <212> DNA
   <213> Mus musculus
<400> 27
   atggtggttg ctctctcctt cccagaagca gatccagcca tgtcacctcc tagtgcccca      60
   gagctgcatc aggatgaagc ccaggtggta gaggagctgg ctgccaatgg aaagcacagt     120
   ctgagttggg agagtcccca aggaccagga tgcgggctcc agaacacagg caacagctgc     180
   tacctgaatg cagccctgca gtgcttgaca cacacaccac ctctagctga ctacatgctg     240
   tcccaggagc acagtcaaac ctgttgttcc ccagaaggct gtaagatgtg tgctatggaa     300
   gcccatgtga cccagagtct cctgcacacc cactcagggg atgttatgaa gccctcccag     360
   aatttgacct ctgccttcca caagcgcaag caggaagatg cccatgagtt tctcatgttc     420
   accttggaaa caatgcatga atcctgcctt caagtgcaca gacaatcaga acccacctct     480
   gaggacagct cacccattca tgacatattt ggaggctggt ggaggtctca gatcaagtgt     540
   caccactgcc agggcacctc atattcctat gatcccttcc tggacatccc cctggatatc     600
   agctcagttc agagtgtgaa gcaagccttg caggatacag agaaggcaga agagctatgt     660
   ggggagaatt cctactactg tggtaggtgt agacaaaaga agccagcttc caagacccta     720
   aagctttata gtgccccaaa ggtactcatg ctagtgttaa agcgcttttc gggctctatg     780
   ggtaaaaagt tggacagaaa agtaagctac ccagagttcc ttgacctgaa gccatacctg     840
   tcccagccta ctggagggcc tttgccttat gccctctatg ccgtcctggt ccatgaaggt     900
   gcaacttgtc acagtggaca ttacttctgt tgtgtcaaag ctggccatgg gaagtggtac     960
   aagatggatg atactaaggt caccagctgt gatgtgactt ctgtcctgaa tgagaatgcc    1020
   tatgtgctct tctatgtgca gcagaatgac ctcaaaaagg gtagtatcaa catgccagag    1080
   ggcagaatac atgaggttct tgatgccaaa taccagctga agaaatcagg ggaaaaaaag    1140
   cataataaaa gcccttgcac agaagatgca ggagagccct gcgaaaacag ggagaagaga    1200
   tcatccaaag aaacctcctt aggggagggg aaagttcttc aggaacagga ccaccagaaa    1260
   gctgggcaga aacaagagaa taccaaactc acgcctcagg aacagaacca cgagaaaggt    1320
   gggcagaacc tcaggaatac tgaaggtgaa cttgatcgac tcagtggtgc aattgtggtt    1380
   taccaaccta tatgcactgc aaactga                                        1407
<210> 28
   <211> 1697
   <212> DNA
   <213> Mus musculus
<400> 28
   atttgaagag gtctttggag acatggtggt ttctctttcc ttcccagaag cagatccagc      60
   cctatcatct cctggtgccc aacagctgca tcaggatgaa gctcaggtag tggtggagct     120
   aactgccaat gacaagccca gtctgagttg ggaatgtccc caaggaccag gatgcgggct     180
   tcagaacaca ggcaacagct gctacctgaa tgcagccctg cagtgcttga cacacacacc     240
   acctctagct gactacatgc tgtcccagga gtacagtcaa acctgttgtt ccccagaagg     300
   ctgtaagatg tgtgctatgg aagcccatgt aacccagagt ctcctgcact ctcactcggg     360
   ggatgtcatg aagccctccc agattttgac ctctgccttc cacaagcacc agcaggaaga     420
   tgcccatgag tttctcatgt tcaccttgga aacaatgcat gaatcctgcc ttcaagtgca     480
   cagacaatca gaacccacct ctgaggacag ctcacccatt catgacatat ttggaggctt     540
   gtggaggtct cagatcaagt gtctccattg ccagggtacc tcagatacat atgatcgctt     600
   cctggatgtc cccctggata tcagctcagc tcagagtgta aatcaagcct tgtgggatac     660
   agagaagtca gaagagctac gtggagagaa tgcctactac tgtggtaggt gtagacagaa     720
   gatgccagct tccaagaccc tgcatattca tagtgcccca aaggtactcc tgctagtgtt     780
   aaagcgcttc tcggccttca tgggtaacaa gttggacaga aaagtaagct acccagagtt     840
   ccttgacctg aagccatacc tgtcccagcc tactggagga cctttgcctt atgccctcta     900
   tgctgtcctg gtccatgaag gtgcgacttg tcacagtgga cattacttct cttatgtcaa     960
   agccagacat ggggcatggt acaagatgga tgatactaag gtcaccagct gcgatgtgac    1020
   ttctgtcctg aatgagaatg cctatgtgct cttctatgtg cagcagactg acctcaaaca    1080
   ggtcagtatt gacatgccag agggcagagt acatgaggtt ctcgaccctg aataccagct    1140
   gaagaaatcc cggagaaaaa agcataagaa gaaaagccct tgcacagaag atgcgggaga    1200
   gccctgcaaa aacagggaga agagagcaac caaagaaacc tccttagggg aggggaaagt    1260
   gcttcaggaa aagaaccaca agaaagctgg gcagaaacat gagaatacca aacttgtgcc    1320
   tcaggaacag aaccaccaga aacttgggca gaaacacagg atcaatgaaa tcttgcctca    1380
   ggaacagaac caccagaaag ctgggcagag cctcaggaac acggaaggtg aacttgatct    1440
   gcctgctgat gcaattgtga ttcacctgct cagatccaca gaaaactggg gcagggatgc    1500
   tccagacaag gagaatcaac cctggcacaa tgctgacagg ctcctcacct ctcaggaccc    1560
   tgtgaacact gggcagctct gtagacagga aggaagacga agatcaaaga aggggaagaa    1620
   caagaacaag caagggcaga ggcttctgct tgtttgctag tgttcactca cccactcaca    1680
   caggctcctg tggacac                                                   1697
<210> 29
   <211> 1638
   <212> DNA
   <213> Mus musculus
<400> 29
   atggtggttt ctctttcctt cccagaagca gatccagccc tatcatctcc tggtgcccaa 60
   cagctgcatc aggatgaagc tcaggtagtg gtggagctaa ctgccaatga caagcccagt 120
   ctgagttggg aatgtcccca aggaccagga tgcgggcttc agaacacagg caacagctgc 180
   tacctgaatg cagccctgca gtgcttgaca cacacaccac ctctagctga ctacatgctg 240
   tcccaggagt acagtcaaac ctgttgttcc ccagaaggct gtaagatgtg tgctatggaa 300
   gcccatgtaa cccagagtct cctgcactct cactcggggg atgtcatgaa gccctcccag 360
   attttgacct ctgccttcca caagcaccag caggaagatg cccatgagtt tctcatgttc 420
   accttggaaa caatgcatga atcctgcctt caagtgcaca gacaatcaga acccacctct 480
   gaggacagct cacccattca tgacatattt ggaggcttgt ggaggtctca gatcaagtgt 540
   ctccattgcc agggtacctc agatacatat gatcgcttcc tggatgtccc cctggatatc 600
   agctcagctc agagtgtaaa tcaagccttg tgggatacag agaagtcaga agagctacgt 660
   ggagagaatg cctactactg tggtaggtgt agacagaaga tgccagcttc caagaccctg 720
   catattcata gtgccccaaa ggtactcctg ctagtgttaa agcgcttctc ggccttcatg 780
   ggtaacaagt tggacagaaa agtaagctac ccggagttcc ttgacctgaa gccatacctg 840
   tcccagccta ctggaggacc tttgccttat gccctctatg ctgtcctggt ccatgaaggt 900
   gcgacttgtc acagtggaca ttacttctct tatgtcaaag ctggacatgg gaagtggtac 960
   aagatggatg atactaaggt caccagctgc gatgtgactt ctgtcctgaa tgagaatgcc 1020
   tatgtgctct tctatgtgca gcagactgac ctcaaagagg tcagtattga catgccagag 1080
   ggcagaatac atgaggttct cgaccctgaa taccagctga agaaatcccg gagaaaaaag 1140
   cataagaaga aaagcccttg cacagaagat gtgggagagc cctccaaaaa cagggagaag 1200
   aaagcaacca aagaaacctc cttaggggag gggaaagtgc ttcaggaaaa gaaccacaag 1260
   aaagctgggc agaaacacga gaataccaaa ctcgtgcctc aggaacagaa ccaccagaaa 1320
   ctggggcaga aacacaggaa caatgaaatc ttgcctcagg aacagaacca ccagaaaact 1380
   gggcagagcc tcaggaacac ggaaggtgaa cttgatctgc ctgctgatgc aattgtgatt 1440
   cacctgccca gatccatagc aaattggggc agggatactc cagacaaggt gaatcaaccc 1500
   tggcacaatg ctgacaggct cctcacctct caggaccttg tgaacactgg gcagctctgt 1560
   agacaggaag gaagacgaag atcgaagaag gggaagaaca agaacaagca agggcagaag 1620
   cttctgcttg ttcgctag 1638
<210> 30
   <211> 1650
   <212> DNA
   <213> Rattus Norvegicus
<400> 30
   atgcaatctg atttcactgc ttcagaagga gatagagcag gaattaagaa agtttttgcg      60
   attttctgga gggaaagtct tccttctgct cacttagaaa actcaagcag gttattttaa     120
   gatgatcata gaaatatggt gactgctcac tccttcacag aagaagatcc agccatgtca     180
   ccacctgcta ccccagagct ccatcaagat gaagcacggg tgctagagga gctgtctgcc     240
   aaggggaagc ccagtctgag tttgcagagg atccaaagcc cagggtcagg gctccagaac     300
   ataggcaaca gctgctacct gaatgcagtc ctgcagtgct tgacacacac accacctctt     360
   gccgactata tgctgtccca ggagcattct cagaggtgtt gttacccaga aggctgtaag     420
   atgtgtgcta tggaagctca tgtgacccag agtctcctcc actcccactc aggaggtgtc     480
   atgaaaccct ccgagatatt gacctctacc ttccacaagc acaggcagga agatgcccat     540
   gaatttctca tgttcacctt gaatgccatg catgaatcct gccttcgagg gtgcaagcaa     600
   tcagaaacct cctctaagga cagctccctg atctatgaca tatttggagg ccagatgaga     660
   tctcagatca agtgtcacca ctgccagggc accttagatt cctacgatcc cttcctgaac     720
   ctcttcctgg atatctgctc tgctcagagt gtgaagcaag ccttggagga cttagtgaag     780
   ctagaagagt tgcaggggga caacgcctac tactgtggta ggtgtagaga gaagatgcca     840
   gcttctaaga ccacgaaggt tcagactgcc tcaaaggttc tcctgctagt gttaaaccgc     900
   tcctacgatt tcgggggtga caagttgaac agggtagtaa gctacccaga gtaccttgac     960
   ctgcagccat acctgtcaca gccaactgca ggacccctgc cttatgctct ctatgccgtc    1020
   ctggtccatg atggggtgac ttgttccagt ggacattact tctgttatgt caaagccagc    1080
   catgggaagt ggtataagat ggatgattct aaggtcacca ggtgcgatgt gtcctctgtc    1140
   ctgagcgagc ctgcctatct gctcttctat gtccagcaga ctgaccttga gaaggtcaat    1200
   gttgatgtgt ctgtgggcag agtacatggg gttcttcacc ctgaatccca gcagaagaaa    1260
   acgcggaaga aaaagcacaa gagaagctct tgcacagaag ctgtacacat gccccgagaa    1320
   aacagggaaa atacagccac caaagaaacc tccttagggg aggggaaagt gcttcaggaa    1380
   cagaaccacc agaaagctgg gcagaacctc aagactacca aagtcaattt gtcagccaac    1440
   ggaactgtga ttcatcagcc cagatatacc gcaaactggg gcaggaatgc tccagacaag    1500
   gacgatcaac cagggcacag tggtgacaga ctcctcacca ctcagggctc catgaacact    1560
   gggcaactct gtggtcatgg agggagccaa agatctaaga agaggaagaa caagaacaag    1620
   caagggcaga ggcctctgct tgtttgctag                                      1650
<210> 31
   <211> 1407
   <212> DNA
   <213> Microtus ochrogaster
<400> 31
   atggcagctc ctgccgcccc agacctgcgt ccagatgaag ggctagtggt agcagagctg        60
   gctgccaggg ccaagcccag aatgagttgg gagagaatcc acagcgtggg tgcgggtctc       120
   cagaacactg gaaacagttg ctaccttaat gcagccttgc agtgtctgac gcacacgcca       180
   cctcttgcca actacatgct ctcccgggag cactctcaga gctgcggtca ccagggaggc       240
   tgtccaatgt gtgccatgga agctcatgtg acccagagtt tccgccactc cggggaggtc       300
   atgcagcctt caaagaagct gactggagcc ttccacaagc acaagcagga ggatgcccac       360
   gagtttctga tgttcacctt gaacgccatg cacgaatcct gccttcgagg gagcaagtac       420
   tcaggagccc catctgagaa cagcacccct atccacgcga tatttggagg ctcatggaga       480
   tctcagatca agtgtctcca ctgccagggc acctcagatt cctttaaccc gttcctggac       540
   atatccctgg atatccacgc ggctcagagt gtgaagcaag ccttggagga tttagtgcag       600
   gccgaagtgc tgtgtggaga aaatgcctac cactgtgacc actgccaggg gaagacgaca       660
   gcttcaaaga ccctgatggt ccaaactgcc ccgaaggttc tcatgctggt cttgaatcgc       720
   ttctcaggtt tcacgggcga caaagtcgac agaaaagtga gctaccctga gtcccttgac       780
   atgcggccat acatgactca gcctaataga ggaccatcgg tctatgtact ctatgctgtg       840
   ctggtccatg ccggtttgac gtgccacagt ggacattact tctgttatgt cagagctggc       900
   aatgggaagt ggtataagat ggacgactcg aaagtcgcca ggtgtgatgt gacttctgtc       960
   ctgagtgagc cagcctatgt gcttctctat gttcgggaga ctgaactcca aaaggacagc      1020
   gtcactgggc cagtagacac agttggccaa gaccgacaga gaaagctcaa cagaggatct      1080
   tgtgtgggag ctgcagagcc acgcaggccc gtggagagcg ctgcagccaa agaaatctcc      1140
   ttagaccagt ggaaagcgct tctggaacac acccgcccga atcccgcgct gaacctcagg      1200
   aaaactgagt ccactctgcc agttgatgct gttgttattc accagcccag acacagaggg      1260
   cactgggaca caaatggtcc cgacaaggag aattaccctt gtcacacttc aaccaggttg      1320
   ctgcctgctc agagagccat gggcactcag ggaggaagat ccagaaccaa gaagaacaag      1380
   caaaggtgga ggtctctggt tgtttaa                                          1407
<210> 32
   <211> 1335
   <212> DNA
   <213> Mesocricetus auratus
<400> 32
   atggacgtct cagtagatcc tgccctgtca tctcctgatc aaccagacct gccccaggag 60
   gaagctcagg tggtgccaga gctggctgtt agggaggagc acaggcttag ttggaagagg 120
   ccccatggtg tgggagctgg tctcgagaac accggtaata gctgctacct gaatgcagcc 180
   ctgcagtgtc tgacacacac accacctctt gccagctaca tgttgtcccg ggagcactct 240
   cagaactgtt gtcaccgagg agcctgcatg atgtgtgcta tggaagctca tgtgacccaa 300
   agtttcctct actctgggga tgtcatccag ccctcagaga tgctgactgc tgccttccac 360
   aagcacaggg aggaagacgc ccacgagttt ctgatgttca ccttgaatgc catgcacaca 420
   tcctgtctgc cagggagcaa gctcatggga tgcacatcta agcagagctc cataatccat 480
   gagatatttg gaggctcctg ggaatctaag atcaagtgtc tctgttgcca ggcgaccaca 540
   gacaccttag agcccttcct tgacatcact ctggatatcc aaactgctca gagtgtgaac 600
   caagccttgg aaaatttagt aaaggaggaa aagctctgtg gggaaaatgc ctaccattgt 660
   gacatttgtt ggaagaacac accggcttcc aagaccctga ttgtgaaaga tgccccacag 720
   gttctcttgc tggtgttgaa tcgcttcgaa gagttcacag gtgataaaaa ggacagggaa 780
   gtgagttact ctgagttcct tgacttccag ccatacgtat ctcagtcccc tagagaccca 840
   ttgctttatg tcctgtatgc tgtgctggtc catgatggta tgacttgtca cagcggtcat 900
   tacttctgtt atgtcagagc tggcaatggt cactggtata agatgaatga ttctagtgtc 960
   accaggtgtg atatgaaatc tgtcctaagt gagcctgcct acgtgctctt ctatgtccag 1020
   cagactgagc tcaaaaagaa tttatggatg ctcccacagg cagaacacca ggcaggggaa 1080
   tccaggcaca caacgatcaa cagaggatcc cccacagaag ctgaagaggc cccagatcac 1140
   atagagaata caacagtcca agacttctta ggccactgga aagcgccaaa gccattgacg 1200
   gactggagga agaaccatct tgacagggag aatagtccca tcaggcttct gccaggcttc 1260
   tgtctttctc accaggagac catggacact gggcagctct gtagtaaggg agagagacca 1320
   agatccaaga agtag 1335
<210> 33
   <211> 1449
   <212> DNA
   <213> Cricetulus griseus
<400> 33
   atgaagaaaa gacgtaggca tttgcaggag ggaaaggatc cttctgacca cagtcagcac 60
   tccagaacca tatctggaag caaccctgag gatatggagg ctgctaggga gctctcagta 120
   ggtgagtctc acagtaagtc actttcagtt tacatggcct caaccaaggc tgttggcact 180
   gaagtgtatt tgtcttcttg ccctgccaca gatcctaccc tgtcatctcc tgacgaacca 240
   aaccggcctc agaatgaagc tcaggtggta ccagagctgg ctgctaagga ggagttccat 300
   cttagttggc agaggcccca tgatgtggga gctggactcg agaacacagg taatagctgc 360
   tacatgaatg cagtactgca gtgtctgaca cacacaccac ctcttgtcaa ctacatgttg 420
   tctcgggagc actctcagaa ctgttgtcac caaggagact gcatgatttg tgctatggaa 480
   gctcatgtga cccggagtct cctctactct ggggatgtca tccagccctc agagaagttg 540
   actgctgcct tccacaagca caggcaggaa gatgcccatg agtttctgct gttcaccttg 600
   aatgccatgc acacatcctg tctgccaggg agcaagctcc tgggatgcac atctgagcag 660
   agctccctga tccatgagat atttggaggc tcctggaaat ctcagatcaa gtgtctccac 720
   tgcaatgaga ccacagacct cttagagccc ttccttgaca tcaccctgga tatccaaact 780
   gctcagagtg tgaaccaagc cttggaaaat ttagtaatgg aggaacagct gtgtggggaa 840
   aatgcctacc attgtgacaa ctgtaggcag aagacaatgg cttccaagac cctgactgtg 900
   aaagatgccc caaaggttct cttgctggtg ttgaatcgct tctcagagtt cacaggtgac 960
   aaaaaggaca ggaaagtgag ctatcctgag tcctttgact tccagcccta catatctcag 1020
   tcccatagac aaccattgtt ttatagcctg tatgctgtac tggtccatga tggtgtgact 1080
   tgtcacagtg gtcattactt ctgttatgtc aaagctggca atggtcactg gtataagatg 1140
   gatgattcta gtgtcaccag atgtgatgtc aattctgtcc taagtgaacc tgcttatgtg 1200
   ctcttttatg tccagcagac tgatctcaga acgaatttgt gggtgctctc acaggcagaa 1260
   caccaggtag gggaatcctg gtatacaacg atcaacagag gatcccccac agaagctgca 1320
   gagcccccgg atcacacaga gaatacagct gccaaaaatt tcttagacca ctggaaaact 1380
   cttctgaaca tgaacaccaa agcctttggt gaaacttgga aaacacagac ctactctgag 1440
   agcaaatga 1449
<210> 34
   <211> 1590
   <212> DNA
   <213> Nomascus leucogenys
<400> 34
   atggagcacg actcactcta ctcggggggt gagtggcact tcagccgctt ttcaaaactc 60
   acatcttctc ggccacatgc agcttttgct gaaatccagc ggacttctct ccctgagaag 120
   tcaccactct catctgagac ccgtgtcgac ccctgtgatg atttggctcc tgtggcaaga 180
   cagcttgctc ccagggagaa gcttcctctg agtagcaggg gtcctgctgc ggtgggggct 240
   gggctccaga atatgggaaa tacctgctac gtgaatgctt ccctgcagtg cctgacatac 300
   acaccgcccc ttgccaacta catgctgtcc cgggagcact ctcaaacttg tcatcgtcac 360
   aagtgctgca tgctctgtac tatgcaagct cacatcacac gggccctcca ccgtccaggc 420
   gatgtcatcc agccctcaca ggcattggct gctggcttcc atagaggcaa gcaggaagat 480
   gcccacgaat ttctcatgtt cactgtggat gccatgagaa aggcatgcct tcccgggcac 540
   aagcaggtag atcctcactc taaggacacc accctcatcc accaaatatt tggagggtac 600
   tggagatctc aaatcaagtg tctccactgc cagggcattt cagacacctt tgacccttac 660
   ctggacatcg ccctggatat ccaggcagct cagagtgtga agcaagcttt ggaacagttg 720
   gtgaagcccg aagaactcaa tggagagaat gcctatcatt gtggtctttg tctccagaag 780
   gcgcctgcct ccaagacgtt aactttacac acttctgcca aggtcctcat cctcgtactg 840
   aagagattct ccgatgtcac aggcaacaaa cttgccaaga atgtgcaata tcctgagtgc 900
   cttgacatgc agccatacat gtctcagcag aacacaggac ctcttgtcta tgtcctctac 960
   gctgtgctgg tccacgctgg gtggagttgt cacaacggac attacttctc ttatgtcaaa 1020
   gctcaagaag gccagtggta taaaatggat gatgccgagg tcactgcttc tggcatcact 1080
   tctgtcctga gtcaacaggc ctatgtcctc ttttatatcc agaagagtga attggaaaga 1140
   cacagtgagg gtgtgtcaag aggcagggaa ccaagagccc ttggccctgc agacacagac 1200
   aggcgagcaa cgcaaggaga gctcaagagg gaaccctgcc tccaggtacc cgagttggac 1260
   gagcactcgg tggaaagagc cactcaggaa agcaccttag accactggaa attcctccaa 1320
   gagcaaaaca aaacgaagcc tgagttcaac gtcagaaaag tcgaaggttc cctgcctccc 1380
   aacgtagttg tgattcatca atcaaaatac aagtgtggca cgaaaaacca tcatcctgaa 1440
   cagcaaagct ccctgctaaa cctctcttcg acgaacccga cagatcagga atccatcaac 1500
   actggcacac ccgcttctcg gcaagggagg accaggagat ccaaagggaa gaacaaacac 1560
   agcaacaggg ctctgcttct gtgccagtga 1590
<210> 35
   <211> 3360
   <212> DNA
   <213> Pan troglodytes
<400> 35
   atggaggacg actcactcta cttgggaggt gagtggcagt tcaaccactt ttcaaaactc      60
   acatcttctc ggccagatgc agcttttgct gaaatccagc ggacttctct ccctgagaag     120
   tcaccactct catctgagac ccgtgtcgac ctctgtgatg atttggctcc tgtggcaaga     180
   cagcttgctc ccggggagaa gcttcttctg agtagcagga gacctgctgc ggtgggggct     240
   gggctccaga atatgggaaa tacctgctac gtgaacgctt ccctgcagtg cctgacatac     300
   acaccgcccc ttgccaacta catgctgtcc cgggagcact ctcaaacgtg tcatcgtcac     360
   aagggctgca tgctctgtac tatgcaagct cacatcacac gggccctcca cattcctggc     420
   catgtcatcc agccctcaca ggcattggct gctggcttcc atagaggcaa gcaggaagat     480
   gcccatgaat ttctcatgtt cactgtggat gccatggaaa aggcatgcct tcccgggcac     540
   aagcaggtag agcatcactc taaggacacc accctcatcc accaaatatt tggaggctac     600
   tggagatctc aaatcaagtg tctccactgc cacggcattt cagacacttt tgacccttac     660
   ctggacatcg ccctggatat ccaggcagct cagagtgtcc agcaagcttt ggaacagttg     720
   gtgaagcccg aagaactcaa tggagagaat gcctatcatt gtggtctttg tctccagagg 780
   gcgccggcct ccaagacgtt aactttacac acttctgcca aggtcctcat ccttgtattg 840
   aagagattct ccgatgtcac aggcaacaaa cttgccaaga atgtgcaata tcctgagtgc 900
   cttgacatgc agccatacat gtctcagcag aacacaggac ctcttgtcta tgtcctctat 960
   gctgtgctgg tccacgctgg gtggagttgt cacaacggac attacttctc ttatgtcaaa 1020
   gctcaagaag gccagtggta taaaatggat gatgccgagg tcaccgcctc tagcatcact 1080
   tctgtcctga gtcaacaggc ctatgtcctc ttttacatcc agaagagtga atgggaaaga 1140
   cacagtgaga gtgcgtcaag aggcagggaa ccaagagccc ttggcgctga agacacagac 1200
   aggcgagcaa cgcaaggaga gctcaagaga gaccacccct gcctgcaggc acccgagttg 1260
   ggcgagcact tggtggaaag agccactcag gaaagcacct tagaccactg gaaattcctt 1320
   caagagcaaa acaaaacgaa gcctgagttc aacgtcagaa aagtcgaagg taccctgcct 1380
   cccaacgtac ttgtgattca tcaatcaaaa tacaagtgtg ggatgaagaa ccatcatcct 1440
   gaacagcaaa gctccctgct aaacctctct tcgacgaccc cgacagatca ggagtccatg 1500
   aacactggca cactcgcttc cctgcagggg aggaccagga gatccaaagg gaagaacaaa 1560
   cacagcaaga gggctctgct tgtgtgccag tgatctcagt ggaagtaccg acccacacat 1620
   gacattcagt gtgtatttct gaatatgacc taccgacgtg taggtttgcg tgtgaggtaa 1680
   atggaggacg actcactcta cttgggaggt gagtggcagt tcaaccactt ttcaaaactc 1740
   acatcttctc ggccagatgc agcttttgct gaaatccagc ggacttctct ccctgagaag 1800
   tcaccactct catctgagac ccgtgtcgac ctctgtgatg atttggctcc tgtggcaaga 1860
   cagcttgctc ccggggagaa gcttcttctg agtagcagga gacctgctgc ggtgggggct 1920
   gggctccaga atatgggaaa tacctgctac gtgaacgctt ccctgcagtg cctgacatac 1980
   acaccgcccc ttgccaacta catgctgtcc cgggagcact ctcaaacgtg tcatcgtcac 2040
   aagggctgca tgctctgtac tatgcaagct cacatcacac gggccctcca cattcctggc 2100
   catgtcatcc agccctcaca ggcattggct gctggcttcc atagaggcaa gcaggaagat 2160
   gcccatgaat ttctcatgtt cactgtggat gccatggaaa aggcatgcct tcccgggcac 2220
   aagcaggtag agcatcactc taaggacacc accctcatcc accaaatatt tggaggctac 2280
   tggagatctc aaatcaagtg tctccactgc cacggcattt cagacacttt tgacccttac 2340
   ctggacatcg ccctggatat ccaggcagct cagagtgtcc agcaagcttt ggaacagttg 2400
   gtgaagcccg aagaactcaa tggagagaat gcctatcatt gtggtctttg tctccagagg 2460
   gcgccggcct ccaagacgtt aactttacac acttctgcca aggtcctcat ccttgtattg 2520
   aagagattct ccgatgtcac aggcaacaaa cttgccaaga atgtgcaata tcctgagtgc 2580
   cttgacatgc agccatacat gtctcagcag aacacaggac ctcttgtcta tgtcctctat 2640
   gctgtgctgg tccacgctgg gtggagttgt cacaacggac attacttctc ttatgtcaaa 2700
   gctcaagaag gccagtggta taaaatggat gatgccgagg tcaccgcctc tagcatcact 2760
   tctgtcctga gtcaacaggc ctatgtcctc ttttacatcc agaagagtga atgggaaaga 2820
   cacagtgaga gtgcgtcaag aggcagggaa ccaagagccc ttggcgctga agacacagac 2880
   aggcgagcaa cgcaaggaga gctcaagaga gaccacccct gcctgcaggc acccgagttg 2940
   ggcgagcact tggtggaaag agccactcag gaaagcacct tagaccactg gaaattcctt 3000
   caagagcaaa acaaaacgaa gcctgagttc aacgtcagaa aagtcgaagg taccctgcct 3060
   cccaacgtac ttgtgattca tcaatcaaaa tacaagtgtg ggatgaagaa ccatcatcct 3120
   gaacagcaaa gctccctgct aaacctctct tcgacgaccc cgacagatca ggagtccatg 3180
   aacactggca cactcgcttc cctgcagggg aggaccagga gatccaaagg gaagaacaaa 3240
   cacagcaaga gggctctgct tgtgtgccag tgatctcagt ggaagtaccg acccacacat 3300
   gacattcagt gtgtatttct gaatatgacc taccgacgtg taggtttgcg tgtgaggtaa 3360
<210> 36
   <211> 1910
   <212> DNA
   <213> Macaca mulatta
<400> 36
   tattatatgt gcctatcatc ctgaggagta atttgattca ggtgttctgg aagtcatgat       60
   gtgggctgtg tctgttgaat tcccatcgat gcaaggggac acaccctgtg actcattcct      120
   gaatggagtg ctgatatttg attggtttat ggcgcacctg atgagtgggt ggggtgttcg      180
   cggttggtgg gggtgagttc tagaagggct gatgcggcca gagagctcgt catttgaaga      240
   ctctctcgga agagatagcg tctttctgca acctgcggtc ccagcagaaa aaccttgtga      300
   tccttgttcc agtcgacatg gaggacgact cactctactt gggaggtgag tggcagttca      360
   accacttttc aaaactcaca tcttctcggc cagatgcagc ttttgctgaa atccagcgga      420
   cttctctccc tgagaagtca ccactctcat ctgagacccg tgtcgacctc tgtgatgatt      480
   tggctcctgt ggcaagacag cttgctccca gggagaagct tcctctgagt agcaggagac      540
   ctgctgcggt gggggctggg ctccagaata tgggaaatac ctgctacgtg aacgcttccc      600
   tgcagtgcct gacgtacaca ccgccccttg ccaactacat gctgtcccgg gagcactctc      660
   caacgtgtca tcgtcacaag ggctgcatgc tctgtactat gcaagctcac atcacacggg      720
   ccctccacat tcctggccgt gtcatccagc cctcacaggc attggctgct gacttccata      780
   gaggcaagca ggaagatgcc catgaatttc tcatgttcac tgtggatgcc atgaaaaagg      840
   catgccttcc cgggcacaag caggtagatc atcactctaa ggacaccacc ctcatccacc      900
   aaatatttgg aggctactgg agatctcaaa tcaagtgtct ccactgccac ggcatttcag 960
   acacttttga cccttacctg gacatcgccc tggatatcca ggcagctcag agtgtcaagc 1020
   aagctttgga acagttggtg aagcccgaag aactcaatgg agagaatgcc tatcattgtg 1080
   gtctttgtct ccagagggcg ccggcctcca agacgttaac tttacacact tctgccaagg 1140
   tcctcatcct tgtattgaag agattctccg atgtcacagg cagcaaactt gccaagaatg 1200
   tgcactatcc tgagtgcctt gacatgcagc catacatgtc tcagcagaac acaggacctc 1260
   ttgtctatgt cctctatgct gtgctggtcc acgctgggtg gagttgtcac aacggacatt 1320
   acttctctta tgtcaaagct caagaaggcc agtggtataa aatggatgat gccgaggtca 1380
   ccgcctctag catcacttct gtcctgagtc aacaggccta tgtcctcttt tacatccaga 1440
   agagtgaatg ggaaagacac agtgagagtg cgtcaagagg cagggaacca agagcccttg 1500
   gcgctgaaga cacagacagg cgagcaacgc aaggagagct caagagagac cacccctgcc 1560
   tgcaggcacc cgagttggac gagcacttgg tggaaagagc cactcaggaa agcaccttag 1620
   accactggaa attccttcaa gagcaaaaca aaacgaagcc tgagttcaac gtcagaaaag 1680
   tcgaaggtac cctgcctccc aacgtacttg tgattcatca atcaaaatac aagtgtggga 1740
   tgaagaacca tcatcctgaa cagcaaagct ccccgctaaa cctctcttcg acgaccccga 1800
   cagatcagga gtccgtgaac actggcacac tcgcttccct gcaggggagg accaggagat 1860
   ccaaagggaa gaacaaacac agcaagaggg ctctgcttgt gtgccagtga 1910
<210> 37
   <211> 1490
   <212> DNA
   <213> Bos taurus
<400> 37
   atgggagccc tgagcaggca ggcggcagac agagccacct gcgggggtgt ggcctttggg      60
   aaggaggtcg acgtcctcag ggagggggct ggccctccgc cgcaggtcgc cccgcagacc     120
   cgcagactga gggtggagcg cgaggaacca aggttcctgg ggcaccgtga gcccctcctg     180
   cccttggcgc acccggccac ctcccaaaga cgccgcggtc ccttgagggt gggggttgtg     240
   ggcccagcgc cgctggtgcg gatgcccttc ggggaccctc tgtccctgag ggccgtcccg     300
   gcgcttcggc gtcccagcgg gacaccagca gaagatgctc acgagtttct gatgttcact     360
   ctgaatgcca tgcagcaagg gtgcttgagt gcatcccagc cgtcgggtca tgcctccgag     420
   gacaccaccg tcatccgtca gatcttcggc gggacctgga ggtctcagat ccagtgtctc     480
   cgctgcctcg gtgtctcgga cacgttcgac ccttatctgg acatcagcct ggatatcacg     540
   gcggctcaga gtgtggagca agctctgaga gagctggtga agcccgagaa gctggacgcg     600
   gacaatgcct atgactgtgg cgtctgtctc cggaaggtgc ctgccaccaa gaggttgact     660
   ttgcacagca cctcccaggt cctggtgctg gtgctgaagc ggttcacacc ggtgagcggg     720
   gccaaaaggg ctcaggaggt gcgctatccc cagtgcttgg acctgcagcc ctacacgtcc     780
   gagcggaagg cagggccact gggctacgtg ctctatgccg tgctggtgca ctccgggtgg     840
   agctgtgagc gaggacacta cttctgttac gtccgagcgg gcaacggcca atggtataag     900
   atggacgatg ccaaggtgac cgcctgtgac gagactgctg ccctgagcca gagcgcctac     960
   gtcctgttct acgcccggga gggtgcgtgg gaagggggcg ctgggggagg ggcagcggcc    1020
   cccgtcgggg ctgaccccac agacccgggg cagcctgcag gagacgccag cggcagagct    1080
   cctgggtcgg aggagtcccc gggggacacg gacgtcgaag ggatgagctt agagcagtgg    1140
   cgacgcctgc aagaacacag ccgaccgaag ccggccttgg agctgcggaa ggtccagtct    1200
   gccctgcctg ccggcgcagt cgtgattcac cagtccaaac acggaggagg gagaaaccgc    1260
   acgccgcccc aacaggagca cgagcggctc gaccgtccca gcacggacac cccgcctccg    1320
   gggccgaaga acgtcggcaa cggcccttgt gccggcggga gggccagagc caccaagggg    1380
   aagaacaaga agccgcggcc gtctctgggg ctgtggcggt aggtcggctc tgacgcacat    1440
   gcgtgcagac gcccaggcac acgctgtgtg gggcacgccc tgtgtgacgc                1490
<210> 38
   <211> 1608
   <212> DNA
   <213> canis lupus familiaris
<400> 38
   atggaggctg cccacctcca cccctcagag gagcctcagt tcagcgcctc tcccaaaccc        60
   cagtcatact ggtcaagggg aggtggtgct gaagtccacg gaggaccctc tgtgcccgag       120
   acgacatccc ctgcatcaaa gacactctcc tccccgactg acccgttggc tcccacatca       180
   gcagggctgc ctcccaccaa gacgcctctg agttggagga gcctttccca ggtgggagcc       240
   gggcttcaga acatgggcaa cacttgctat gtgaatgcga ccctacagtg tctgacctac       300
   acagagcccc tcgccagcta catgctgtcc cagcagcacg ggaccacctg taggaggcag       360
   acatcctgca tgctgtgtac cctgcaggct cacctgacgc gggttctctg ccatcctgga       420
   cgtgtgctcc ggcccctgcc actcctgctc gccgccttcc acagacacaa gcaggaagat       480
   gcccatgagt atctcatgtt cattctggat gcaatgcagc aagcgtgctt gcctgaggac       540
   aagctctcag accctgagtg tcctcaggac agcaccctca tccagcaact ctttgggggg       600
   tactggaggt ctcaaatcca gtgtctccac tgccaaggca tttcgagcac tctggaacct       660
   tacctggaca tcagcctgga catcggggct gctcacagca tcagccaagc cttggagcag       720
   ttgatgaagc ccgaactgct ggaaggtgaa aatgcctacc attgtagtaa gtgtctggag       780
   aaggtgcctg cgtccaaggt gttgacttta cacacttccc cgaaggtcct catcctggtc       840
   ttgagacgat tctcagactt gacaggcaac aaaatgacta aggaggtgca atatcctgag       900
   cgccttgaca tgcaacacta cctgtctgag cagagggcag gacccttggt ttatgtgctc       960
   tatgccgtgc tggtgcacgc tgggaggagt tgccacagcg gacattactt ctgtttcgta      1020
   aaggcaggaa atggccagtg gtataaaatg gatgatgcta aggtcagcgc ctgtgatgtg      1080
   acttgcgcgc tacgccaacc tgcctatgtc ctcttttata tgcagaagac tgatctggag      1140
   agagaccttg ggagggagtc agtcgaggag ggaggactcg catctcccga ggcagacccc      1200
   acggtggtgg gtgaggcctc aggagagccg gcaaccgatc cctccgggaa ccatcctgag      1260
   ttggaggagc gtggggaaga gacctcaagg caacaaatga cattagacca gtggagatgc      1320
   ctccaagaat gcaaccgccc taagcctgaa ctccatgtca ggagaagaga aattgctctt      1380
   cctgcgaacg cagtcatcct tcaccactcc aaatacagac ctgagatgcc gaagaatcat      1440
   cctcagccga ccgtcgacct gctcaccact gcagctggga tgctcccacc tcaggtggcc      1500
   ggggacatgg ccaaagtccc gcgtgtgcca gggagagccc gacctaccaa gaggacgagc      1560
   aagaagggac agaggtctgg ggaagcagtc cagggatgtg tctcctaa                   1608
<210> 39
   <211> 12
   <212> PRT
   <213> Artificial Sequence
<220>
   <223> peptide derived from Dub3 protein
<400> 39
   Met Ser Pro Gly Gln Leu Cys Ser Gln Gly Gly Arg
   1 5 10
<210> 40
   <211> 19
   <212> RNA
   <213> Artificial Sequence
<220>
   <223> siRNA directed against cdc25A
<400> 40
   gaaauuuccc ugacgagaa 19
<210> 41
   <211> 19
   <212> RNA
   <213> Artificial Sequence
<220>
   <223> siRNA derected against Dub3
<400> 41
   ggcuguaaga ugugugcua 19
<210> 42
   <211> 19
   <212> RNA
   <213> Artificial Sequence
<220>
   <223> derived from Dub3
<400> 42
   uagcacacau cuuacagcc 19
<210> 43
   <211> 10
   <212> RNA
   <213> Artificial Sequence
<220>
   <223> linker for shRNA
<400> 43
   cuuccuguca 10